(19) Europäisches Patentamt / European Patent Office / Office européen des brevets

(11) **EP 4 069 336 B1**

(12) **EUROPEAN PATENT SPECIFICATION**

(45) Date of publication and mention of the grant of the patent:
**22.10.2025 Bulletin 2025/43**

(21) Application number: **20897136.6**

(22) Date of filing: **04.12.2020**

(51) International Patent Classification (IPC):
*G01N 13/04* (2006.01)  *G01N 33/49* (2006.01)
*G01N 15/00* (2024.01)  *G01N 15/10* (2024.01)
*G01N 15/14* (2024.01)  *G01N 15/06* (2024.01)
*G01N 15/01* (2024.01)

(52) Cooperative Patent Classification (CPC):
**G01N 13/04; G01N 33/4915;** G01N 15/06;
G01N 2015/012; G01N 2015/1024;
G01N 2015/1486

(86) International application number:
**PCT/US2020/063335**

(87) International publication number:
**WO 2021/113652 (10.06.2021 Gazette 2021/23)**

(54) **IMPROVED METHODS FOR MEASURING CELL NUMBERS AND/OR CELL PROPERTIES**

VERBESSERTE VERFAHREN ZUR MESSUNG VON ZELLZAHLEN UND/ODER ZELLEIGENSCHAFTEN

PROCÉDÉS AMÉLIORÉS POUR MESURER DES NOMBRES DE CELLULES ET/OU DES PROPRIÉTÉS DE CELLULES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **04.12.2019 US 201962943757 P**

(43) Date of publication of application:
**12.10.2022 Bulletin 2022/41**

(73) Proprietors:
• **Shine, Ian Basil**
**Swampscott, MA 01907 (US)**
• **Shine, Thomas Adam**
**Brookline, MA 02445 (US)**

(72) Inventors:
• **Shine, Ian Basil**
**Swampscott, MA 01907 (US)**
• **Shine, Thomas Adam**
**Brookline, MA 02445 (US)**

(74) Representative: **Schiweck Weinzierl Koch Patentanwälte Partnerschaft mbB Ganghoferstraße 68 B 80339 München (DE)**

(56) References cited:
US-A- 5 656 034          US-A- 5 939 326
US-A1- 2002 022 802      US-A1- 2004 214 314
US-A1- 2006 079 003      US-A1- 2010 211 003
US-A1- 2013 079 599      US-A1- 2017 159 003
US-A1- 2017 276 591      US-A1- 2019 234 399
US-B- 6 211 956          US-B1- 6 668 229
US-B2- 7 482 165

• **GUPTA VISHAL ET AL: "ApoStream(TM), a new dielectrophoretic device for antibody independent isolation and recovery of viable cancer cells from blood", BIOMICROFLUIDICS, vol. 6, no. 2, 27 June 2012 (2012-06-27), US, XP093139339, ISSN: 1932-1058, DOI: 10.1063/ 1.4731647**

## Description

### CROSS-REFERENCE

This application claims priority to and the benefit of US Application No. 62/943,757 filed on December 4, 2019.

### BACKGROUND

The measurement of the physical properties of red blood cells, such as cell volume, shape, and radius, has been found to have significant diagnostic value. For example, a low average volume of red blood cells may be used to diagnose the etiology of anemia and is associated with symptoms of dietary insufficiencies (e.g., iron deficiency). Accordingly, a number of diagnostic devices and methods have been developed to attempt to accurately assess red blood cell features, such as hemoglobin concentration, volume, and/or to assess at what osmolality level red blood cells may achieve a maximum volume.

### SUMMARY

The present disclosure provides additional methods, systems and techniques for improving osmotic gradient generating systems for use in technologies to accurately determine red blood cell volume and the osmolality at which cells achieve a maximum volume. In particular, the invention relates to methods for measuring a concentration or a physical property of cells in a biological sample as defined in the independent claims 1, 3, 6, 7 and 11. Further aspects of the invention are described in the dependent claims.

### DEFINITIONS

*A or An:* The articles "a" and "an" are used herein to refer to one or to more than one (*i.e.,* to at least one) of the grammatical object of the article. By way of example, "an element" refers to one element or more than one element.

*About:* The term "about", when used herein in reference to a value, refers to a value that is similar, in context to the referenced value. In general, those skilled in the art, familiar with the context, will appreciate the relevant degree of variance encompassed by "about" in that context. For example, in some embodiments, the term "about" may encompass a range of values that within 25%, 20%, 19%, 18%, 17%, 16%, 15%, 14%, 13%, 12%, 11%, 10%, 9%, 8%, 7%, 6%, 5%, 4%, 3%, 2%, 1%, or less of the referred value.

*Agent:* In general, the term "agent", as used herein, may be used to refer to a compound or entity of any chemical class including, for example, a polypeptide, nucleic acid, saccharide, lipid, small molecule, metal, or combination or complex thereof. In appropriate circumstances, as will be clear from context to those skilled in the art, the term may be utilized to refer to an entity that is or comprises a cell or organism, or a fraction, extract, or component thereof. Alternatively or additionally, as context will make clear, the term may be used to refer to a natural product in that it is found in and/or is obtained from nature. In some instances, again as will be clear from context, the term may be used to refer to one or more entities that is man-made in that it is designed, engineered, and/or produced through action of the hand of man and/or is not found in nature. In some embodiments, an agent may be utilized in isolated or pure form; in some embodiments, an agent may be utilized in crude form. In some embodiments, potential agents may be provided as collections or libraries, for example that may be screened to identify or characterize active agents within them. In some cases, the term "agent" may refer to a compound or entity that is or comprises a polymer; in some cases, the term may refer to a compound or entity that comprises one or more polymeric moieties. In some embodiments, the term "agent" may refer to a compound or entity that is not a polymer and/or is substantially free of any polymer and/or of one or more particular polymeric moieties. In some embodiments, the term may refer to a compound or entity that lacks or is substantially free of any polymeric moiety.

*Biological Sample:* As used herein, the term "biological sample" typically refers to a sample obtained or derived from a biological source (e.g., a tissue or organism) of interest, as described herein. In some embodiments, a source of interest comprises an organism, such as an animal or human. In some embodiments, a biological sample is or comprises biological tissue or fluid. In some embodiments, a biological sample may be or comprise blood; blood cells; cell-containing body fluids; other body fluids; secretions; and/or excretions; and/or cells therefrom, etc. In some embodiments, a biological sample is or comprises cells obtained from an individual. In some embodiments, obtained cells are or include cells from an individual from whom the sample is obtained. In some embodiments, a sample is a "primary sample" obtained directly from a source of interest by any appropriate means. For example, in some embodiments, a primary biological sample is obtained by methods selected from the group consisting of surgery, collection of body fluid (e.g., blood, plasma, cerebrospinal fluid, etc.), etc. In some embodiments, as will be clear from context, the term "sample" refers to a preparation that is obtained by processing (e.g., by removing one or more components of and/or by adding one or more agents to) a primary sample. For example, centrifugation to remove fluid not containing material of interest or dilution via a buffer (e.g., a phosphate buffered saline (PBS) solution, a solution of a particular pH, a solution of a particular osmolality etc.). As used herein, the terms buffer and diluent are used interchangeably. Such a "processed sample" may comprise, for example cells extracted from a sample or obtained by subjecting a primary sample to techniques such as isolation and/or purification of certain components, etc.

*Cell Parameters:* As used herein, the term "cell parameters", "cell properties" or "parameters" typically is used to refer to any physical parameters or properties of a cell that may be characterized, either directly or indirectly, through the use of the methods, systems, and apparatus as described herein. In some embodiments, a physical parameter is a size measurement of a cell, diameter of the cell, a radius of the cell, a surface area of a cell, a thickness of a cell, or a volume of a cell. In some embodiments, a physical parameter is an electrical resistance of a cell, a voltage, a current passing to / through a cell, an inductance, a capacitance, or an impedance of a cell. In some embodiments, a cell parameter is a concentration of hemoglobin in a cell. In certain embodiments, a cell parameter is a change in any one of these properties as discussed herein.

*Cell Size*: As used herein, the term "cell size" refers to a physical measure of a cell that may be determined, either indirectly or directly, from one or more measurements conducted on the cell. In certain embodiments, the cell size refers to the volume, diameter, surface area, thickness, or radius of a cell.

*Cell Shape:* As used herein, the term "cell shape" refers to one or more physical measures of a cell that may be determined, either directly or indirectly, from the one or more measurements conducted on a cell and is used to describe the shape of the cell. In certain embodiments, the cell shape may refer to the sphericity, surface-area-to-volume ratio, or cell thickness.

*Cell Suspension*: As used herein, the term "cell suspension" refers to any liquid sample comprising cells of one or more types. In certain embodiments, a cell suspension is a whole blood sample obtained directly from an individual. In certain embodiments, a cell suspension is a whole blood sample which has been diluted using one or more diluents. In preferable embodiments, a cell suspension comprises, at least, red blood cells obtained from a subject. In certain preferable embodiments, a cell suspension comprises other cell types and/or cell lines in a sample obtained from a subject.

*Cancer*: The terms "cancer", "malignancy", "neoplasm", "tumor", and "carcinoma", are used herein to refer to cells that exhibit relatively abnormal, uncontrolled, and/or autonomous growth, so that they exhibit an aberrant growth phenotype characterized by a significant loss of control of cell proliferation. In some embodiments, a tumor may be or comprise cells that are precancerous (e.g., benign), malignant, pre-metastatic, metastatic, and/or non-metastatic. The present disclosure specifically identifies certain cancers to which its teachings may be particularly relevant. In some embodiments, a relevant cancer may be characterized by a solid tumor. In some embodiments, a relevant cancer may be characterized by a hematologic malignancy. In general, examples of different types of cancers known in the art include, for example, carcinoid cancer, common bile duct cancer, bronchial cancer, colon cancer, endometrial cancer, gall bladder cancer, ileal carcinoid carcinoma, leukemia, lung cancer, lymphoma, malignant melanoma, multiple myeloma, mycosis fungoides, ovarian cancer, rectal cancer, renal cancer, sarcoma, stomach cancer, testicular cancer, thyroid cancer, cancers of unknown origin, hematopoietic cancers including leukemias, lymphomas (Hodgkin's and non-Hodgkin's), myelomas and myeloproliferative disorders, melanomas, adenomas, carcinomas of solid tissue, squamous cell carcinomas of the mouth, throat, larynx, and lung, liver cancer, genitourinary cancers such as prostate, cervical, bladder, uterine, and endometrial cancer and renal cell carcinomas, bone cancer, pancreatic cancer, skin cancer, cutaneous or intraocular melanoma, cancer of the endocrine system, cancer of the thyroid gland, cancer of the parathyroid gland, head and neck cancers, breast cancer, gastro-intestinal cancers and nervous system cancers, benign lesions such as papillomas, and the like.

*Comparable*: As used herein, the term "comparable" refers to members within sets of two or more conditions, circumstances, agents, entities, populations, etc., that may not be identical to one another but that are sufficiently similar to permit comparison there between, such that one of skill in the art will appreciate that conclusions can reasonably be drawn based on differences or similarities observed. In some embodiments e.g., as set forth herein, comparable sets of conditions, circumstances, agents, entities, populations, etc. are typically characterized by a plurality of substantially identical features and zero, one, or a plurality of differing features. Those of ordinary skill in the art will understand, in context, what degree of identity is required to render members of a set comparable. For example, those of ordinary skill in the art will appreciate that members of sets of conditions, circumstances, agents, entities, populations, etc., are comparable to one another when characterized by a sufficient number and type of substantially identical features to warrant a reasonable conclusion that differences observed can be attributed in whole or part to non-identical features thereof.

*"Diluent"*: As used herein, the terms "diluent" and "buffer" are used interchangeably. In certain embodiments, a diluent refers to any liquid solution that is mixed and/or added to another substance, another liquid, and/or a sample comprising cells as described herein. In certain embodiments, a diluent is a saline solution. In certain embodiments, a saline solution is or comprises a phosphate buffered saline solution (e.g, PBS, Dulbecco's PBS), water, and/or sodium chloride. In certain embodiments, a diluent is and/or comprises water.

*Dilution ratio:* As used herein, the term "dilution ratio" indicates the relative amount of two or more liquids mixed together to create a "diluted sample". In some embodiments, the dilution ratio is calculated as a ratio of the amount of a first liquid added to or diluted with a second liquid. In certain preferred embodiments, the dilution ratio is determined to be the volume of the first liquid as compared to a second liquid.

*Environment:* As used herein, the term "environment" is used in reference to the testing conditions to which one or more cells are being subjected to. In certain embodiments, the environment refers to one or more parameters of the solution in which the cells are present. In certain embodiments, the environment refers to physical stresses under which cells and/or samples may be places. In certain embodiment physical stresses include heat, ultrasound, electrical fields, gravitational (e.g., centrifugal) forces In certain embodiments, a "single environment" indicates that one or more parameters (e.g., up to and including all parameters) of the solution (e.g., pH, osmolality, temperature, agent concentration) and/or physical conditions (e.g., up to and including all conditions) remain about uniform for a given duration of time.

*Hypertonic:* As used herein, the term hypertonic refers to the tonicity of a first solution being higher than the tonicity of a second solution (e.g., when the first solution has a higher osmolality than the second solution) (e.g., when the second solution is a biological sample, e.g., blood); the first solution is said to be "hypertonic" as compared to the second solution. For example, blood is hypertonic as compared to water. In certain embodiments, a hypertonic solution (e.g., for a human subject) has an osmolality greater than 290mOsm/kg, greater than 300mOsm/kg, greater than 310mOsm/kg, greater than 320mOsm/kg, greater than 330mOsm/kg, greater than 350mOsm/kg, greater than 400mOsm/kg, greater than 450mOsm/kg or greater. In certain embodiments, the range of hypertonic solutions changes based on an individual and/or species being tested. In certain embodiments, the term "hypertonic" is used relate the tonicity of a solution (e.g., an environmental property of a solution, e.g., a diluent, a sample) with a measured and/or predicted tonicity biological sample (e.g, blood). In certain preferable embodiments, the term hypertonic relates the tonicity of a diluent and/or buffer to the tonicity (e.g., measured and/or predicted) that is higher than the tonicity of a biological sample (e.g., a whole blood sample, a diluted blood sample) or a component thereof (e.g., serum).

*Hypotonic:* As used herein, the term hypotonic refers to the tonicity of a first solution being less than as the tonicity of a second solution (e.g., when the first solution has a lower osmolality than the second solution) (e.g., when the second solution is a biological sample, e.g., blood); the first solution is said to be "hypotonic" as compared to the second solution. For example, water is hypotonic as compared to whole blood. In certain embodiments, a hypotonic solution has an osmolality of less than 290mOsm/kg, less than 280mOsm/kg, less than 270mOsm/kg, less than 260mOsm/kg, less than 250mOsm/kg, less than 200mOsm/kg, less than 150mOsm/kg, less than 100mOsm/kg, less than 50mOsm/kg, less than 20mOsm/kg, less than 10mOsm/kg, less than 5mOsm/kg, or less. In certain preferable embodiments, the osmolality of a hypotonic solution is about 0mOsm (e.g., water). In certain embodiments, the term "hypotonic" is used relate the tonicity of a solution with a measured or predicted tonicity biological sample. In certain embodiments, the term hypotonic relates the tonicity of a diluent or buffer to the tonicity (e,g,, measured and/or predicted) of a whole blood sample or a component thereof (e.g., the serum).

*Isotonic:* As used herein, the term isotonic refers to when the tonicity of a first solution is about the tonicity of a second solution (e.g., when the osmolality of the first solution is about the osmolality of the second solution). In certain embodiments, an isotonic solution has an osmolality of about 240-340 mOsm/kg, about 250-330 mOsm/kg, about 260-320 mOsm/kg, about 270-310 mOsm/kg, about 275-300 mOsm/kg. In certain preferable embodiments, the osmolality of an isotonic solution is about 280 mOsm/kg (e.g., for human samples). In certain embodiments, an isotonic solution has an osmolality about the same as the osmolality as a measured or predicted tonicity of a biological sample.

*"Improved," "increased" or "reduced":* As used herein, these terms, or grammatically comparable comparative terms, indicate values that are relative to a comparable reference measurement. For example, in some embodiments, an assessed value achieved with an agent or method of interest may be "improved" relative to that obtained with a comparable reference agent or method, respectively. Alternatively or additionally, in some embodiments, an assessed value achieved in a sample or system of interest may be "improved" relative to that obtained in the same sample or system under different conditions (e.g., prior to or after an event such as using an agent of interest), or in a different, comparable subject (e.g., in a comparable subject or system that differs from the subject or system of interest in presence of one or more indicators of a particular disease, disorder or condition of interest, or in prior exposure to a condition or agent, etc). In some embodiments, comparative terms refer to statistically relevant differences (e.g., that are of a prevalence and/or magnitude sufficient to achieve statistical relevance). Those skilled in the art will be aware, or will readily be able to determine, in a given context, a degree and/or prevalence of difference that is required or sufficient to achieve such statistical significance.

*Predetermined:* By predetermined is meant deliberately selected, for example as opposed to randomly occurring or achieved.

*Reference:* As used herein, the term "reference" refers to a standard or control relative to which a comparison is performed. For example, in some embodiments, an agent, animal, individual, population, sample, sequence, or value of interest is compared to a reference or control agent, animal, individual, population, sample, sequence, or value. In some embodiments, a reference or control is tested and/or determined substantially simultaneously with the testing or determination of interest. In some embodiments, a reference or control is a historical reference or control, optionally embodied in a tangible medium. Typically, as would be understood by those skilled in the art, a reference or control is determined or characterized under comparable conditions or circumstances to those under assessment. Those skilled in the art will appreciate when sufficient similarities are present to justify reliance on and/or comparison to a particular possible reference or control.

*Sample:* As used herein, the term "sample" typically refers to an aliquot of material obtained or derived from a source of interest, as described herein. In some embodiments, a source of interest is a biological source. In some embodiments, a source of interest may be or comprise a cell or an organism, such as a microbe. In some embodiments, a source of interest is or comprises biological tissue or fluid. In some embodiments, a biological tissue or fluid may be or comprise amniotic fluid, aqueous humor, ascites, bile, bone marrow, blood, breast milk, cerebrospinal fluid, cerumen, chyle, chime, ejaculate, endolymph, exudate, feces, gastric acid, gastric juice, lymph, mucus, pericardial fluid, perilymph, peritoneal fluid, pleural fluid, pus, rheum, saliva, sebum, semen, serum, smegma, sputum, synovial fluid, sweat, tears, urine, vaginal secretions, vitreous humour, vomit, and/or combinations or component(s) thereof. In some embodiments, a biological fluid may be or comprise an intracellular fluid, an extracellular fluid, an intravascular fluid (blood plasma), an interstitial fluid, a lymphatic fluid, and/or a transcellular fluid. In some embodiments, a biological tissue or sample may be obtained, for example, by aspirate, biopsy (e.g., fine needle or tissue biopsy), swab (e.g., oral, nasal, skin, or vaginal swab), scraping, surgery, washing or lavage (e.g., bronchoalveolar, ductal, nasal, ocular, oral, uterine, vaginal, or other washing or lavage). In some embodiments, a biological sample is or comprises cells obtained from an individual. In some embodiments, a sample is a "primary sample" obtained directly from a source of interest by any appropriate means. In some embodiments, as will be clear from context, the term "sample" refers to a preparation that is obtained by processing (e.g., by removing one or more components of and/or by adding one or more agents to) a primary sample. For example, filtering using a semi-permeable membrane. Such a "processed sample" may comprise, for example nucleic acids or proteins extracted from a sample or obtained by subjecting a primary sample to one or more techniques such as amplification or reverse transcription of nucleic acid, isolation and/or purification of certain components, *etc.*

*Tonicity:* As used herein, the term tonicity is used to refer to the relative amount of solutes in one solution as compared to another. In certain embodiments, the tonicity refers to the relative comparison of the osmolarity between the two solutions. In certain embodiments, the terms "hypertonic", "isotonic", and "hypotonic" are used in relation of the tonicity of a diluent to the tonicity of a sample (or a portion thereof). In certain embodiments, the terms "hypertonic", "isotonic", and "hypotonic" are used in relation to a fixed value. In certain embodiments, the fixed value is about 250mOsm/kg, about 260mOsm/kg, about 270mOsm/kg, about 280mOsm/kg, about 290mOsm/kg, about 300mOsm/kg, about 310mOsm/kg, about 320mOsm/kg, about 330mOsm/kg, about 340mOsm/kg, about 350mOsm/kg. In certain preferable embodiments, the fixed value is about 290mOsm/kg.

## BRIEF DESCRIPTION OF THE DRAWING

The foregoing and other objects, aspects, features, and advantages of the present disclosure will become more apparent and better understood by referring to the following description taken in conjunction with the accompanying drawings, in which:

FIG. 1 is a schematic drawing of an embodiment of a device described herein used to dilute a biological sample (e.g., a whole blood sample) and subject the sample to a changing environment;
FIG. 2A is a schematic drawing of an embodiment of a device described herein used to dilute a biological sample (e.g., a whole blood sample) and subject the sample to a changing environment;
FIG. 2B is a schematic drawing of an embodiment of a device described herein used to dilute a biological sample (e.g., a whole blood sample) and subject the sample to a changing environment;
FIG. 3 is a schematic drawing of an embodiment of a device described herein used, in certain embodiments, to dilute a biological sample (e.g., a whole blood sample) serially or create multiple aliquots of different samples for multiplexed sample creation;
FIG. 4 is a schematic drawing of an embodiment of a device described herein used to dilute a biological sample (e.g., a whole blood sample) and subject the sample to a changing environment;
FIG. 5 is a schematic drawing of an embodiment of a device described herein used to dilute a biological sample (e.g., a whole blood sample) and subject the sample to a changing environment;
FIG. 6 is a perspective drawing of an embodiment of a high resolution, digitally driven syringe pump as described herein;
FIG. 7A is a schematic drawing of an embodiment of a device described herein used to dilute a biological sample (e.g., a whole blood sample) and, in certain embodiments, subject the sample to changing environments while performing measurements using a sensor;
FIG. 7B is a schematic drawing of an embodiment of a device described herein used to dilute a biological sample (e.g., a whole blood sample) and, in certain embodiments, subject the sample to a changing environments while performing measurements using a sensor;

FIG. 7C is a schematic drawing of an embodiment of a device described herein used to dilute a biological sample (e.g., a whole blood sample) and, in certain embodiments, subject the sample to changing environments while performing measurements using a sensor;

FIG. 8 is a schematic drawing of an embodiment of a device described herein used to dilute a biological sample (e.g., a whole blood sample) and subject the sample to a changing environment;

FIG. 9A is a cross-section of an embodiment of a sensor arrangement used to as described herein for making a DC voltage measurement of cells as they traverse a small aperture;

FIG. 9B is an exploded perspective view of the embodiment of the sensor of FIG. 9A used to as described herein for making a DC voltage measurement of cells as they traverse an aperture;

FIG. 10 is a schematic drawing of an embodiment of a vortex mixing chamber;

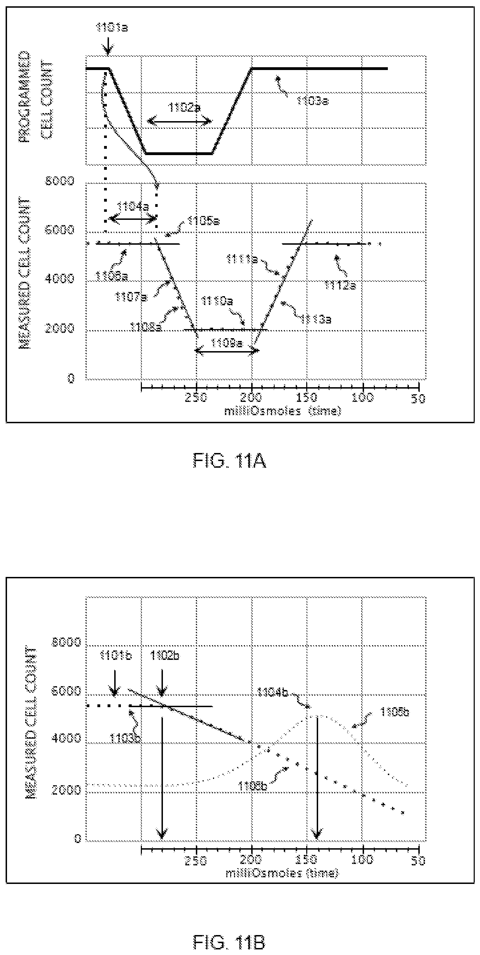

FIG. 11A is an illustrative graph of an exemplified embodiment of a programmed cell count as compared to a measured cell count at various osmolalities, which are calculated relative to a unit of time;

FIG. 11B is an illustrative graph used to demonstrate a programmed change in the cell count with a programmed change in osmolality over time, in an embodiment of the device;

FIG. 12 is an illustrative graph of a spherical red blood cell (1202) and a biconcave red blood cell (1201) passing through a sensor of a device, as recorded in an embodiment of the device;

FIG. 13 is a schematic drawing of an embodiment of a device as described herein having multiple sensors for multiplexed mixing and measuring of cells in suspension;

FIG. 14 is a schematic drawing of an embodiment of a device as described herein having multiple sensors for multiplexed mixing and measuring of cells in suspension;

FIG. 15A is a graph of data collected in a cell-by-cell analysis showing the voltage recorded for individual red blood cells of a healthy individual at discrete osmolalities (in a range from 280 mOsm/kg to 54 mOsm/kg);

FIG. 15B is a graph of the mean cell voltage / size as collected in the cell-by-cell analysis of FIG. 15A at discrete points, showing data points and an interpolated curve;

FIG. 15C is a graph of change in cell volume with respect to change in osmolality of a test sample ("Cell Scan Plot") as generated from the discretized data as obtained in FIG. 15A;

FIG. 16A is a graph of data collected in a cell-by-cell analysis showing the voltage recorded for individual red blood cells of a healthy individual over decreasing osmolality (in a range from 280 mOsm/kg to 54 mOsm/kg). Population density is represented by color, with zero density corresponding to white, the lowest nonzero density corresponding to the darkest points (e.g., blue), and, as density progressively increases, color of the points lightens (e.g., from green to yellow to orange to red to black to aqua), as recorded using an embodiment of the device discussed herein;

FIG. 16B is a fluid flux curve (FFC) plotting the percent change of rate of fluid flux with respect to changes in osmolality of a test sample, as recorded using a predetermined window filter in an embodiment of the device discussed herein;

FIG. 16C is a fluid flux curve (FFC) plotting the percent change of rate of fluid flux with respect to changes in osmolality of a test sample, as recorded using a sample specific sample specific, variable window filter in an embodiment of the device discussed herein;

FIG. 16D is a graph of change in cell volume with respect to change in osmolality of a test sample ("Cell Scan Plot");

FIG. 17 is a graph showing the change in Pk0 (peak osmolality) at different concentrations of cells;

FIG. 18 is a block diagram of an exemplary cloud computing environment, used in certain embodiments;

FIG. 19 is a block diagram of an example computing device and an example mobile computing device used in certain embodiments;

FIG. 20 is a schematic diagram of mechanisms to decrease the effect of mechanical vibrations and/or electrical noise of an embodiment of the apparatus; and

FIG. 21 is a graph of the effect of the inner diameter of tubing on Pk0 of red blood cells as the length of the tubing is altered, while also varying tubing diameter. Tubing having a 0.5mm inner diameter is labelled using diamonds and tubing having a 0.8mm inner diameter is labelled using o's;

FIG. 22 is a graph of the effect of the inner diameter of tubing on Pk0 of red blood cells as the mixing time of the sample is altered. Tubing having a 0.5mm inner diameter is labelled using diamonds and tubing having a 0.8mm inner diameter is labelled using o's;

FIG. 23 is a graph of the effect of temperature on Pk0 of red blood cells as the incubation time is altered. Samples were incubated at 4°Celcius (C), 37° C, 40°C, and 42°C for up to 44 hours. Each sample is labelled with the temperature at which it was incubated;

FIG. 24 is a graph of the effect of pH on Pk0 of red blood cells;

FIG. 25 is a graph of percent volume change of a cell and osmolality;

FIG. 26A is an illustrative schematic of an exemplary device for measuring cell properties and/or number, as disclosed herein; and

FIG. 26B is an illustrative schematic of an exploded view of an exemplary device for measuring cell properties and/or number, as disclosed herein.

The features and advantages of the present disclosure will become more apparent from the detailed description set forth below when taken in conjunction with the drawings. Same numbers used in the drawings are indicative of same or like components.

## DETAILED DESCRIPTION OF CERTAIN EMBODIMENTS

The present disclosure provides certain technologies for assessing one or more red blood cell parameters, for example in particular red blood cell preparations and/or as may respond to certain environmental change(s).

Those skilled in the art, reading the present disclosure will appreciate that its teachings are not limited to the particular embodiments exemplified herein.

Throughout the description, one or more articles, devices, systems, and/or architectures may be described as "having", "including", or "comprising" particular components, and/or one or more processes or methods may analogously be described as "having", "including", or "comprising" particular steps; those skilled in the art, reading the disclosure, will appreciate that, in some embodiments, such articles, devices, systems, and architectures, or processes and methods may consist essentially of, or consist of, the recited particular element(s).

Those skilled in the art, reading the present disclosure, will also appreciate that, a particular order of steps or order for performing certain actions described herein may, in some embodiments, be varied without destroying operability of provided methods; in some embodiments, one or more steps or actions specifically described separately may be conducted simultaneously.

The mention herein of any publication, for example, in the Background section, is not an admission that the publication serves as prior art with respect to any of the claims presented herein. The Background section is presented for purposes of clarity and is not meant as a description of prior art with respect to any claim.

Where there is any discrepancy in the meaning of a particular term between and incorporated reference and the Definition section provided herein, the meaning provided in the Definition section above is controlling.

Headers are provided for the convenience of the reader - the presence and/or placement of a header is not intended to limit the scope of the subject matter described herein.

## Prior Technologies

Prior publications (*See, e.g.,* WO1997/024601; US Pat. No. 6,668,229, US Pat. No. 6,422,065) have described certain apparatuses and methods for measuring cell volume by altering certain cell environmental parameter(s) (e.g., sample osmolality) and, subsequently, measuring resultant changes to one or more physical characteristics (e.g., volume, shape, surface area) of the cells through the use of electrical impedance measurements, and have demonstrated value of such measurements in certain contexts.

US 2017/159003 A1 discloses a device which automatically performs a step in which expanded and cultured cells are diluted to a desired cell concentration.

In particular, such prior publications have described certain mechanical, gradient generating systems, and techniques for their use in varying environmental parameter(s) to which cells are exposed as they are passed through an aperture, which permit recording of cell size as it relates to an electrical signal generated by the cell passing through the aperture. The gradient generating system as described creates a mixture of the sample and a buffer solution with different osmolalities over a continuous range by controlling the rate at which the sample and buffer are mixed by using a slowly rotating, constant speed cam that has been cut with a pre-determined profile. The size of the cells are recorded at various osmolalities as the cells pass through the aperture of the device.

While celebrating the usefulness of such prior system(s), the present disclosure provides certain insights that identify the source(s) of particular problems with such systems and/or identify opportunities for their improvement. For example, the present disclosure provides an insight that the use of fixed osmotic gradients in such systems reduces their flexibility. The present disclosure further identifies that these systems can suffer from a lack of accuracy when determining properties (e.g., the peak osmolality, the volume) of the red blood cells within a biological sample. Accordingly, the present disclosure teaches that there is a need for the development of systems which enable a higher degree of accuracy and flexibility in the determination of red blood cell shape and the osmolality at which red blood cells achieve a maximum volume.

## Provided Technologies

In certain embodiments, present disclosure provides technologies (e.g., methods and/or apparatuses) that represent improvements to, and/or can be used to improve upon, one or more prior technologies, e.g., as described above.

To give but a few specific examples:

(a) in some embodiments, application of one or more provided technologies to systems and/or methods disclosed in US Pat. No. 6,668,229, can permit more accurate and/or precisely quantification of one or more cellular parameters such as, for example, one or more of Pk0, cell volume, cell diameter, cell thickness, cell permeability, among others.
(b) in some embodiments, application of one or more provided technologies to systems and/or methods disclosed in US Pat. No. 6,422,065 may achieve more rapid and/or accurate, or otherwise improved, determination (e.g., quantification) of one or more cell parameters such as, for example, surface area, surface area to volume ratio, sphericity index, cell diameter, cell thickness, etc, and combinations thereof
(c) in some embodiments, application of one or more provided technologies to systems and/or methods disclosed in U.S. Pat. No. 6,422,065 may achieve certain improvements, for example, in, methods and/or apparatuses for altering cell parameters to determine the point at which cells, particularly red blood cells, achieve a spherical volume (e.g., the Pk0).

As described herein, the present disclosure identifies a need for, and also provides, improved methods and/or systems for maintaining a uniform concentration of cells and/or for achieving precise control over a sample environment while measuring clinically important diagnostic parameters of red blood cells, such as an osmolality at which cells achieve a maximum volume (Pk0). Accordingly, methods and devices as developed herein are, in certain embodiments, achieve increased levels of control and/or precision of control over the sample environment.

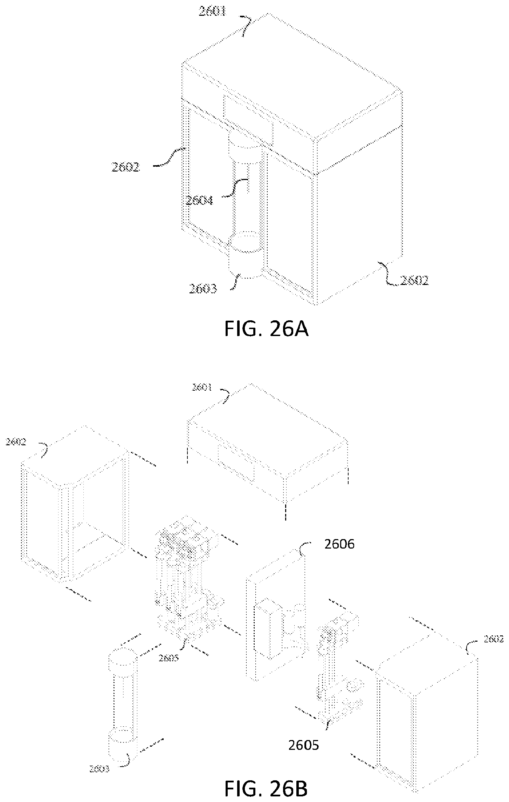

By way of example and not of limitation, exemplary schematics of a system used to achieve goals set forth in this disclosure is shown in FIGs. 26A and 26B. These figures show an isometric view (FIG. 26A) of an instrument in a configuration with approximate dimensions of 14.25" x 9.35" x 15.25" (W x D X H) and an exploded view (FIG. 26B). FIG. 26A shows an electronics bay 2601, syringe drive compartment 2602, sampling area 2603, and sample probe 2604. The electronics bay 2601 houses electrical components (e.g., computational components) of the exemplary system. The sample probe provides a means by which a biological sample can be aspirated into the system. In the syringe drive compartment 2602, a syringe system 2605(as described herein) is used to dilute a sample and/or subject a sample to particular environmental conditions and/or agents. A sensor used to measure cell properties along with other fluidic components are shown mounted on a plate 2606.

The present disclosure teaches that, when measuring a parameter (e.g., the Pk0, diameter, volume) of one or more cells (e.g., red blood cells, white blood cells, platelets) from a sample (e.g., a whole blood sample) using a system as described herein, in some embodiments it may be important or desirable to maintain a reasonably uniform concentration of cells being passed to and/or through a sensor. Without wishing to be bound by any particular theory, the present disclosure provides that number of cells (e.g., red blood cells, white blood cells, platelets) in a biological sample varies from one sample to another due, for example, to natural variations of cell concentrations within a population of subjects and/or due to a health condition (e.g., either chronic or transient) a subject is experiencing. Furthermore, the present disclosure provides an insight that such variation may materially impact accuracy, quality, and/or reproducibility of measurement of one or more cell parameters in systems as described herein. The present disclosure thus provides an insight that ensuring uniformity of cell concentrations passing through a sensor, and/or ensuring fixed rate of cell passage through such sensor, can achieve important improvements, for example, in minimizing sample-to-sample and/or inter-sample variation and thus improving accuracy, quality, and/or reproducibility of result(s) achieved.

Insights provided herein can improve accuracy, quality, and/or reproducibility of measurements including cell concentration (e.g., red blood cells, white blood cells, platelets), including cell shape, including cell size, including within a whole blood sample (e.g., a complete blood count (CBC)).

In prior technologies such as automated cell analyzers, biological samples are typically diluted prior to testing using a fixed dilution ratio of approximately 1:5000 (volume of biological sample : volume of diluent) of biological sample to a diluent, such as phosphate buffered saline (PBS) or other similar isotonic solutions. In such prior technologies, the biological sample is aspirated by the sampling unit and diluted by a small bore sampling loop in conjunction with sampling valves. In other instances, biological samples are diluted using auto-pipettes.

The present disclosure provides an insight that use of a fixed dilution ratio can create a problem in certain instances, for example, when the concentration of cells of the diluted sample is too high to be properly measured by the sensor.

Prior efforts to correct for errors or inaccuracies attributable to variations in cell density across samples have included reliance of one or more "correction factors" when calculating cell parameter(s) such as size, diameter, volume, concentration, etc. The present disclosure appreciates, however, that such reliance introduces artificiality into determined values and therefore may distort or obfuscate biological reality.

To give but a few examples, use of a fixed dilution ratio of a whole blood sample may, in some instances, distort assessment of cell concentration in a tested sample. For example, when the concentration of cells in the diluted sample is higher than a threshold value, multiple cells may pass through the sensor's aperture at the same time, a phenomenon sometimes termed "coincidence"; those skilled in the art appreciate that coincidence distorts results determined in assessments of cell parameters as described herein.

For example, if two red blood cells pass through the aperture of a sensor at, or nearly at, the same time, a measurement made by the sensor may not be able to distinguish between the two cells. This would result in an inaccurate number of red blood cells being counted, for example, when determining a complete blood count (CBC) or when determining certain other cellular attributes. Alternatively or additionally, two (or more) red blood cells may appear as one large red blood cell passing through a sensor. Accordingly, the volume of the measured "red blood cell" passing through the sensor would be assessed as abnormally large. As a result, the mean cell volume (MCV) of a sample would therefore be inaccurate.

Alternatively or additionally, in certain instances, a high cell concentration in a tested sample being passed through a sensor can result in a bias in the electronics. For example, some sensors require passage of a time interval of a certain minimum length between measurements. With a high concentration of cells and/or high flow rate of cells through a sensor, the sensor may not have sufficient time recover between measurements. This can lead to an inability to distinguish individual cells passing through an aperture of a sensor and/or an inaccurate measurement of a parameter of a cell, for example, a volume of a cell (e.g., of a red blood cell). The present disclosure teaches, among other things, that in certain embodiments it may be desirable to allow a longer recovery period between individual cells either through decreasing a flow rate to a sensor and/or decreasing cell concentration in a tested sample. In some embodiments, such strategies may allow electronics to return to their initial, baseline condition. Additionally, such strategies reduce the chance of a cell arriving during the recovery time. In certain preferred embodiments, more significant decreases in cell concentration prior to assessment of a sample, and/or analysis of a plurality of dilutions, may be preferable to changing (e.g., decreasing) flow rate to and/or through the sensor, as changing (e.g., decreasing) flow rate can result in a need for additional compensation factors and/or leads to additional variations in measurements being made by a sensor.

For example, if a first red blood cell passes through a sensor and a second red blood cell passes through the sensor later, but during the recovery period, this would produce an inaccurate determination of cell volume of the second red blood cell. The cell volume of the second red blood cell may appear larger or smaller (depending on the features of the sensor) than if it were to have passed through at a later period.

As described herein, testing of samples having a more uniform cell (e.g., red blood cell, white blood cell) concentration improves reproducibility and/or sensitivity of one or more measured parameters of cells and/or samples. For example, testing of samples having a uniform cell concentration has been determined to improve measurement of cell parameters, e.g., size (e.g., volume, diameter, radius) of cells. In other instances, reproducibility and/or sensitivity of numbers of cells counted by a sensor of an apparatus is improved.

In certain embodiments, biological samples (e.g., whole blood samples) are diluted prior to being passed through the sensor using the technologies described herein. In certain preferred embodiments, biological samples (e.g., whole blood samples) are diluted using a variable dilution ratio in order to achieve a particularly desirable, low concentration of cells passing through a sensor. In some embodiments, serial dilutions and/or continuous concentration gradients, may be utilized to achieve comparable and/or uniform cell concentrations in analyzed samples as described herein.

Without limitation, a more uniform cell concentration between samples of different test runs and/or over an individual test run, can diminish the need for the use of correction factors used to calculate one or more measured cell or sample characteristics or parameters. Correction factors are often employed to calculate cell parameters such as, without limitation, cell size (e.g., volume) and/or cell count. A value of one or more correction factors used may depend on a concentration of cells in a sample being passed to and/or through a sensor of an apparatus. Alternatively or additionally, one or more correction factors may be employed to improve calculations of a concentration of cells (e.g., red blood cells, white blood cells, platelets) within a sample. However, by passing a uniform concentration of cells to a sensor of an apparatus within a desired range of cell concentrations, one or more correction factors may not be required. In certain instances, this may be achieved by making variable dilutions of cells that produce uniform concentrations of cell populations with different samples being passed to a sensor, thus minimizing cell count and/or size measurement errors. In certain embodiments, the correction factors are substantially the same for all samples having a similar concentration of cells being passed to a sensor.

In certain embodiments, one or more artifacts produced by coincidence are minimized by passing cells (e.g., red blood cells, white blood cells, platelets) to and/or through the sensor at an approximately uniform, low concentration of about 400 cells/sec, 500 cells/sec, about 1000 cells/sec, about 2000 cells/sec, about 3000 cells/sec, about 4000 cells/sec, about 5000 cells/sec, about 6000 cells/sec, about 10,000 cells/sec, about 20,000 cells/sec or more. In certain embodiments a flow rate of cells to and/or through a sensor is about 5000 cells/sec. In certain embodiments, a uniform dilution is achieved through a variable dilution of a sample as described herein. A uniform cell concentration diminishes a need for correction factors as all samples are tested under substantially similar conditions.

Furthermore, the technologies described herein have the advantage of extending the range limits of cell concentrations of biological samples that may be automatically tested. Biological samples with very high or low cell concentrations, which fall outside traditional, automated testing limits, may be compensated for by actively varying the dilution ratio.

In some embodiments, technologies described herein are useful for screening, diagnosing, and/or monitoring subjects, e.g., based on red blood cell membrane permeability parameters, such as described in International Application No. PCT/US19/64543, filed on December 4, 2019. In some embodiments, technologies described herein are useful for identifying subjects who are relatively susceptible or resistant to malaria infection, as described in International Application No. PCT/US20/29114, filed on April 21, 2020.

## 1. Exemplified Two Syringe Sample Dilution System

In certain embodiments of technologies described herein, a uniform cell concentration within a tested sample passed to a sensor of a device is achieved by making an initial, standard fixed dilution of a biological sample with a diluent, counting a number of cells within a portion of the diluted sample by flowing the diluted sample and a diluent to a sensor, and then adjusting a dilution ratio between a diluent and biological sample to achieve a desired cell concentration. In certain embodiments, technologies described herein may be used with traditional cell analyzers to achieve a uniform concentration of cells when testing in a single environmental condition, for example, such as when measuring cell size or number or other common CBC (complete blood count) parameters. Common CBC parameters include: hematocrit, platelet count, red blood cell count, white blood cell count, hemoglobin concentration (e.g., in g/L), and mean corpuscular volume (mcv) and the cv (coefficient of variation) of the volume.

Preferably, dilution of biological samples and/or flow of samples to and/or through a sensor is carried out by an automated process using computer controlled, digital syringe pumps. Among other benefits, use of digital syringe pumps allows for highly accurate, automated dilutions of biological samples. Moreover, digital syringe pumps are dynamically adjustable in terms of their flow rates and/or delivery volumes. This flexibility allow for highly customizable dilutions of biological samples and precise control over sample environment. Rather than using fixed dilutions or having to manually prepare a wide range of diluted samples, customizable and real-time dilutions allow for biological samples of varying initial cell concentrations to be diluted without additional input and/or manual effort from a user. Thus, iterative processes of biological sample calculation and/or dilution as discussed and disclosed herein may be carried out in an automated manner.

In certain embodiments, a disclosed technology creates a uniform cell concentration by iterating through various diluent to biological sample ratios. For example, after diluting a volume of biological sample with a volume of a first diluent, a diluted sample is passed to a sensor of the device along with a second diluent. If a sensor determines that the concentration of cells of the diluted sample is not within at least 50%, at least 20%, at least 15%, at least 10%, at least 5%, at least 1%, at least 0.5%, at least 0.25% or less of a desired concentration, a ratio between the biological sample and either first and/or second diluent is adjusted to achieve the desired concentration. Preferably, a concentration of cells passed to the sensor differs less than 1% from the desired concentration.

In an embodiment, an initial assessment of the concentration of cells within a first diluted, biological sample being flowed to a sensor of an apparatus. If a desired concentration of cells as measured by the sensor is not achieved by the first diluted sample, a dilution ratio between the biological sample and the diluent is adjusted by either increasing the amount of diluent or sample to create a preferred concentration cells in suspension of the diluted sample. In certain embodiments as described herein, adjusting relative volumes of a sample comprising cells and a diluent is performed to achieve a desired cell count per unit time.

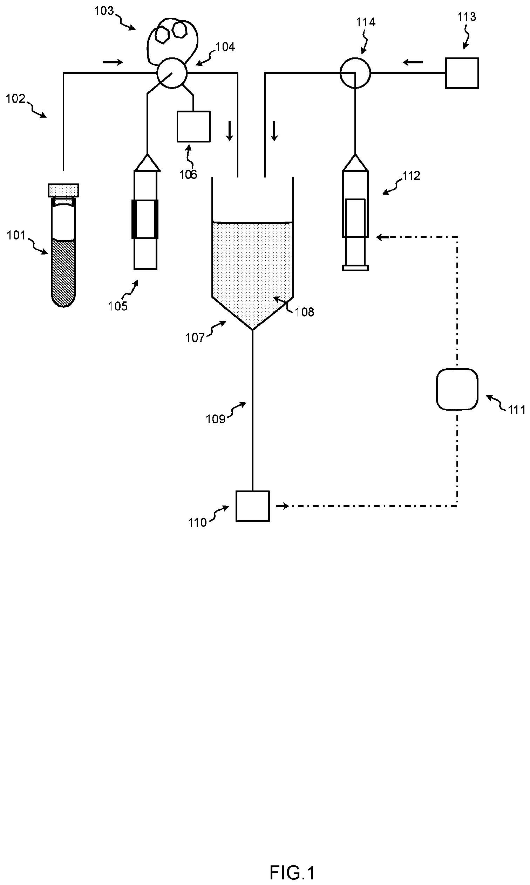

FIG. 1 depicts an illustrative schematic of an embodiment of an apparatus used to perform dilutions as described herein. A whole blood sample 101 is aspirated by syringe 105 through probe 102. In certain embodiments, one or more syringes as depicted are controlled by a high resolution syringe pump. A whole blood sample is drawn through sampling loop 103, which has a known interior volume. In certain embodiments, an interior volume of the sampling loop is about 1 $\mu$l, about 5 $\mu$l, about 10 $\mu$l, about 20 $\mu$l, or higher. A sampling valve 104 is then rotated and whole blood in sampling loop 103 is expelled into vial 107 by a known volume of diluent (e.g., a phosphate saline buffer, an isotonic liquid, a hypertonic diluent, a hypotonic diluent) from syringe 105 together with the sample, creating dilute suspension of cells 108. In certain embodiments, an initial dilution ratio of whole blood sample volume to diluent volume is about 1: 1000, about 1:2000, about 1:5000, about 1: 10,000, about 1:20,000, or higher. A dilution ratio of whole blood sample to diluent depends on the biological sample being analyzed and/or goals of the analysis being carried out. In certain embodiments, a diluent has the same tonicity (e.g., osmolality) as the cell sample. In other embodiments, a diluent is a hypertonic solution (e.g., a saline solution). In an embodiment, a suspension of diluted cells 108 has a higher cell concentration than a desired target concentration. In certain preferable embodiments, a preferable cell concentration passes about 5000 cells / second to a sensor of the device at a flow rate of 200 $\mu$l/sec (i.e., a concentration of about 25,000 cells/ml). In certain embodiments, a diluted whole blood sample (108) is at least 1.5x, at least 2x, at least 3x, at least 4x, or more concentrated than is desired for passing to a sensor. In certain preferable embodiments, a diluted whole blood sample is at least 2x more concentrated than is desired for testing. This allows for adjusting cell concentration before testing and running samples at a uniform cell concentration for all samples, so that the same (or a similar) number of cells are tested for all samples, regardless of an amount of cells (e.g., a percentage) passing through a sensor.

In addition, a volume of the diluted sample 108 is sufficient for multiple repeat tests, with a number of repeated tests being selectable before a dilution is made. In certain embodiments, a volume of diluted whole blood sample is at least 5ml, at least 10ml, at least 20ml, at least 50ml or more. In certain preferable embodiments, the amount of suspension tested in an individual test is at least 20ml.

A diluted whole blood sample 108 is then moved through tube 109 to a sensor 110, wherein measurements of the cells of the diluted whole blood sample are performed. In certain embodiments, a sensor measures impedance of the cells, cell count (e.g., total cell count, red blood cell count, white blood cell count), cell size, hemoglobin concentration, and other measurements indicative of the presence of a cell passing to / through the sensor (e.g., magnetic resonance, sound, optical (e.g., an image, a fluorescent signal), current, voltage, resistance, impedance, conductivity, light scattering and/or absorption). In certain embodiments, once a cell count has been determined by a processor 111, an additional volume of diluent 113 is added to a diluted whole blood sample 108 by adding a known amount of diluent from syringe 112, to reduce the concentration of cells in suspension 108. In another embodiment, an additional volume of the biological sample is added to suspension 108 by adding a known amount of a biological sample, to increase a concentration of cells in suspension 108.

In certain embodiments, a diluted biological sample 108 is tested additional times by passing diluted sample to a sensor 113 using other dilution ratios until a desired cell concentration of the sample being flowed to the sensor is achieved. In certain embodiments, a concentration of cells in the sample is determined from knowledge of a volume of blood 101 of the tested sample, diluent 110 required to create a target concentration of suspended cells 108, and a cell count as determined by the sensor 110. Syringe 112 and syringe 105 refill from stock diluent reservoirs 106 and 113 (respectively) when valves 104 and 114 are arranged to connect to diluent 106, 113. In certain embodiments, syringe 105 and syringe 112 can be combined as a single syringe.

Based on disclosure provided herein, it will be apparent to one of skill in the art that additional arrangements are possible using similar arrangements of automated sampling syringes.

**2. Exemplified Multi-Syringe System for Osmotic Gradients**

In some embodiments of technologies provided by the present disclosure, a concentration of cells in a biological sample is adjusted to a desired value by altering relative flow rates of biological sample and at one two other streams of liquid (e.g., one or more diluents). In an embodiment, one stream containing a cell suspension (e.g., either a dilute biological sample or an unadulterated biological sample) is mixed with a second stream containing a diluent without cells. As a combined flow rate of diluents and sample can be dynamically adjusted, concentrations of cells passed to a sensor may be adjusted without altering an environmental parameter, such as, osmolality, fluid pressure, or overall flow rate through the device.

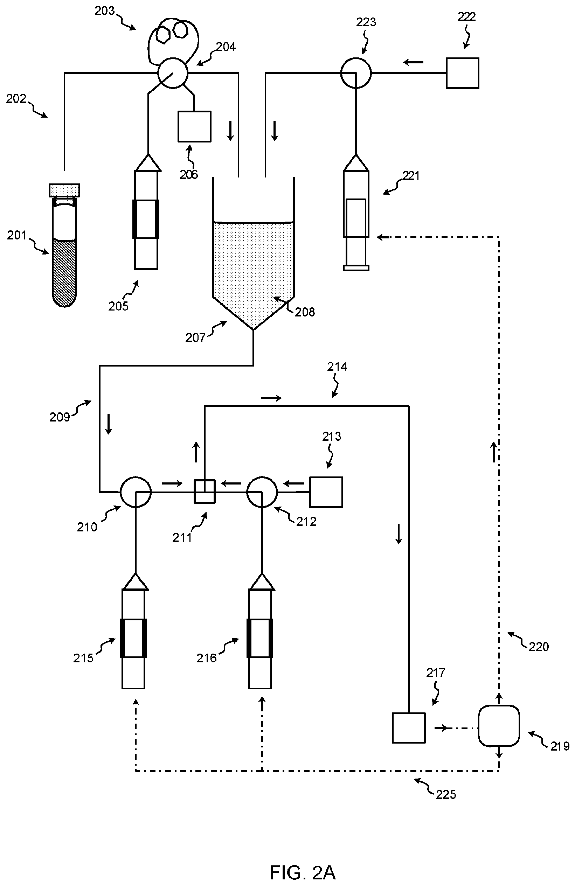

FIG. 2A depicts an illustrative schematic of an embodiment used to perform dilution methods as described herein. FIG. 2A shows an arrangement where, a whole blood sample 201 is aspirated by a first syringe 205 through a probe 202. Whole blood is drawn through sampling loop 203 which has a known interior volume, typically of a few microliters. In certain embodiments, an interior volume of the sampling loop is about $1\mu l$, about $5\mu l$, about $10\mu l$, about $20\mu l$, or higher. Sampling valve 204 is then rotated and whole blood in sampling loop 203 is expelled into vial 207 by a known volume of diluent from a first syringe 205 together with the sample, creating a dilute suspension of cells 208. In certain embodiments, a dilution ratio of the sample volume to the diluent volume is about 1:1000, about 1:2000, about 1:5000, about 1:10,000, about 1:20,000, or higher. In an embodiment, an initially created cell suspension 208 may have a cell concentration higher than the desired target concentration. In certain preferable embodiments, a preferable cell concentration passes about 5000 cells / second to a sensor of the device at a flow rate of $200\mu l$/sec (i.e., a concentration of about 25,000 cells/ml). In certain embodiments, a diluted whole blood sample 207 may be at least 1.5x, at least 2x, at least 3x, at least 4x, or more concentrated than is desired for passing to a sensor. In certain preferable embodiments, the concentration of cells in a diluted whole blood sample is at least 2x more concentrated than is desired for passing to a sensor. In certain embodiments, subsequent dilutions may be made in order to achieve the desired concentration of cells in the diluted biological sample.

Diluted whole blood sample 208 is then drawn into a syringe 215 through a valve 210. The valve connects the syringe 215 to mixing chamber 211. Sample is then passed at a known rate towards a sensor apparatus 217 via tube 214. In certain embodiments, a sensor apparatus comprises one or more sensors. In certain embodiments, a sensor apparatus comprises one or more sensors capable of measuring one or more parameters of cells in a sample (e.g., cell size, cell impedance, impedance of a cell, an amount of current passing through the cell, a voltage passing through the cell, hemoglobin concentration, an optical property, the number and/or density/concentration of cells in a sample). Concurrently, syringe 220 aspirates a diluent 210 towards sensor 212 through valve 221, which mixes with suspension 208 in mixing chamber 205. Diluted whole blood sample travels to the sensor apparatus 217 of the device via tubing 214. Properties of the cells passing through a sensor are measured as discussed elsewhere herein.

Once a cell count and/or cell concentration has been determined by processor 219, a ratio of flow of a fourth syringe 216 to a third syringe 215 is adjusted by processor 219 to either dilute or concentrate a stream of suspended cells until a desired cell suspension concentration is reached. In an embodiment of the device, flow through a sensor 217 may be maintained at a constant flow. In other embodiments, a flow rate to a sensor may be altered by a processor 219. A cell concentration is determined from the volume of whole blood 201 (sample), sample loop 203 volume, diluent volume 208, and flow rates required to create a target cell concentration. Syringes 221, 213, and 222 refill from one or more reservoirs of a stock diluent 206 when the valve 204, 210 and 212 are arranged to connect to diluent (e.g., 206, 213, and 222). In certain embodiments, a single syringe may replace syringes 205 and 221.

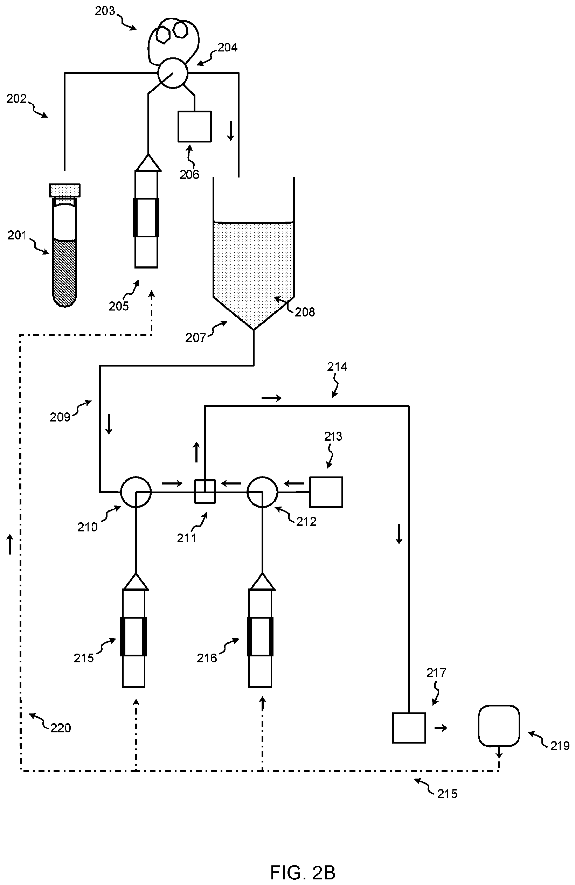

A feedback loop may be created by adjusting relative flow rates from a third syringe 215 and a fourth syringe 216 based on a measured cell count per unit time at a sensor. Altering the relative flow rates of the sample and diluent results the creation of a desired cell concentration. In an embodiment, a combined flow rate of fluid flowing from syringes is maintained at a constant rate by adjusting a ratio of flows of fluid from the syringes. In certain embodiments, a cell concentration of sample being flowed through a sensor can be dynamically altered during a test. Once a preferred ratio of flows between syringes are determined based on obtaining a desired cell concentration, repeated testing can be performed at a desired cell concentration without the need for further adjustments. In certain embodiments, syringe 105 and syringe 112 can be combined as a single syringe, as shown in Fig. 2B, which is otherwise similar to FIG 2A.

## 3. Multiplexed Sample Processing via Advanced Sample Preparation

For high throughput automated sample testing, high speed sample preparation and testing is critical. However, it may be desirable to wait a particular equilibration time such that cells in a sample may achieve a desired property, such as, a desired cell shape. Due to, in some cases, incubation times, high throughput testing of cell samples requires multiplex preparation of samples prior to testing. Accordingly, there is a need to improve the ability to test one or more cell parameters as discussed herein using multiplexed sample processing techniques.

In certain embodiments, diluted biological samples for testing may be created from whole blood samples within seconds (e.g., within 1 sec, within 2 sec, within 3 sec, within 5 sec, within 10 sec, within 20 sec, within 30 sec, within 60 sec). In certain embodiments, diluted biological samples may be tested within tens of seconds of creation or preparation (e.g., within 5 sec, within 10 sec, within 20 sec, within 30 sec, within 60 sec, within 80 sec, or more). Accordingly, automated preparation of multiple dilute biological samples is desirable to facilitate rapid testing of diluted biological samples.

Under some testing conditions, cell properties (e.g., cell shape, cell diameter, cell volume) of a population of cells to be observed (e.g., red blood cells, white blood cells) are altered when a diluent is added to a sample. These changes in initial cell properties take place over an equilibration period (e.g., about 1 minute, about 2 minutes, about 3 minutes, about 4 minutes, about 5 minutes, about 10 minutes, or about 30 minutes, or more). In certain embodiments, diluted biological samples comprising cells are tested only after an equilibration period in order to make an accurate evaluation of initial cell properties. Accordingly in certain embodiments, diluted biological samples (e.g., 108, FIG. 1; e.g., 208. FIG. 2A) are allowed to equilibrate for a period of time (e.g., at least 1, minute, at least 5 minutes, at least 10 minutes, at least 15 minutes, at least 20 minutes, at least 30 minutes, or more) before being tested. In a preferable embodiment, an equilibration time is about 5 minutes.

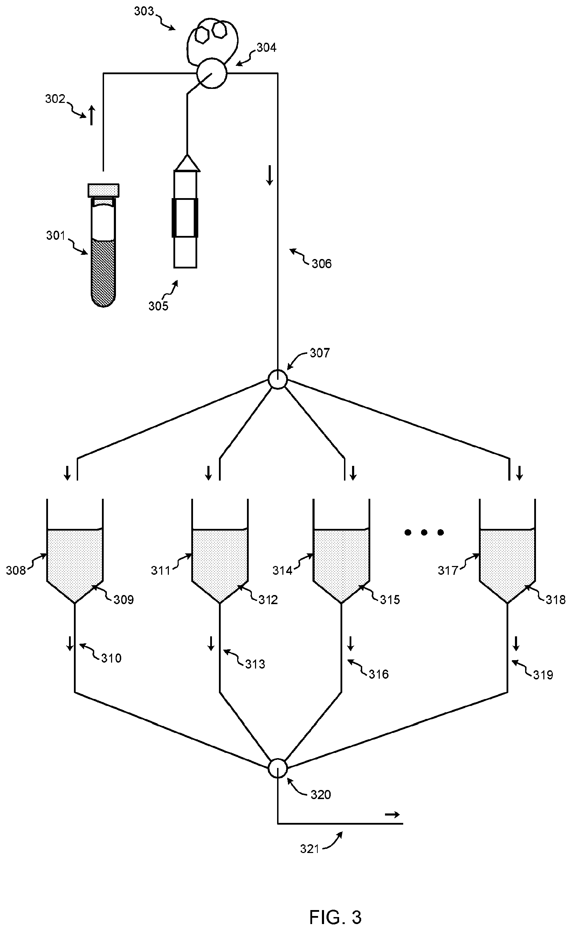

In certain embodiments, use of multiple equilibration containers may be employed to multiplex sample preparation. This allows for multiple samples to be prepared in parallel and enhance higher throughput testing. An embodiment of a multiplexed device is shown in FIG 3. FIG. 3 shows an exemplary schematic for a high throughput automated testing apparatus that allows for an equilibration of the cells of the diluted biological samples for a period of time (e.g., at least 5 minutes, at least 10 minutes, at least 15 minutes, at least 20 minutes, at least 30 minutes, or more) prior to subjecting samples to testing.

Through a process substantially similar to methods as used in embodiments as depicted in FIGs. 1, 2A and 2B, a whole blood sample 301 is aspirated by syringe 305 through probe 302. The whole blood sample is drawn through sampling loop 303. Sampling valve 304 is then rotated and the whole blood in sampling loop 303 is expelled through selection valve 304 into a suitable container (e.g., a vial), such as 308, 311, 314, etc. A selection valve 307 allows sample and a diluent (e.g., phosphate buffered saline) to be directed into any of the containers 308, 311, 314 or 317. In certain embodiments, there may be two containers. In certain embodiments there may be more than two containers (e.g., at least 4, at least 10, at least 20, at least 50, at least 100 or more). Once a diluted cell suspension has been created through an addition of diluent, a selection valve 307 directs the diluted sample to a different vial. In this manner, cell suspensions may be prepared, in advance of testing, at high speeds and allowed to equilibrate.

After the diluted biological sample has equilibrated for a pre-determined period of time as discussed herein, the diluted biological sample is drawn down tube 310, 313, 316, or 319 through selection valve 320 and then down tube 321 to the testing and sensing apparatus. The arrangement of FIG. 3 may be incorporated, without limitation, into the arrangements of FIGS. 1, 2A and 2B to provide for multiplexed preparation of samples being tested.

In certain embodiments, mechanisms for rinsing vials between samples are also employed to remove contaminants or residual sample from vials between testing.

## 4. Exemplified Method of Testing Through Adjusting Sample and Diluent Flow Rates

In certain embodiments, the technologies discussed herein provide for adjusting concentrations of cells in a biological sample tested in changing environments. A biological sample comprising cells is passed to and/or through a sensor along with one or more diluents. Flows of diluent and biological sample are adjusted relative to one another in order to modulate an environmental condition (e.g., pH, osmolality, temperature, reagent concentration, agent concentration) which cells are subjected to at a sensor, thus allowing for one or more cell parameters (e.g., cell shape, cell size, cell permeability e.g, cell volume) to be evaluated at one or more environmental conditions.

For a sufficiently robust statistical evaluation of cell parameters to be made without experiencing significant effects due to coincidence, at least 500 cells, at least 1,000 cells, at least 1,500 cells, at least 2,000 cells, at least 5,000 cells or more are evaluated by a sensor of the device per environmental condition.

In certain embodiments where osmolality of an environment is constantly changing, about 1000 cells are evaluated over each interval of osmolality which cells are subjected to. In certain embodiments, an interval is about 0.1 mOsm/kg, about 0.5mOsm/kg, about 1mOsm/kg, about 1.5mOsm/kg, about 2mOsm/kg, about 3mOsm/kg, about 5mOsm/kg, about 10mOsm/kg or more. In certain preferable embodiments, the osmolality of a sample passing to and/or through a sensor is decreased by 1mOsm/kg sec using water. In certain embodiments, a number of cells in a sample passing to and/or through a sensor is adjusted to a rate of about 500 cells per second, about 1,000 cells per second, about 2,000 cells per second, about 3,000 cells per second, about 4,000 cells per second, about 5,000 cells per second, about 6,000 cells per second, about 7,000 cells per second, about 8,000 cells per second, about 9,000 cells per second, about 10,000 cells per second or more. In certain preferable embodiments, a number of cells being passed to and/or through a sensor is about 5,000 cells per second. In certain embodiments, the environment may be a continuously changing with no distinct interval. In certain preferable embodiments, the osmolality of a sample passing to and/or through a sensor is decreased by 5mOsm/kg sec using water.

In certain embodiments, an overall flow of a sample and diluent (e.g., the combined flow rate) to the sensor is adjusted to ensure that a sensor is able to detect each cell individually. A flow rate of sample to a sensor is about 50 $\mu$l/sec, about 100 $\mu$l/sec, about 150 $\mu$l/sec, about 200 $\mu$l/sec, about 250 $\mu$l/sec, about 300 $\mu$l/sec, or more. In certain preferable embodiments, a flow of sample to a sensor is about 200 $\mu$l/sec.

In preferable embodiments of the device, flow to the sensor is about 200 $\mu$l/sec and osmolality of the diluted biological sample is adjusted in such that the osmolality of sample drops in a linear manner at a rate of about 5mOsm/sec. In preferable embodiments, about 5000 cells are evaluated per second.

## 5. Exemplified Embodiment of a Gradient Generating Apparatus

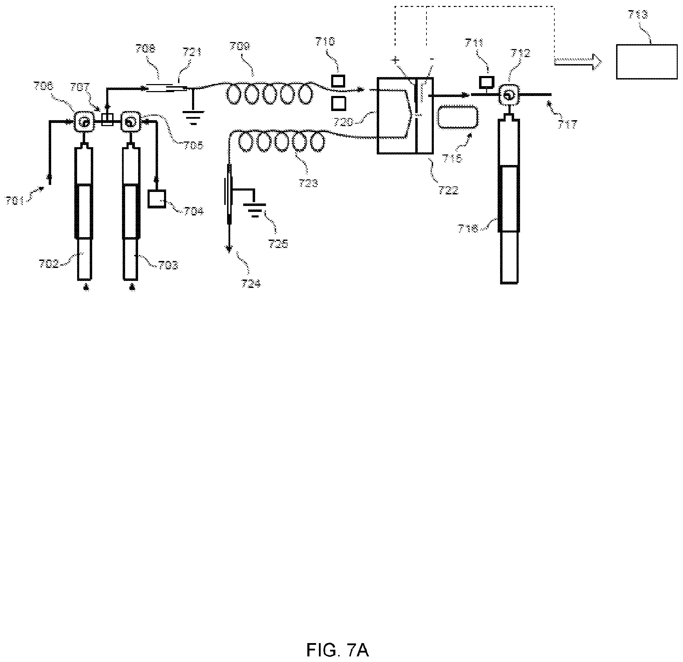

Embodiments of an apparatus for measuring one or more cell properties are depicted in FIGs. 7A-7C. FIGs. 7A-7C are illustrative schematics of improved devices for testing biological samples comprising cells (e.g., red blood cells, white blood cells, platelets) in a changing environment (e.g., osmolality, pH, temperature, reagent concentration, agent concentration).

In certain embodiments, osmolality of a sample is altered in a controlled way by combining the output of a first syringe 702 and second syringe 703. In certain preferable embodiments, one or more syringes are controlled by high resolution syringe pumps as described herein so as to produce highly accurate and/or controlled dilutions. A first syringe 702 contains a diluted biological sample comprising cells. In certain embodiments, diluent with which a biological sample has been pre-diluted with is nearly isotonic (e.g., having about the same osmolality as the biological sample). In certain embodiments, diluent with which a biological sample has been pre-diluted with is hypertonic, but once mixed with the diluent from syringe 703 will create a substantially isotonic suspension. A second syringe 703 contains a hypotonic solution, which may be refilled from a reservoir 704. In certain preferable embodiments, a hypotonic solution is water. Contents of two syringes are combined in mixing chamber 707 (e.g., a vortex mixing chamber) as flow rates from each syringe 702, 703 is controlled so as to create a gradient of tonicities from an output of the mixing chamber 707. In certain embodiments, a gradient of tonicities is a linear gradient. In certain embodiments, a gradient may be a custom gradient generated by a user. However, the system as described requires further modifications in order to maintain a consistent number of cells passing through a sensor in cases where flow to and/or through a sensor is desirable, but an environmental parameter being altered by the diluent is consistently changing. Accordingly, different syringe arrangements as discussed herein may be utilized to achieve a consistent flow of cells to a sensor.

## Exemplified Improved Multi-Syringe System

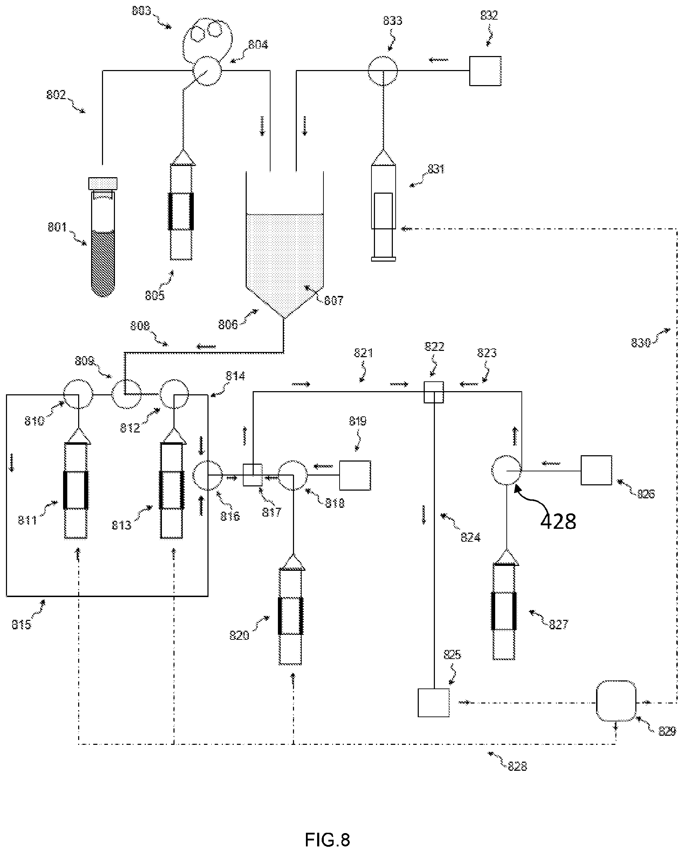

In an embodiment of technologies described herein, FIG. 8 shows an arrangement which is used to maintain a consistent number of cells being delivered to and/or through a sensor, while simultaneously varying an environmental condition (e.g., osmolality, pH) cells experience at a sensor of the device.

FIG. 8 represents an improvement over prior devices (e.g., WO 97/24529, US Pat. No. 6,422,0651) in that syringes are controlled via an automated, high accuracy syringe pump as described herein. Furthermore, a preferred number of cells being flowed to and/or through a sensor is kept constant over a given time interval during testing of an individual sample as an environmental condition (e.g., osmolality, pH, reagent concentration, agent concentration) is varied using a diluent. In addition, despite having, in some instances, biological samples starting with different cell concentrations, adjustments are made so that a preferred number of cells being flowed to and/or through a sensor per unit time is kept consistent between runs on different samples.

In a particular embodiment as depicted in FIG. 8, a sample syringe 813 contains biological sample, which has been diluted with a hypertonic diluent. A second syringe 820 contains a second solution with similar properties as the hypertonic diluent used to dilute the biological sample. When combined with a diluent syringe 827, which contains a hypotonic diluent, the arrangement creates a multi way-syringe gradient generator that can adjust concentrations and/or rates at which cells are being flowed to and/or through a sensor and allow for a dynamically changing sample environment.

A whole blood sample 801 is aspirated by syringe 805 through probe 802. In certain embodiments, the syringes as depicted in the schematic are controlled by a high resolution syringe pump. The whole blood sample is drawn through sampling loop 803, which has a known interior volume. In some embodiments, the interior volume is about $1\mu l$, about $5\mu l$, about $10\mu l$, about $20\mu l$, or higher. A sampling valve 804 is then rotated and the whole blood in sampling loop 803 is expelled into vial 806 by a known volume of hypertonic diluent from syringe 831 together with the sample, creating dilute biological sample 807. In certain embodiments, the sample 807 may be further diluted by syringe 831 expelling additional diluent into vial 806. In certain embodiments, an initial dilution ratio of whole blood sample volume to diluent volume is about 1:1000, about 1:2000, about 1:5000, about 1:10,000, about 1:20,000, or higher. A dilution ratio of whole blood sample to diluent depends on sample being analyzed and/or goal of an analysis. In certain embodiments, a diluent used to dilute the sample has the same tonicity (e.g., osmolality) as the sample. In other certain embodiments, a diluent is a hypertonic solution. In an embodiment, a dilute biological sample 807 has an initial higher cell concentration than the desired target concentration. In certain embodiments, a cell concentration of diluted whole blood sample (807) may be at least 1.5x, 2x, 3x, 4x, or more times more concentrated than is desired for testing using a sensor. In certain embodiments, a diluted biological sample may be further diluted by a second or subsequent diluent prior to testing. In certain preferable embodiments, a concentration of cells of the diluted whole blood sample is at least 2x more concentrated than is desired for testing using a sensor. In addition, a volume of the diluted sample (e.g., the diluted whole blood sample) 807 is sufficient for multiple repeat tests, with the number of repeats being selectable before a dilution is made. In certain embodiments, a volume of a sample is at least 5ml, at least 5ml, at least 10ml, at least 20ml, at least 50ml. In certain preferable embodiments, a volume of diluted whole blood sample is at least 20ml.

A diluted whole blood sample 807 is then moved through tube 808 to a syringe 813. A combined flow of syringes 813 and syringe 817, and their combined output is then mixed in a vortex mixing chamber 820 is mixed in a vortex mixing chamber 822 with fluid output by syringe 827. A combined flow rate of syringes 813 and syringe 820 decreases over time, while a flow rate from a syringe containing a hypotonic solution 827 (e.g.,, water) increases over time to create an osmotic gradient. The osmolality of a dilute biological sample being passed to and/or through the sensor typically is set to decrease in tonicity. A combined flow rate of syringes 813, 820 and 827 to a sensor is typically uniform. A combined flow rate to the sensor is about 50 $\mu l/sec$, about 100 $\mu l/sec$, about 150 $\mu l/sec$, about 200 $\mu l/sec$, about 250 $\mu l/sec$, about 300 $\mu l/sec$, or more. In certain preferable embodiments, a combined flow rate to a sensor is about 200 $\mu l/sec$. In certain embodiments, syringe 813 and syringe 820 typically move at about the same relative rate. In certain embodiments, a flow rate of syringes 813 and 820 combined is about 10%, about 20%, about 30%, about 40%, about 50%, about 60%, about 70%, about 80%, about 90% of an overall flow to a sensor with a remainder being provided for by syringe 827.

Once a cell count has been determined by processor 829, flow of sample from syringe 813 and flow of diluent from syringe 820 is adjusted by a processor 829 to dilute or concentrate cells in the sample being flowed to a sensor by increasing or decreasing the flow rate from each syringe 813 and syringe 820 without changing their combined flow rate. Adjustments are made until a desired number of cells passing to/through a sensor per unit time is reached.

In certain embodiments when there are too few cells passing through and/or to a sensor per unit time, flow of sample from syringe 813 is increased, while decreasing the flow rate from syringe 820. In certain embodiments, a flow rate of sample from syringe 813 is increased. In certain embodiments, flow rate of sample from syringe 820 is decreased.

Conversely, in certain embodiments when there are too many cells passing through and/or to a sensor per unit time, flow of sample from syringe 813 is decreased, while increasing flow from syringe 820. In certain embodiments, a flow rate of sample from syringe 813 is decreased. In certain embodiments, flow rate of sample from syringe 820 is increased.

In certain embodiments, syringe 811 may optionally be present to contain an additional volume of a sample (e.g., an additional volume of the same sample, a volume of a different sample) to facilitate more rapid and/or additional sample testing, for instance, syringe 811 can be refilling with a new sample before a test is complete.

A total number of cells in the original sample is determined based on a volume of biological sample (e.g., whole blood) used, volumes of diluents used, and flow rates from syringes 813, 820, and 827 required to achieve a desired number of cells flowing to and/or through a sensor per unit time. Syringes 805, 831, and 820 refill from one or more reservoirs containing a stock diluent (e.g., 819, 832) when valves 804, 833, and 818 are arranged to connect to the stock diluent. A stock diluent reservoir connected to syringe 805 is not shown. In certain embodiments, one or more syringes used to deliver diluent may be connected via a valve to a diluent reservoir. In certain embodiments, syringes 805 and 831 can be functionally simplified to being a single syringe, similar to the arrangement as shown in FIG. 2B.

## 6. Improved Dilution Method And Apparatus

US Pat. No. 6,422,065 describes testing cells in a changing environment. In a certain embodiment, a method as described by the prior patent uses two syringes, such that when flow from a sample syringe slows in order to alter tonicity (e.g., osmolality) of a sample being passed to a sensor, the number of cells passing through the sensor per unit time directly decreases in proportion to the sample syringes flow rate. Variation in the number of cells passing through the sensor per unit time causes errors in determining cell count and/or errors in determining cell size, which are similar to the errors for automated cell analyzers using fixed dilutions, as described herein. Accordingly, overcoming problems in determining cell count and/or cell size is desirable.

A response of a cell to its environment is not static. Furthermore, as described herein, red blood cells from different subjects will display a different maximum volume and/or spherical volume at different osmolalities (e.g., at differing Pk0's). In addition, different red blood cell numbers passing through a sensor over the same period of time have been found to also affect volumes of the cells measured by a sensor at different osmolalities. Accordingly, it is advantageous to create a system that is able to maintain a consistent number of cells passing to and/or through the sensor per unit time, while allowing for changes in environmental parameters (e.g., osmolality, pH) cells experience at a sensor. A uniform flow of cells eliminates the need for correction factors or repeated tests to adjust for variation in cell concentration, as explained herein.

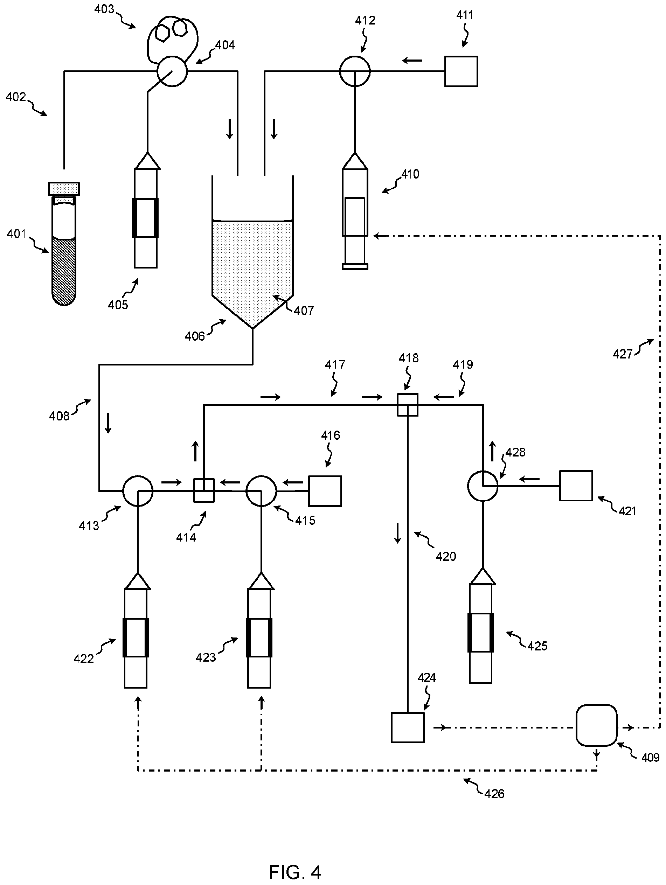

In a preferred embodiment, the technology is used to maintain good mixing and/or a uniform flow of cells to a sensor by introducing at least another syringe into a gradient generator system as described herein, as shown in FIG. 4. Thus a single syringe containing cells in a diluent is separated into a sample-containing suspension syringe 422 in which a sample has been diluted with, for example, a saline diluent and a sample-free saline syringe 423 containing only a hypertonic diluent (e.g., a hypertonic saline diluent) having no cells. In certain embodiments, both syringes contain solutions comprising the same tonicities. In certain embodiments, both syringes contain solutions having different tonicities. Sample from a sample-containing syringe 422 and diluent from sample-free syringe(s) 423 are mixed so as to act as a single syringe when creating a gradient. In certain embodiments, a flow out of one of the syringes does not exceed the flow of the other syringe by more than 4x, more than 10x, more than 20x or more. In certain embodiments, the syringes may be of different sizes (e.g., volumes).

In a certain embodiment, a syringe containing a diluted sample 422 (i.e., a sample syringe) is set to expel fluid at a constant rate of 10% of total flow of fluid to a sensor 424 of an apparatus. In order to maintain a uniform concentration of cells, a second syringe 423 (i.e., the hypertonic syringe) is set to expel a hypertonic fluid at an initial rate of 70% of total flow to a sensor 424. A third syringe 425 (i.e., the hypotonic syringe) is set to expel a hypotonic fluid at an initial rate of 20% of total flow to a sensor 424. As the system, in this example, is set to decrease tonicity of the cell environment as the sample passes through a sensor of the apparatus, the flow from the hypertonic syringe 820 is set to decrease in relative proportion to the overall flow, while the relative proportion of hypotonic syringe 827 is set to increase. Thus, by the end of the test, the relative flow of the sample syringe still remains at 10% of the total flow to a sensor of the apparatus, while the relative flow of the hypertonic syringe 820 decreases to 10% and the relative flow of the hypotonic syringe increases to 80% of the total flow.

As described herein, the embodiment of FIG. 4 is very similar to an arrangement described herein regarding a three-syringe gradient generator, except the two saline syringes 422 and 423 move at a variable rate and not at a fixed ratio.

In a preferred arrangement, a whole blood sample 401 is aspirated by syringe 405 through probe 402. Whole blood sample is drawn through sampling loop 403 which has a known interior volume, typically of a few microliters. Sampling valve 404 is then rotated and whole blood in sampling loop 403 is expelled into vial 406 by a known volume of diluent from syringe 405 together with the sample, creating dilute suspension of cells 407.

A dilution 407 is then drawn into syringe 422 through valve 413. A valve 413 subsequently connect syringe 422 to mixing chamber 414 and then aspirated at a known rate towards a sensor 424 via tube 417 and tube 420. A sensor may perform one or more measurements of the sample or cells therein, including, but not limited to, the cell count, cell size, cell shape, and hemoglobin concentration. Concurrently, syringe 423 expels diluent 416 towards a sensor 424 through valve 415, which mixes with suspension 407 in a mixing chamber 414. This diluted suspension travels to sensor 424 via tubing 414.

A combined fluid flow output by syringes 423 and syringe 422 is mixed in a mixing chamber 418 with an output of hypotonic diluent syringe 425. In certain embodiments, the mixing chamber 418 is a vortex mixing chamber. In certain embodiments, a combined flow fluid from syringe 422 and syringe 423 decreases over time, while flow from syringe 425 increases over time. Such an arrangement may be use create a gradient of environmental conditions (e.g., an osmotic gradient).

An osmotic gradient created using the aforementioned arrangement typically decreases in tonicity from 80% saline and 20% water to 20% saline and 80% water. A combined flow rate of syringes 422, 423 and 425 is typically uniform. Syringe 425 and syringe 423 typically move at different relative rates, while a sample syringe 422 expels sample at a constant rate. In a preferable embodiment, syringe 425 and diluent reservoir 421 are set to contain water.

Once a cell concentration has been determined by processor 409, flow from syringe 422 and syringe 423 is adjusted by processor 409 to dilute or concentrate suspended cells passing to a sensor without changing the combined flow rate, until a desired cell concentration is reached. When a sample is too dilute, either flow from syringe 422 is increased and/or flow from diluent syringe 423 is decreased. Conversely, when the sample is too concentrated, either flow from syringe 422 is decreased and/or the flow rate from diluent syringe 425 is increased.

In certain preferable embodiments, a combined flow rate from all three syringes is held constant. The combined flow rate to the sensor is about 50 $\mu$l/sec, about 100 $\mu$l/sec, about 150 $\mu$l/sec, about 200 $\mu$l/sec, about 250 $\mu$l/sec, about 300 $\mu$l/sec, or more. In certain preferable embodiments, a combined flow rate to a sensor is about 200 $\mu$l/sec. In certain embodiments, a flow rate from a suspension syringe 422 is adjusted to achieve a target concentration of cells and/or the cell count per unit time. To make up for a change in syringe 422 flow, syringe 423 is adjusted so that a combined output of syringe 422 and syringe 423 does not change. Creating a gradient of tonicities is achieved by decelerating flow from syringe 423 and accelerating the flow from syringe 425, thus making a three-syringe arrangement to create a local environment that is improved over the prior art in that a constant cell count per unit time and/or concentration of cells passing through a sensor may be maintained when using different mixture gradient profiles, e.g., tonicity gradient profiles.

In certain embodiments when syringe 422 is a dilute mixture of a biological sample comprising cells and a hypertonic saline diluent, there is an even distribution of cells in the flow from syringe 422. A sufficient flow from both syringe 422 containing a dilute sample and syringe 423 containing a cell-free hypertonic diluent allows for uniform and efficient mixing. Combined flow from the mixing chamber 414 decreases in tonicity, but not cell count as the flow rate from the syringe 425 comprising water increases. The flow from mixing chamber 414 is combined with flow from the water syringe 425 in mixing chamber 418 whose output travels to sensor 424 via tube 420. The combined flow rate is substantially constant as mentioned previously and the number of cells passing through the sensor per unit time is determined as described herein.

In certain embodiments, order of mixing diluents and sample may be important. Without wishing to be bound by theory, it is proposed that decreasing the difference between the diluent and sample being mixed enables more thorough and complete mixing of the diluent and sample. For example, in one embodiment whole blood is mixed with a hypertonic diluent, creating a suspension of about 1: 5,000, about 1:10:000, or greater (whole blood : hypertonic diluent). During a test with a constant cell concentration or number being passed to a sensor, this biological sample is then mixed with two other diluents. First, the biological sample is mixed, using a device as described herein, with the same hypertonic diluent to maintain the cell count by varying a flow ratio from two syringes. This approach ensures that cell number can be controlled accurately and ensures even mixing. A third syringe containing a hypotonic diluent such as water can be used to create a suspension with a falling osmolality. By varying flow rates of all three syringes, a uniform cell concentration can be maintained as osmolality to which cells in the biological sample are exposed is decreased as it passes to a sensor of an apparatus described herein.

In some embodiments, this sequence can be beneficially rearranged to improve the mixing of the osmotic gradient. Rather than mix the diluents in sequence as described above, a diluent without cells and water are first mixed. This can be performed using a syringe system as described herein. This combined cell-free suspension is then mixed with the suspension containing a biological sample. This mixing procedure creates exactly the same osmolality and suspension concentration as before, but with improved mixing by minimizing the osmotic difference of the biological sample containing cells and water. As discussed herein, fluids with very different osmolalities do not mix as efficiently as fluids with similar osmolalities, and by minimizing the difference between the biological sample's osmolality and the gradient-inducing diluent, better mixing is achieved. Further, pre-mixing diluent effectively lowers the osmotic difference between a biological sample and a diluent, thus minimizing the risk of osmotic shock of prematurely rupturing cells that mix with water.

### Additional Exemplified Multiple Syringe Arrangements

In other embodiments, different syringe arrangements are possible in order to achieve other testing goals as set forth herein. To further expand the range of environments that can be tested, additional independently controlled, high resolution syringes with additional diluents may used. In order to enhance testing speed, these syringes may be refilled in a manner that is optimized to minimize delays in testing. In certain embodiments, the syringes may be refilled during a test.

Such embodiments may be flexible and useful in allowing, without limitation, non-linear gradients, expanded or contracted range of osmolalities, increased the ranges of sample or environmental parameters the test, increased sensitivity in testing for diagnostic applications and those applications mentioned herein. Furthermore, gradients with which samples may subjected to can be created using drugs or other agents as described herein. For example, soluble components of prostheses (e.g., artificial heart valves) have been found to cause hemolysis. This allows a toxic effect to be found during development of a prostheses rather than as a side effect after implantation.

In certain embodiments of the technology described herein, multiple syringes containing different diluents that are of pragmatic or experimental interest are employed to determine the response of cells (e.g., red blood cells, white blood cells, platelets) as measured by a sensor of an apparatus. For instance, there are many agents that are known to interfere with the cell pore function and the cell permeability. Accordingly, using additional syringes with dilute suspensions of agents including, without limitation, drugs that effect some property of the cells, it is possible to evaluate quantitatively and/or qualitatively the responses of cells to multiple environments. In certain embodiments, cells in the sample may be subjected to different environmental conditions either sequentially or simultaneously.

For instance, by adding a pore blocker such as mercuric chloride (i.e., $HgCl_2$) to a diluent syringe and slowly subjecting cells of a sample to an increasing concentration of the pore blocking agent, a response of the cells (e.g., changes in cell size and/or cell shape) to the increasing concentration of the pore blocker can be measured by a sensor. In certain embodiments, the sample may also be exposed to changing osmotic gradients.

Similarly, many sugars also have been found to have an effect on cell permeability, such as L- and D-fucose, glucose, and rhamnose. In certain embodiments, a diluent used to create a gradient over time in the cell test environment may comprise one or more of such agents. An effect of agents on cell parameters (e.g., cell shape, cell size) may be monitored as one or more other environmental parameters (e.g., osmolality) are changed.

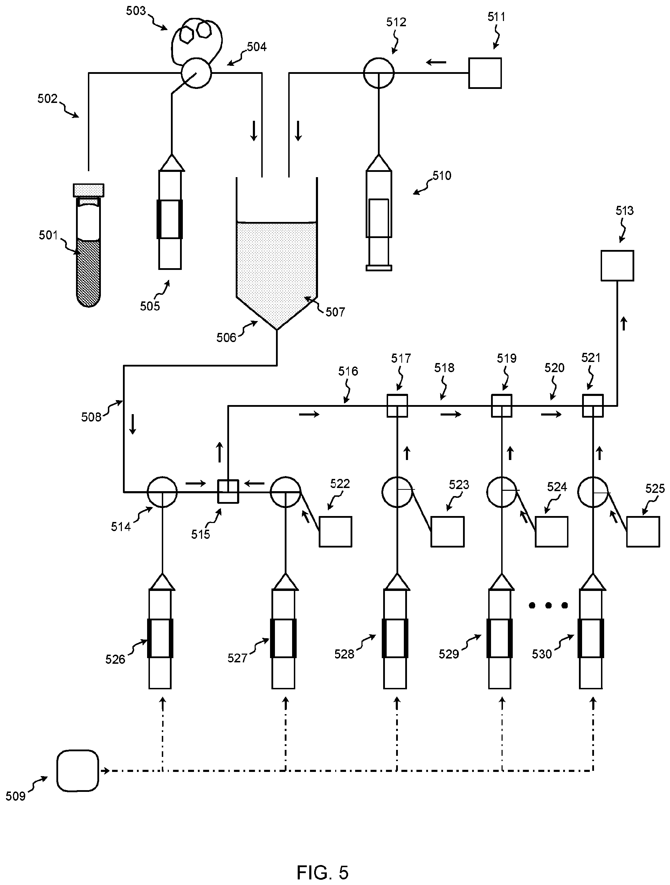

In an embodiment, FIG. 5 shows one arrangement with multiple syringes where a whole sample of blood 501 is diluted and delivered to a sample syringe 526. This is combined with a sample-free syringe 527 containing a first diluent through mixing chamber 515 and sent to sensor 513 via tube 516. Additional diluents with agents from syringes 528, 529 and 530 can be selectively added to the flow through mixing chambers 517, 519 and 521. Syringes 527, 528, 529, and 530 with diluents having one or more agents each have their own diluent reservoirs 523, 524, 525 and 522. In certain embodiments, additional syringes or agent reservoirs may be employed. Each syringe is independently controlled via controller 509. Syringes 526, 527 and 528 can also be arranged to create a three-syringe gradient generator with a constant cell count, as described herein.

## 7. Exemplified High Resolution Digital Syringe Pumps

Digitally controlled syringes (often referred to as syringe pumps) are common in clinical automated instrumentation. However these standard digital syringe pumps are noisy and/or are of low resolution, which negatively affects measurements made by many of kinds of sensors, as used herein. Accordingly, in embodiments, high resolution syringe pumps are used to control fluid flow. Such high resolution, digitally controlled syringe pumps as described below may be employed in any arrangement described herein.

In certain embodiments, a syringe pump system as described herein may be used to replace conventional predetermined cam profiles discussed in a prior patent US. Pat. No. 6,070,476, which is incorporated by reference in its entirety. Series of syringes whose positions and/or velocities are adjustable and digitally controlled through computer controlled movements, with real-time position information and position calculations may be used. Using digitally driven systems has many advantages over existing methods, particularly when generating gradients (e.g., osmotic gradients, agent gradients) as gradients may be determined dynamically based on a user's need and/or sample requirements during a test being conducted.

For example, digitally driven syringes allow for a user or system to select start and end osmolalities of a sample passed to a sensor. Additionally, digitally driven systems allow for the user to generate arbitrary, custom gradient profiles other than linear gradients. In certain embodiments, custom gradients may include, without limitation, a curvilinear gradient.

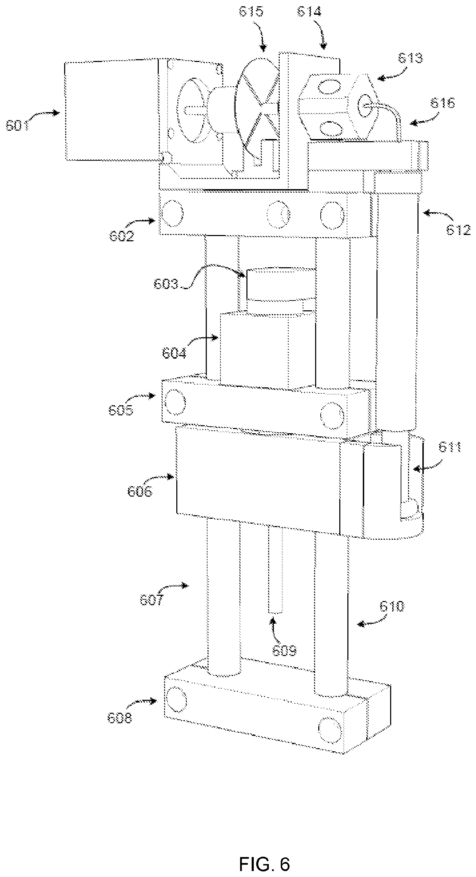

In an embodiment, FIG. 6 shows an arrangement of a digitally driven system. The system is comprised of digitally controlled motors 604 that drives a lead screw 609 that transmit its force to a syringe plunger 611 via slide 606. In certain embodiments, the syringe pump comprises a brushless DC motor 604 with a high-resolution optical encoder 603. A fine lead screw 609 moves a slider 606 and subsequently piston 611 of syringe 612 to aspirate or eject fluid. In certain embodiments, the fine lead screw has a pitch of about 3mm, about 2mm, about 1mm, about 0.5mm, about 0.25mm, or less. In certain preferable embodiments, the fine lead screw has a pitch of about 1mm. A combination of these mechanical elements creates a syringe pump that moves with minimal mechanical noise over a wide range of speeds. A reduction of vibrations and/or mechanical noise is desirable as many sensors used are sensitive to vibrations.

In certain embodiments, high resolution syringe pumps are used to reduce mechanical noise over prior systems. In certain embodiments, mechanical noise is dampened by tuning a brushless DC (direct current) motor so that magnetic poles just balance its rotational movement. Motor speed is adjusted about every 15μsec, about every 10μsec, about every

5μsec or less based on an optical encoder's reported position in order to achieve this. In certain preferable embodiments, motor speed of a brushless DC motor is adjusted about every 10μsec. Certain combinations of higher encoder resolution and/or finer lead screw pitch lead to higher resolution, but may not necessarily reduce vibrations (e.g., by making the motor quieter).

In certain embodiments, a high resolution optical encoder used is a digital 10,000 pulse per revolution encoder (PPR). Such an encoder provides for about 90 revolutions per 5ml stroke of the syringe. This gives about 900,000 selectable positions over the entirety of a full stroke. An encoder emits 20,000 selectable positions, and may, in certain embodiments, lead to about 1.8M positions per full stroke using the full encoder resolution. In combination with increasing the pulley ratios and/or decreasing the lead screw pitch, further resolution may be gained. In certain embodiments, a resolution of an encoder is about 10 arcminutes, about 5 arcminutes, about 4 arcminutes, about 3 arcminutes, about 2 arcminutes, about 1 arcminute or less. In a certain embodiment, a resolution is about 2 arcminutes when an encoder is being run at 10,000 pulses per revolution. In other embodiments, 1 arcminute or less is preferable.

Fluid enters or exits the syringe pump via a four-way valve assembly comprising a stepper motor, 601 position sensor disc 615 mounting bracket 614 and 4-way distribution valve 613 and connecting tubing 616. In an embodiment, a precision lead screw 609 and high-resolution encoder 603 create about 600,000 divisions for a typical full stroke of a precision glass syringe with a travel of approximately 60mm. Accordingly, this enables position selection of a syringe to within about 200nm, about 150mn, 100nm, about 50nm, or less. In certain embodiments when used with a 5ml syringe, the syringe is able to eject and/or aspirate volumes of about 8.33 nanoliters per addressable position. Control over nanoliter scale volumes enables very high precision when creating a gradient from mixing fluids output from two or more syringes controlled by high resolution syringe pumps.

A position of a syringe is sensed by closed-loop encoders 603 within a motor assembly 604. In certain embodiments, a syringe position may be controlled by an open-loop recording of a distance the motor has been instructed to move. In other embodiments, a number of steps taken for a stepper motor may be used to control syringe position. In other embodiments, other open loop drive techniques may be employed to control the syringe position.

In an another embodiment, a motor 604 and lead screw 609 are coupled by a belt and gear with a drive ratio of about 2:1, about 3:2, about 3:1, about 4:1, about 5:1 or higher and, accordingly, increases a resolution. In a preferable embodiment, the belt and gear drive ratio is about 3:1, which increases the resolution of the motor by a factor of 3. In another preferable embodiment, the belt-to-gear drive ratio is about 3:2.

In certain embodiments, precise movements of a syringe and/or flow rate regulation of a fluid stream to be mixed can be achieved with direct pressure regulation of fluid streams. In certain embodiments, piezo-electric actuators, inductive magnetic drive, pulsing pumps (e.g., micro system fluid delivery) or other systems may be employed to control fluid flow rates. For any system described herein, high resolution (e.g., small step increments) and smooth and/or silent motor movements are preferred as sensors employed in some embodiments are sensitive to vibrations due to mechanical shock or acoustic waves. Typically, stepper motors used in standard syringe drives generate such vibrations at some speeds, and as the system uses variable or multiple speeds, stepper motors generally create mechanical noise during some part of the test in its typical arrangement. Accordingly, in certain embodiments brushless DC drives, direct voice coil, piezo-electric or ultrasonic drives are preferred for their near silent movement and high resolution.

In a certain embodiment of a prior method as disclosed in U.S. Pat. No. 6,070,476, a fixed cam profile is used to create a linear gradient of osmolalities over which a biological sample comprising red blood cells is tested. Generally, a test is conducted at a single, uniform linear osmotic gradient. A single test takes about one minute with about 1,000 red blood cells per mOsm/kg being measured. However, some parts of resultant data, particularly around the point where a red blood cell achieves a spherical shape, change greatly over 1 mOsm/kg or less. Moreover, the point at which the red blood cell achieves this shape (i.e., the Pk0) varies between individuals and samples. Accordingly, using prior methods, one way to reveal more detail around these or other selected osmolalities, is to run a test using a different linear osmotic gradient, which changes the osmolality at a lower than desired rate. However, running a test at such a rate reduces a number of tests that can be performed per sample and/or may reduce a range of osmolalities tested. In addition, additional profiles and/or syringe arrangements may be required that would add to the complexity of the system.

Accordingly, use of digital syringes enables a user to more closely analyze regions around the osmolality at which a red blood cell achieves a spherical shape (i.e., the Pk0). By using digital syringes, the osmolality at which a red blood cell achieves this shape can initially be rapidly determined from a standard test covering the complete range of osmolalities, or a range where the peak normally occurs. A gradient can then be changed to test a more narrow range of osmolalities of interest for a much longer period, allowing more accurate data collection and higher resolution measurements of the cell size and osmolality at the peak from additional sample measurements during the longer time of testing. By setting a gradient to only cover a narrow range of osmolalities around this point, preferably after an initial test to locate a peak osmolality (i.e., Pk0), it is possible to increase the resolution of the sphering point by one or two orders of magnitude. In certain embodiments, a narrow range of osmolalities covered is about 20mOsm/kg, about 10 mOsm/kg, about 5 mOsm/kg, about 4 mOsm/kg, about 3 mOsm/kg, about 1 mOsm/kg, about 0.5 mOsm/kg, or less. Very small changes in the osmolality of a sphering pressure are clinically and/or diagnostically useful in diagnosing health issues. Accordingly, increasing sensitivity and/or number of samples gathered around this point is inherently useful. Conversely, some samples have a very broad range of sphering pressures, and concentrating or compressing this range of osmolalities by increasing the rate at which an osmolality gradient changes allows for a more rapid test to be conducted. Other areas of interest, such as the point of maximal fluid flow into or out of a cell (e.g., as measured by "Pymin" and "Pymax") and/or any other region of interest may be examined using this technique.

It is advantageous to slow down a test (e.g., slow a rate of change of the osmolality) during any period to allow for increased sensitivity and/or accuracy of data over a desired parameter range. For example, in a sample where red blood cells have been found to have a peak osmolality of 140mOsm/kg, an environment can be automatically adjusted to test only osmolalities in the range of 135 to 145 mOsm/kg. By running a test over this narrow range with a low rate of osmolality change, a resolution of the test may be increased by 20 times. Without wishing to be bound to any particular theory, such an increase in resolution is important as small shifts of 1-2mOsm/kg at which cells achieve a spherical shape signals the onset disease. For example, such small shifts in osmolality have been determined to be indicative of, in combination with other factors, carcinoma of the pancreas, hemolysis, and ovulation, among other diseases. Increased resolution is also clinically useful as even smaller shifts (e.g., about 1 mOsm/kg, about 0.5 mOsm/kg, about 0.1 mOsm/kg, or less) detected with this method offers the opportunity for even earlier detection of diseases and/or conditions.

As a further advantage of using a digitally controlled syringe system, fluid consumption is minimized for flushing and washing, as syringes are independently controlled. For instance, a single syringe that contains, for example, a biological sample or expired diluent can be rinsed without moving the other syringes. Similarly, reset and refill times, which effect cycle throughput, are reduced as syringes can be independently refilled or rinsed, allowing for optimization of the independent syringe movements and to reduce diluent waste related to having to empty a syringe due to resetting the system.

Moreover, digitally controlled and driven syringe pumps confer many advantages for high-throughput applications over the prior technologies discussed herein. Smaller volumes of samples containing higher concentrations of cells may be used. Accordingly, use of smaller volumes creates fewer contaminated, wetted surfaces. Furthermore, smaller volumes of samples minimizes cross contamination.

## 8. Improved Measurements Related to Diluent Modification

In certain embodiments of the technology discussed herein, cell size under isotonic conditions is determined by measuring cells (e.g., red blood cells) under environments which match the isotonic osmolality of the biological sample. This may be achieved by, without limitation, measuring the osmolality of the biological sample and adjusting osmolality of a diluent and/or a testing environment to match the osmolality of the biological sample. For reference, most automated analyzers test cells in hypertonic solution between 30 and 60 mOsm above isotonic.

As disclosed herein, embodiments of the technologies using syringe systems allow for the generation of gradients that can be used to create a wide range of osmotic gradient profiles including, without limitation, those profiles disclosed in certain embodiments of U.S. Pat. No. 6,070,476 and U.S. Pat. No 6,422,065. However in prior technologies, the isotonic osmolality and, therefore, the molality of the diluents used to dilute the biological sample was presumed to be a fixed value for all subjects and samples. For the majority of biological samples, an isotonic osmolality varies between biological samples and subjects. Accordingly, the osmolality of each biological sample is evaluated prior to testing in order to accurately determine the isotonic osmolality and, accordingly, adjust the osmolality of a diluent and/or testing environment.

In a certain embodiment, US Pat. No. 6,668,229 taught how to make red blood cell volume and shape measurements by monitoring changes in volume of a population of cells as osmolality of cells' environment changed. Varying osmolality of a sample was necessary to finding the osmolality at which the cells achieved a spherical shape (i.e., the Pk0). Then, a shape of red blood cells at the isotonic osmolality may be determined by using a sample specific shape correction factor.

For example, a spherical volume of a cell that is achieved by the cell at the Pk0 can be calculated using a measured property (e.g., a voltage) of a cell. The spherical volume of a red blood cell can be calculated using the following equation:

$$\text{Spherical volume} = \text{measured voltage} \times K_{volts}$$

Wherein $K_{volts}$ is a correction factor that is determined for the particular instrument tested and stored in the memory of the instrument. The measured voltage would be obtained by a sensor of the instrument. It would be understood by one of skill in the art how to obtain the correction factor ($K_{volts}$) for a particular instrument.

In certain embodiments where the spherical volume is determined at the Pk0, the isotonic volume is determined by the equation:

Volume$_{iso}$ = Voltage$_{iso}$ x K$_{volts}$ x K$_{shape}$

where Voltage$_{iso}$ is the measured voltage at the Pk0 and K$_{shape}$ is a shape correction factor. It would be understood by one of skill in the art how to obtain the correction factors.

In certain embodiments, adjusting osmolality of a diluent to match the in vivo osmolality of blood and/or a measured plasma osmolality is achieved adjusting flow rates and/or volumes from sample and diluent syringes to create a desired isotonic osmolality from which an in vivo cell size (e.g., volume) and/or shape is determined. In certain embodiments, as a large range of cell sizes are can be measured using a large range of osmolalities, the cell shape corresponding to the in vivo plasma tonicity can be identified and used for subsequent cell parameter calculations. In certain embodiments, plasma or serum may be used as the "isotonic" diluent.

As discussed previously above, a certain embodiment of the prior patent is used to determine cell volume from an isotonic cell size measurement with a dynamic shape correction factor, which was an improvement on existing fixed shape correction factors. An embodiment of the present invention further improves this technique by using a measured, rather than assumed, isotonic tonicity value. This increases the accuracy of automated sizing by removing artifacts related to diluents inducing a change in the cell volume/or size from suspending and/or testing cells in an assumed in vivo isotonic solution, revealing previously undetected diseases and abnormalities as described in International Application No. PCT/US 19/64543, filed on December 4, 2019 and International Application No. PCT/US20/29114, filed on April 21, 2020. For example, a large increase in spherical cell size (i.e., cell volume) of red blood cells can be detected in women during labor as compared to during the term of the pregnancy. All but one woman with high blood pressure in pregnancy had normal or low spherical sizes in comparison with the non-pregnant hypertensive, all of whom had values above the mean.

Further, this technique can be used when testing cells using standard automated sizing equipment where no gradient is used. Sample tonicity (e.g., osmolality) may be adjusted to match the in vivo tonicity of a biological sample (e.g., the whole blood sample) by automatically adjusting the osmolality of a diluent. In certain embodiments, an osmolality of the isotonic diluent is less than 5mOsm/kg, less than 4mOsm/kg, less than 3mOsm/kg, less than 2mOsm/kg, less than 1mOsm/kg, less than 0.5mOsm/kg, less than 0.25mOsm/kg, less than 0.1mOsm/kg, less than 0.05mOsm/kg or less different than the osmolality of the biological sample.

### 9. Exemplified Embodiments of Electrical Noise Reduction Implementations

For many cell and particle sizing techniques, an electrical pulse at a sensor is made as a particle passes through a sensor, and subsequently, the recorded pulse is used to determine a size of a particle. However sources of noise such as vibrations and/or electrical fields distort the recorded pulse size. Accordingly, it is preferable to minimize electrical, vibrational, and/or other sources of interference at a sensor to improve readings.

In existing techniques such those employed in modem analyzers, electrical noise is a small problem as a tested sample suspension is electrically isolated from a conducting path. Testing sample suspensions used in such techniques are good conductors, and suspending sample suspensions minimizes electronic and/or other sources of noise that eventually appear as electrical noise.

In embodiments of the present apparatus, there is a continuous fluid path between a sensor and syringes. A continuous fluid path creates an electrical path for conduction of stray electrical signals. Furthermore in certain embodiments, a sensor also becomes increasingly sensitive to mechanical and/or electrical noise. Sensors are particularly sensitive when subjected to low osmolality testing environment which does not typically occur in traditional cell analyzers that test cells above isotonic values. Accordingly, in order to obtain accurate results over the range of osmolalities tested, it is important to develop and implement improvements where electrical, mechanical, and/or other sources of noise are minimized. One such improvement is use of external shielding with a metal enclosure around incubation tubing used to carry sample from a sampling unit comprising the osmotic gradient generator to a sensor.

In embodiments as discussed herein, FIGs. 7A-7C may be used to show improvements to the electrical shielding of the apparatus. In certain embodiments, electrical shielding 721 comprises a segment of tubing surrounding at least a portion of the incubation tubing and in contact with the tubing's contained sample. In certain embodiments, the segment of tubing is or comprises a conductive metal. In certain embodiments, the conductive metal is or comprises stainless steel. In certain embodiments, the conductive metal is or comprises an inert or precious metal (e.g., gold, platinum). In certain embodiments, the segment of tubing is about 1cm long, about 1cm long, about 2cm long, about 3cm long, about 4cm long. In certain preferable embodiments, the segment of tubing is about 2.5cm long. In certain embodiments, electrical shielding comprises copper tubing surrounding, at least a portion of, the incubation tubing 709. In certain embodiments the copper tubing is the same length as the incubation tubing. In certain embodiments, the copper tubing connectively interfaces with a grounded Faraday cage enclosing and/or surrounding a sensor of the device. In certain embodiments, the improvement further comprises grounding one or more of the components as discussed herein.

In a preferred arrangement, a 1000mm incubation tube 709 between a sensor and syringes is shielded using copper tubing surrounding the incubation tube and grounded to inhibit electrical interference. Fluid can act as conductor and grounding fluid at the start of the incubation tube via electrical shielding 721 reduces electrical noise and/or increases signal isolation.

In some certain embodiments, fluid is in electrical contact with a positive electrode of a sensor. Accordingly, grounding a positive electrode (e.g., by grounding the fluid) would generally be avoided and highly unusual. Despite conventional wisdom, grounding fluid at the start of an incubation tube by passing the fluid through a short section of conducting metal as described previously herein diminishes electrical interference and/or improves signal accuracy of a sensor. In certain embodiments, a short section of a conducting metal tubing is located about 0.25m, about 0.5m, about 0.75m, about 1m, about 1.5m or further from a sensor.

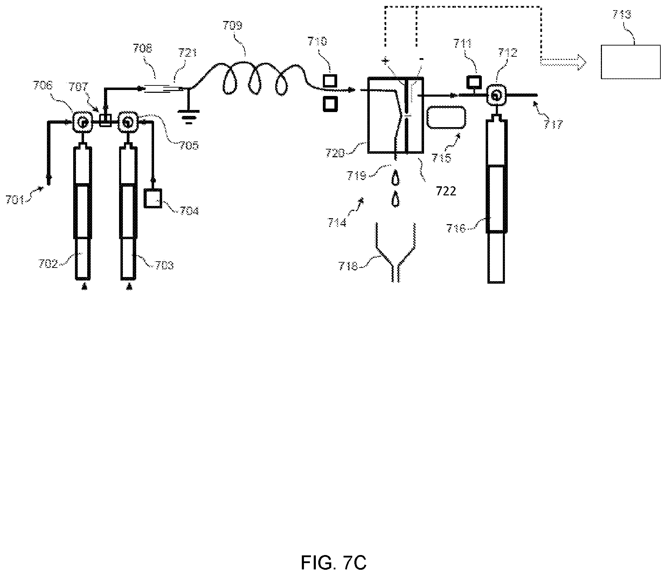

In certain embodiments, electrical noise can be improved by elimination of a drip air-gap (see e.g., FIG. 7C, at 714). Such improvement may, in some embodiments, provide for fewer malfunctions. The drip chamber 718 occasionally fills or blocks, and drips (e.g., as shown at FIG. 7C, 719) create an electrical short as a drip chamber 718 is grounded and drips are positively charged. This malfunction ruins data gathered by a device, and requires a repeat test. Sometimes there is a partial grounding, which appears as corrupted data due to spikes in data produced by a sensor. For example, drips act as a variable capacitor. As drips come closer to bridging a gap between a device and drip chamber, data becomes altered.

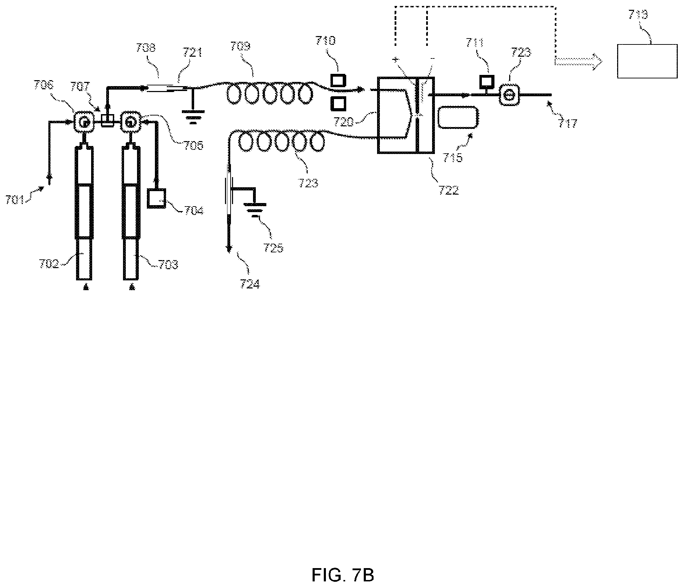

Elimination of a drip air-gap can be performed, for example, by adding a length of incubation tubing (e.g., as seen in 723 in FIGs. 7A and 7B) after an aperture. This is exemplified in FIGs. 7A and 7B. A portion of the sample fluid would flow through an aperture from one side of a chamber 720 to the second side of the chamber 722. The remaining waste sample would be passed to a second length of incubation tubing 723. In certain embodiments, the second length of incubation tubing 723 can be grounded 725.

## 10. Exemplified Embodiments of Mechanical Isolation of the Sensor

In certain embodiments, a sensor is extremely sensitive to mechanical noise and/or acts as a microphone under typical test conditions. Sensitivity to mechanical noise degrades accuracy and/or precision of a measured signal. Accordingly, improvements are desirable to mechanically isolate and/or dampen effects of vibrations on a sensor.

In certain embodiments, a sensor is mechanically isolated and mounted on vibration dampening supports to minimize an effect of mechanical vibrations on the sensor. In certain embodiments, the sensor is enclosed, either partially or fully, within a Faraday cage to minimize electro-magnetic and/or RF (radio-frequency) noise.

In certain embodiments, mechanical noise related to and/or conducted from syringe pumps and/or syringes is further isolated by encasing an incubation tube in a rigid, protective case.

In certain embodiments, mechanical noise is reduced by installing vibration absorbing materials around a sensor. Further, as noise travels along an entire fluid path, mechanically isolating a syringe drive, motor(s), and/or all mechanical noise paths improves signal fidelity at a sensor. Similarly, using electrically and/or mechanically quiet, high resolution syringe pumps and motors used to drive the syringe pumps as described herein further improves the fidelity of the signal.

## 10.1 Embodiment of Mechanical Isolation of the Sensor Using Deformable Tubing

In certain embodiments of the apparatus, an incubation tube is mechanically decoupled from the syringe drives and/or gradient generator by a short section of deformable tubing 708 to mechanically isolate the sensor and/or dampen fluid carried noise. In certain embodiments, a short section of deformable tubing used to decouple the systems is or comprises soft silicone tubing. In certain embodiments, the deformable tubing has about a similar or the same diameter as an incubation tube.

For example, a 1 cm length of silicon tubing at the start of an incubation tube improves performance by dampening mechanical noise inherent in the fluid stream as it leaves a mixing chamber 707.

## 11. Exemplified Embodiment of Mechanical and Electrical Isolation of a Sensor

In certain embodiments as described herein, a sensor is mechanically and/or electrically isolated. Mechanical isolation of the apparatus is, in some embodiments as discussed herein, necessary to prevent mechanical disturbances (e.g., vibrations, shockwaves) from disrupting measurements being made by a sensor.

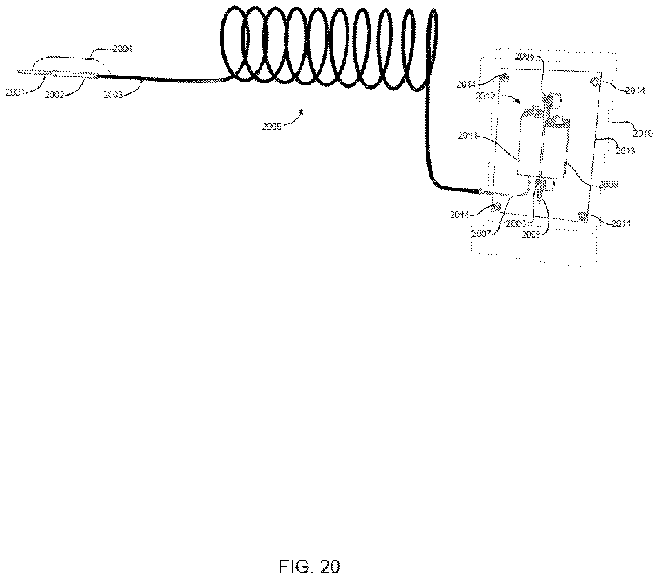

FIG. 20 demonstrates an embodiment of improvements to a device to provide for mechanical isolation of a sensor. In certain embodiments, as fluid leaves a mixing chamber and passes to a sensor, fluid passes through and is in contact with an electrically conducting tube 2001. In certain embodiments, the electrically conducting tube 2001 is a metal tube (e.g., a stainless steel tube). Fluid then passes through and is in contact with a deformable tube 2002. In certain embodiments as described herein, the deformable tube 2002 is a silicon tube. The deformable tube 2002 absorbs mechanical vibrations from fluid and/or incubation tubing. Fluid then enters a chemically inert incubation tube 2007 which is housed within a metal shielding tube 2003. In certain embodiments as discussed herein, the incubation tube 2007 is a PTFE tube (Polytetrafluoroethylene). In certain embodiments as discussed herein, a metal shielding tube 2003 substantially surrounding an incubation tube 2007 is a conductive metal (e.g., a conductive copper tubing). The metal shielding tube 2003 is electrically connected (e.g., through a conductive pathway 2004) to an electrically conducting tube 2001 and/or a metal shielding box 2010 around a sensor assembly 2012. In certain embodiments, a metal shielding box 2010 is and/or serves as a Faraday cage to prevent electrical interference with a sensor.

Fluid passes through an incubation coil 2005 and enters a sensor box 2010. Fluid then flows into one side of a sensor 2011. Fluid then passes through a sensor plate 2008 and into another side of a sensor 2009. In certain embodiments, a sensor assembly 2012 is mechanically isolated by vibration dampening mounts 2006 which themselves are connected to a backplane 2013. The backplane is connected to the metal shielding box 2010 and mechanically isolated by vibration dampening mounts 2014.

## 12. Exemplified Embodiments of Improved Refilling

In certain embodiments, two or more similar sample syringes are present and connected in parallel to one another at a substantially similar location in the fluid delivery path. Accordingly, this minimizes refill and/or wash time of sample syringes between samples being tested. While one syringe is being used for testing, a second syringe may be undergoing rinsing or refilling with a new sample. In certain embodiments, selecting a syringe for testing or rinsing is automated, and fluid paths are determined and controlled using well-known fluid valve controls. Thus, as soon as a test is complete, there is no time wasted for rinsing or refilling of sample syringes between tests. Accordingly, this increases the throughput and decreases operating costs, which is a benefit in diagnostic applications, wherein high throughput is required for an apparatus.

In an embodiment, FIG. 8 shows such an arrangement which functions similarly to an embodiment depicted in FIG. 4, but a second valve 810 and sample syringe 811 have been added. Sample is refilled into to a syringe 811 or 813 as controlled by a valve 809. Syringe contents are delivered to a mixing chamber 817 from each syringe via a valve 816. While syringe 813 is used during a test, syringe 811 is refilled or rinsed. Similarly, when syringe 811 is used during a test, syringe 813 is refilled or rinsed. This process eliminates need for a delay for a refill cycle of a sample syringe. In certain preferable embodiments, lengths of tubes 814 and 815 are about the same.

## 13. Exemplified Embodiment of Blockage Detection and Prevention

In nearly all particle measurements, cells or particles travel across a sensor due to a pressure difference. This difference in pressures also attracts large particles or agglomerations of particles which can block the sensor. Accordingly, it is desirable to employ methods and techniques to minimize or remove blockages from the sensor.

Blockages can be caused by any particle or group of particles that is too large to be forced though the sensor under normal testing conditions. A blockage will alter measurements made by a sensor by obscuring and/or altering a measured signal obtained by the sensor. The blockage usually interferes by inhibiting the free passage of fluids and/or particles across a sensor by creating a constriction.

By running multiple samples at a time (e.g., in triplicate), the results from a particular sensor that differs vastly from another sensor(s) are rejected on the assumption that the particular sensor is blocked. Accordingly, data from an unblocked sensors is used in calculation of results regarding, for example, cell shape and cell size.

In an embodiment, a blockage can be detected from any single aperture of a sensor by monitoring cell size and/or a signal generated continuously, and determining when the signal and/or cell size measurement and/or distribution of such measurements, abruptly changes, beyond normally expected limits. Abrupt changes in cell size would not be expected during testing under a single set of environmental condition, where changes in cell size between successive aliquots is expected to be similar. Also such abrupt changes would not be expected where changes to an environment of cells are known to occur slowly or predictably. For example, before a cell has ruptured, cell size (e.g., cell volume) does not increase more than a percent or so, per mOsm drop in osmolality. An apparent large or near instantaneous change in size or cell concentration indicates a blockage.

In certain embodiments, to remove a blockage, pressure within a sensor is temporarily reversed, by expelling fluid from a syringe to creating a reversal of fluid flow through the sensor, which typically ejects the blockage and flushes it to waste. For example, in an embodiment as depicted in FIG. 7C, syringe 716 is used to create a flow reversal of fluid.

At a sensor, a reverse pressure surge is created by directing a small jet of fluid towards an aperture on the sensor's low-pressure side and/or by increasing the pressure on the low-pressure side by temporarily removing the low-pressure source. Removal of a low pressure source reverses fluid flow across a sensor.

Flushing can be performed manually or automatically when a predetermined change in count, size or other sensor measurement is reached. In certain embodiments, a predetermined change would be a sudden increase in voltage and/or a sudden change in conductivity as measured by a sensor. In certain embodiments, an increase in voltage is at least 100mV, at least 200mV, at least 300mV, at least 400mV, at least 500mV or more. In certain embodiments, an abrupt change would be measured by a significant change in cell volume. In certain embodiments, a change in volume would be a greater than 10% increase, greater than 20% increase, greater than 30% increase, greater than 40% increase or more in cell volume under an applied environmental condition.

## 14. Exemplified Embodiment of a Sensor of the Apparatus

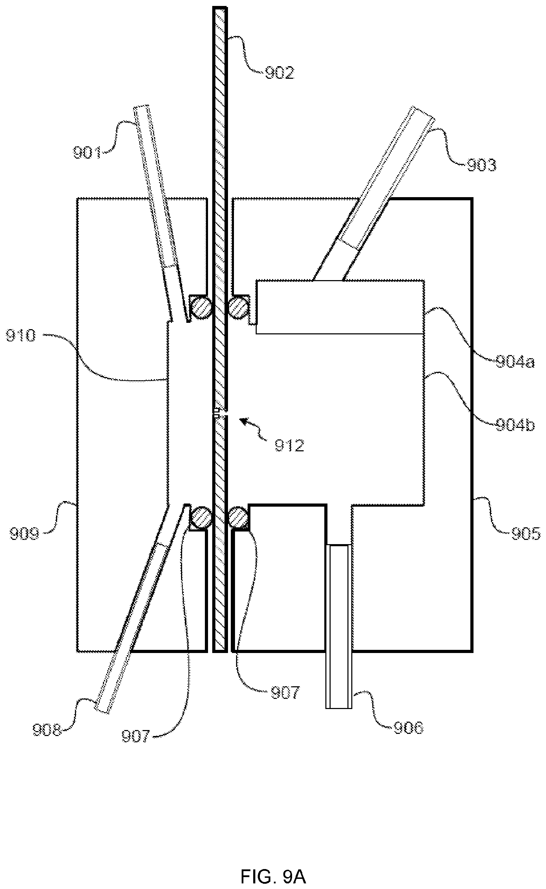

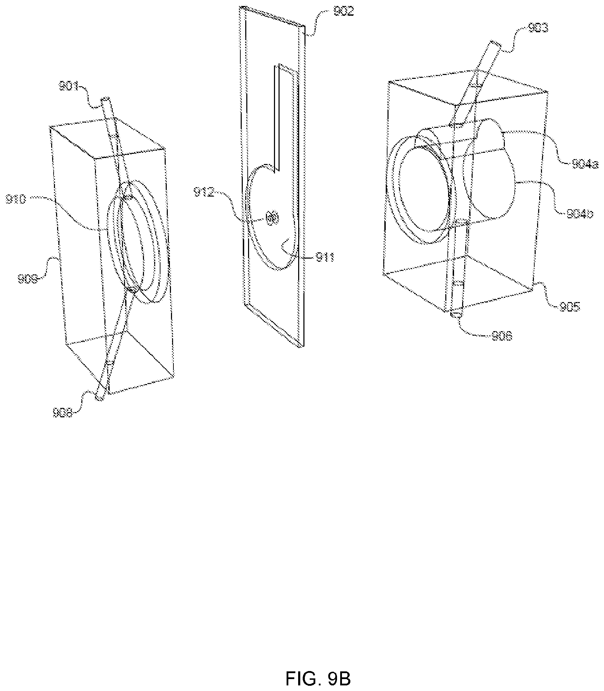

In certain embodiments as disclosed herein, FIG. 9A and FIG. 9B show a typical sensor arrangement. FIG. 9A is a cross-section through a sensor arrangement. FIG. 9B shows an exploded perspective of the same sensor arrangement. Such an arrangement is used for making a DC voltage measurement of cells as they traverse a small aperture. Sample suspended in a fluid is introduced to the bottom of the sensor via tube 908 which directs the sample to aperture 912. Excessive fluid fills chamber 910 and flows out of tube 901. An exit tube 901 is arranged at the top of removable sample assembly 909 to aid in removal of entrapped air, which migrates to the top of chamber 910. A flexible tube from tube 901 leads the excess fluids to a waste vessel via a passive check valve (not shown). This also provides an electrical air gap between the electrode 911 and the excess fluid and the waste vessel. Accordingly, this tube aids in minimizing electrical interference.

A pressure differential between chamber 910 and 904 draws cells and suspending fluid across an aperture 912. The aperture 912 is fastened into ceramic wafer 902, which has inert metal electrodes 911 on both side of the wafer. In certain embodiments, the electrodes comprise or are platinum. The electrodes are wetted by fluids in chamber 910 and 904. Traces from the electrodes connect to controlling electronics (not shown). A chamber 904 (904a and 904b) is filled with fluid from a lower tube 906. Fluids exit through an upper tube 903. Chamber 904 is split into two parts; a main chamber 904b that is substantially the same dimension (e.g., diameter) as the electrodes 911; and an upper smaller section 904a that traps gasses and keeps them from interfering with the electrode 911. A vacuum is maintained in the sensor apparatus by drawing fluid out of the upper tube 903. The upper tube 903 is angled so as to aid the removal of blockages from the aperture 912 when a jet of fluid is injected into the chamber 904 via tube 903. The removable waste assembly 910, ceramic wafer 902 and waste part 905 are clamped or screwed together (not shown) in close proximity as shown in FIG. 9A. Two deformable O-rings 907 are slightly compressed when assembled and provide a fluid seal around the electrodes for both chambers 904, 910 and dampen any vibrations within the assembly. A removable sample assembly 909 and removable waste assembly 905 are preferably made of a transparent material, such as acrylic, so that a camera, such as a CCD, can provide an image of an aperture when seen for end of the fluid volume cylinder 910 or fluid volume cylinder 904b.

In a typical DC sensing arrangement, one side of a sensor is positive, while the other side of the sensor is negative. Polarities are reversed each test to prevent build up on the electrodes (e.g., cathodic build up). Due to the necessary continuous fluid paths used in the present invention, the polarities cannot easily be reversed during a test without grounding the positive electrode and potentially altering the measurements. To overcome this, the electrodes are run with the fluid delivery side as positive during a test and reversed when data is not being collected, such as during refill time, preferably with the current adjusted to match the current applied during the test, thereby neutralizing any polarity effect.

## 15. Exemplified Embodiment of Fine Pressure Control

In existing cell sizing equipment, a pressure differential across a sensor is created by a regulated low-pressure source, such as a regulated vacuum pump. However, regulation of vacuum pressure for existing methods is generally poor, and small fluctuation in pressure regulation, during a test and/or between tests, causes a changes in pressure across a sensor. These variations alter volumes of fluid drawn across a sensor, and thus results in an effect on the number and measured cell parameters (e.g., cell size, cell shape) by the sensor, both directly and indirectly. For example, a direct effect on determining cell count results from a change of rate of cells crossing a sensor as the rate of cells crossing the sensor is used to determine cell and/or particle concentration. In another example, an indirect effect is due to changes in coincidence and cell deformation, which directly alters measured cell size. As some of the parameters measured using the technology disclosed herein are repeatable to 0.01mOsm. Even a very small fluctuation in regulating pressure would cause a degraded measurement.

Accordingly, in an embodiment, pressure regulation is achieved with precise and/or accurate movements of a digitally controlled syringe. For example, an embodiment as shown in FIG. 7C shows syringe 716. In certain embodiments, syringe 716 creates a pressure gradient across the sensor by slowly aspirating fluid through a sensor. This slow aspiration creates a constant low pressure on one side of a sensor. During a run, an aspirating syringe 716 slowly aspirates, creating a negative pressure across the sensor and fills with solution containing cells having passed through an aperture of the sensor. After a test is run, the syringe contents are discarded to waste through valve 712, and the syringe resets.

In certain embodiments, a pressure across a sensor is constant and/or very well regulated. In other embodiments, a pressure across a sensor can be altered during a test, providing another way of exposing cells to a changing environment of pressure. As explained above, an additional advantage of this method is that the digitally controlled syringe that creates the low pressure can, in certain instances, also be used to create a highpressure surge to remove the blockages, as described above, by temporarily changing a direction of fluid flow across a sensor. Combining the blockage removing apparatus and the low-pressure source is inherently valuable, reduces complexity, and/or increases accuracy of measured cell parameters.

### 16. Embodiments of Mechanical Vacuums

In certain embodiments, a mechanical vacuum may be used to regulate osmotic pressure on cells within a tested sample. For example, in an embodiment as depicted in FIG. 7C, pressure on one the sample side 720 of sensor 714 is atmospheric pressure, while the pressure on the waste side 721 leading to syringe 716 is below atmospheric pressure, causing fluid to be drawn across the sensor. Excess sample drains to waste 718 through an air-gap separation 719. In an alternate embodiment, a sample side of the sensor 720 has no drain to waste 718. Through use of digitally controlled syringes on both sides of a sensor/aperture, pressure on each side of the sensor can be independently controlled, enabling the creation of a pressure gradient environment, similar to osmotic pressure. For instance, it is possible to decrease the absolute pressure within the system on both sides of the sensor, while maintaining a constant pressure differential across the sensor. In this way, cells can be subjected to decreasing physical pressure, which simulates pressures cells experience osmotically, while maintaining a constant flow rate and pressure differential across the aperture. Applying physical pressure can be used in conjunction with and/or in lieu of osmotic pressure and/or other concomitant cell tests.
**Monitoring** pressure on both side of the sensor during a test also allows for closed loop regulation of pressure and flow rates.

### 15.1 Embodiments without Mechanical Vacuums

In certain embodiments, a mechanical vacuum pump (e.g., a syringe, a mechanical pump) is not required. For example, Applicants conducted tests with no vacuum pressure being exerted and found that useful data was still generated in various embodiments. Without wishing to be bound by any particular theory, it is proposed that this observation is due to the slight pressure at the end of the incubation tube forcing some cells across the sensor. Not incorporating a mechanical vacuum simplifies test arrangements of devices disclosed herein, as it is possible to eliminate the vacuum and still run certain tests by relying on a slight positive pressure from the incubation tube to move cells across an aperture.
An example of device without a mechanical vacuum is shown in FIG. 7B. For example among other things, FIG. 7B and 7C differ in that FIG. 7B lacks a syringe 716 (as seen in FIG. 7B), which can be used to control pressure across an aperture 722.
The previously discussed effect can be enhanced by, for instance, locating a sensor halfway along a double-length incubation tube (e.g., as seen in FIG. 7C). However, the length of the incubation tube may be varied. Locating a sensor at a point where there is a higher pressure in the incubation tube would create a similar pressure differential across an aperture without need for a vacuum pump or syringe. Those skilled in the art, reading the present disclosure, will appreciate both that and how such effects can be achieved.

### 17. Exemplified Embodiment of Multiple Sized Apertures

In embodiments, red blood cells are a preferred cell population tested due to their abundance and ease of collection. However, other cell populations within a whole blood sample can be tested as well, such as, without limitation, white blood cells, cell fragments, and platelets. These other cell populations may provide additional information on a subject or sample. Accordingly, in certain embodiments, different sized apertures may be employed. For instance in certain embodiments, a diameter for an aperture for red blood cells is about $98\mu m$. In certain embodiments, a diameter for an aperture for white blood cells is about $197\mu m$. In certain embodiments, a diameter for an aperture for cell fragments or platelets is about $30\mu m$.
For instance, by splitting an incubation stream into multiple apertures of a same size sensor, it is possible to get redundant testing. In certain embodiments, apertures and/or sensors may be of different sizes as discussed above to examine different populations of cells. For example, a smaller aperture with different cell concentrations allows the platelets and fragments are measured with less distortion and are subject to the same environmental conditions as the red cells. As described in U.S. Provisional Patent Application for "Apparatus and Controller-Implemented Method for Determining Physiological Condition Based on Measurement of Physical Cell Properties," Application No.: 62/775,703, which is incorporated herein by reference in its entirety, detection of platelets and/or fragments is useful in numerous diagnostic applications. Further, in embodiments having multiple apertures of a same size improves accuracy of a test, by, for example, reducing an effect of coincidence and/or other population concentration distorting effects.

### 18. Exemplified Embodiments of Vortex Mixing Unit

Solutions with different osmolalities do not readily mix. Accordingly, the method and apparatus discussed herein allows for even mixing of a diluent and samples containing cells.

In an embodiment, cell suspension and diluent dispensed from syringes is mixed at one or multiple locations within a fluid path. For example, before entering an incubation tube and/or a sensor. Even mixing ensures maintenance of precise osmolalities and/or even cell distributions within a mixture. Efficiency and/or integrity of mixing cells within their suspending solution and when introduced to a diluent is important to obtaining a precise sensor reading. In certain embodiments, a total flow rate of through a mixing unit is about 50 μl/sec, about 100 μl/sec, about 150 μl/sec, about 200 μl/sec, about 250 μl/sec, about 300 μl/sec, or more. In certain preferable embodiments, a total flow through a mixing unit is about 200 μl/sec.

Ensuring uniform mixing of a sample and suspending diluent, even as their flow rates change or differ significantly, requires a balance between providing adequate mixing so that the cells are exposed to homogeneous, well mixed diluents and keeping successive volumes of suspending diluent distinct to prevent a loss of precision, e.g. through maintaining a gradient (e.g., osmotic gradient). In addition, ensuring a low pressure drop during mixing is important, so that the cells of the sample are not exposed to sudden change in pressures. Sudden changes in pressures cause micro-surges in cell sample and/or diluent, leading to poor mixing and/or inconsistent data. If there is inadequate mixing of diluents, cells are exposed to localized pockets of hypertonic or hypotonic solutions, which influence cell size and/or introduce variability into measurements. These variations would lead to, among other things, significant variations in cell counts per unit time. If there is too much mixing, change in cell size is averaged with adjacent diluents reduces details of cell size changes, leading to imprecise measurements. In other words, if an environment of fluid being mixed changes too rapidly, cells may be experiencing a different environment than was intended and/or predicted. Furthermore, efficient, uniform mixing allows each test to use smaller sample sizes, which minimizes the fluid consumption. Furthermore, a design of a mixing chamber critically influences the resolution and usefulness of these results.

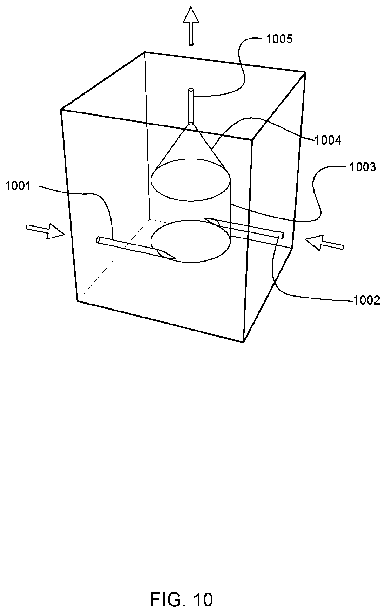

An exemplified embodiment of a vortex mixing unit is depicted in FIG. 10. Mixing occurs for two diluents at a time with each diluent being introduced through tubes 1001 and 1002 into a substantially cylindrical mixing chamber 1003 with an inverted conical top 1004. The mixture exits through a vertical tube 1005. As fluid enters a mixing chamber, fluid streams initially mix and accelerate as they rise through a narrowing cone 1004. This design ensures that there is very good mixing within a very small aliquot of fluid, as after an initial low volume mixing, the different velocity of fluid layers do not readily mix. By placing an exit at the top, entrapped air is flushed. In certain embodiments. multiple mixing chamber may be arranged sequentially. In certain embodiments, a mixing chamber may have multiple tangential entries.

There are many commercially available vortex mixing chambers, such as Lee Co, Visco Jet Micro Mixer, but these are not useful in this application, as they have large volumes and/or produce very high pressure drops (hundreds or thousands of PSI) that would disrupt accuracy and/or precision of measurements. Typical dimensions of a vortex mixing chamber for a 200 μl/ second output are 4mm x 4mm for the cylinder with a top cone that is about 4mm tall. Such mixing chambers may be larger or smaller, depending on the volumes being mixed.

### 19. Exemplified Embodiment of Flow Patterns Used to Create Uni-Directional Positive Pressures

In order to refill a syringe, it is necessary to create a negative pressure within a syringe. The negative pressure draws sample or diluent into a syringe. Accordingly, in certain embodiments, syringes draw fluid in at a slow fluid flow rate (e.g., about 0.5ml/sec), taking 15 seconds to aspirate 5 ml A slow rate of fluid entering a syringe ensures that there is no outgassing. After filling, a syringe pauses for a few seconds, allowing pressure to equilibrate, and for any pressure waves to dissipate before moving a syringe valve. Injection of a sample to a mixing chamber, and subsequent injection of sample into a sensor, continues for a period of time before data collection begins. This arrangement allows for an equilibration time, when pressure settles, and the entrapped air, settled particles and/or gasses created from electrolysis at electrodes are all flushed. While equilibration times are common in testing techniques, use of techniques to improve refilling allows for faster test cycles and/or less wasted sample and/or reagents.

For example, an embodiment, e.g., FIG. 4, may be used to demonstrate points at which it is important to maintain a consistent flow through a device. Valves 413 and 415 from a sample syringe 422 and diluent syringes 423 and 425 are brought into communication via three valves 413, 415 and 428 through mixing chambers 414 and 418, such that there is no movement or contamination of fluids due to pressure differences and direction of fluid flow.

In a preferred embodiment, a syringe always maintain positive pressure and expels fluid when fluid paths are joined with another syringe, to avoid cross contamination. After a syringe has refilled with sample and/or diluent, a diluent syringe and a sample syringe accelerate to test speed while expelling their contents to a sensor. This initial expulsion of fluid primes the sensor. The data is ignored until a fluid stream being delivered to a sensor has equilibrated. This ensures that only even, positive pressure is introduced into a mixing chamber. Subsequently, a smooth acceleration of syringes and/or smooth increases in pressures results in the maintenance of an even and/or consistent flow into a mixing chamber, along the incubation tube and/or into a sensor.

In a preferred embodiment, a combined volume/flow rate of all diluents is constant, so that a pressure within a mixing chamber and beyond is uniform and constant. Without wishing to be bound to any particular theory, a constant flow minimizes compressive waves being delivered to a sensor. Compression waves introduce noise and variation in the sensor readings. Furthermore, compression waves cause uneven mixing. Accordingly, eliminating pressure waves helps to ensure uniform cell populations.

In an embodiment, osmolalities and/or environmental parameters of initial and final environments are selected to ensure that a velocity ratio between two syringes of the apparatus is no more than 1:3, no more than 1:5, no more than 1:6, no more than 1:7 or more. In a preferred embodiment, the velocity ratio between two syringes is no more than 1:4. That is, a speed of a first syringe will be moving at about 4x the speed of a second syringe. In certain embodiments, a speed of a first syringe will be about 80% of full movement speed of the syringe pump while a speed of a second syringe will be about 20% of a full movement speed of the syringe pump. This ensures that there is positive pressure within each syringe such that fluid from each syringe will always be moving towards a mixing chamber.

In a preferred embodiment, a gradient of change in environment between two syringes is such that the gradient is a linear gradient. In an embodiment, the linear gradient is created such that a 1:4 ratio (first syringe fluid flow rate: second syringe fluid flow rate) between a flow rate of fluid from two syringes provides the desired starting osmolality, and a mixture of a 4:1 ratio between two syringes provides the desired ending osmolality.

Without wishing to be bound to any particular theory, using the relative movement velocities of fluids through the apparatus as discussed herein eliminates cross contamination, irregular flow, or pressure waves. Avoiding very low syringe speeds and/or standing starts overcomes common problems with moving small volumes of fluid, including: stiction (a tendency of a piston to stick, deform then suddenly move when a force is first applied), poor mixing due to low volumes, and cross contamination among others. Stiction can create pressure waves and/or resonances within initial fluid movement and/or highly unequal flow rates creates poor mixing, as the low flow rate fluid acts as if it is jittering when being injected into the stream. Cross contamination occurs most readily when unequal pressure forces fluid from one syringe into and/or towards another, though compression of entrapped air and/or from backlash or play within the system, which alters properties of fluids and/or sensor measurements. An initial irregularity and/or imbalance in fluid flow often propagates throughout a test, reducing accuracy and/or precision of results.

In certain embodiments, a syringe used has a volume of about 3ml, about 4ml, about 5ml, about 6ml, about 7ml, about 8ml, about 9ml, about 10ml, about 11ml, about 12ml, about 13ml, about 15ml, about 20ml or more.

In certain embodiments, the one or more syringes containing a sample each have a volume of about 10ml. In certain embodiments, a sample syringe and a first diluent syringe (e.g., syringe 422 and 423 of FIG. 4, syringe 811 and 813 of FIG. 8) have about the same volume. In certain embodiments, a volume of each of said syringes is about 10ml. In certain embodiments, a volume of a diluent syringe used in conjunction with said syringes (e.g., syringe 425 of FIG. 4 and syringe 827 of FIG. 8) has a volume of about 5ml.

## 20. Exemplified Embodiments of Calibration and Fluid Flow Measurement Techniques

Calibration is important in order to provide for robust, accurate results, for example, between test samples, between test runs, between instruments and /or testing facilities. Accordingly, methods for improving calibrations described herein.

Calibration is used with cell testing methods that modify a testing environment of a cell or population of cells in a controlled or measured way, including, for example, certain embodiments of U.S. Pat. No. 6,668,229, DC or AC sizing equipment, laser scatter measurements, CT or MRI measurements, ultrasonic sizing measurements, and/or optical sizing measurements. Regardless of type of sensor used and/or stressing force, calibration ensures accurate and/or consistent readings.

Accurate results are derived from carefully controlling an environment to which a cell is exposed and/or measuring a response of a cell. Time, temperature, pressure, tonicity, flow rate, pH, concentration, suspending reagents, agent concentration, sample age, sample handling, systems parameters (e.g.,. tubing diameters), internal pressures, electrical field strength, noise, system pressure stability, mixing ratios and/or other internal parameters are all preferably controlled and/or measured using calibrations to ensure consistent results.

Even with stable and consistent equipment, calibration is still required to translate results into meaningful measurements. Such measurements include, without limitation, the size of a particle, concentration of particles, incubation time of a sample within the system, and environment at a time of testing. Calibrating these measurements typically depends upon cell size when in spherical and isotonic shapes, an environment at a start, end and/or during a test, system timing delays, system pressure and/or temperature. Controlling and/or calibrating all of the aforementioned parameters of improves accuracy, precision, confidence and/or reliability of a test. Further calibration of the efficacy of mixing, the tonicity of the reagents and the osmotic pressure at the sensor further improves the data. Some of these calibrations are needed to produce meaningful results, while all of them contribute to improved data. In a further improvement, calibrations as described herein for some parameters is conducted during a test, further improving the reliability of data.

## 21. Exemplified Embodiment of Measurement of Flow Rate

It is beneficial to measure flow rate of a cell mixture while cells move towards a sensor, to provide a closed loop feedback of flow rate, to confirm normal operations, and/or to detect any instrument malfunction.

Techniques for measuring fluid flow rate within a small diameter tube, include ultra-sonic doppler shift, heat transfer, and EMF among others. In certain embodiments, as cells move towards a sensor in a rigid tube, a flow rate of cells is proportional to a pressure within a tube. Accordingly, measuring pressure at points along a tube provides a simple measure of the fluid flow rate using a technique as discussed herein.

In an embodiment, flow rate during a test is constant and/or controlled. Accordingly, a deviation from a predicted value in a flow rate measurement is indicative of a malfunction and/or error. In certain embodiments, a measured flow rate is kept constant through use of an automated computer controlled feedback loop.

## 22. Exemplified Embodiment of Incubation Tube Delay Calibration

In certain embodiments, once a cell suspension is mixed with a diluent, it travels down an incubation tube before reaching a sensor. Accordingly, there is a delay of several seconds after cells have been mixed before they are measured. Correspondingly, ensuring that environmental conditions cells experience at a sensor are known and/or calibrated is important for normal operation.

In an embodiment, a method of determining incubation tube delay (i.e., a time it takes for a sample to move from a syringe to a sensor) is by injecting a suspension of particles (e.g., microparticles, e.g., latex spheres, cells) or fluorescent dye into a particle free stream and timing a delay before particles arrive at a sensor.

In another embodiment, a concentration of the particles delivered to a sensor occurs at at least two different rates, and an intersection of the two flow rates provides an accurate determination of incubation tube delay. In one example, cells may be used as the particles of interest. Cells are initially injected at a known rate, such as of 5,000 cells/second. After a timed delay, the rate can be changed to a different rate, such as 1000 cells/second, by for example, changing the relative amounts of diluent and cells being delivered to the sensor. The delay between the change in concentration from the initial concentration (e.g., 5000 cells/sec) to the final concentration (e.g., 1000 cells/sec) indicates the incubation tube delay.

In another embodiment, cells can initially be injected at a known rate and, after a timed delay, the rate can be altered to create a rate that is falling or rising over time. In certain embodiments where the rate of change of particles is programmed to be altered in a linear fashion, the inflection point at which this change occurs may be determined using standard line and/or curve fitting techniques by finding the intersection between the lines describing the first phase in which the number of cells being delivered is uniform, and the second phase in which the number of cells being delivered is changing.

For example, cells may be delivered to a sensor at a rate of 5000 cells/sec for a given period. A rate of cell delivery to a sensor may then be programmed to drop by 100 cells/second after the initial period of uniform delivery of cells to the sensor. The time between the start of the attention of the cell concentration and when it is recorded to happen at the sensor provides a very accurate measure of the incubation and system delay.

## 23. Example of Tubing Diameter Effect on Cell Parameters (e.g., Pk0)

In embodiments, tubing diameter of the incubation tube also has an effect on Pk0, which is calculated as discussed herein. As dimensions of tubing (e.g., diameter, length) are altered, the time it takes for a sample to be transported to the sensor through the incubation tubing changes. For example, increasing the tubing length causes the sample to take longer to travel to a sensor. In certain embodiments, as a length of tubing is changed, a measured Pk0 of the red blood cells also changes due, in part, to the incubation time.

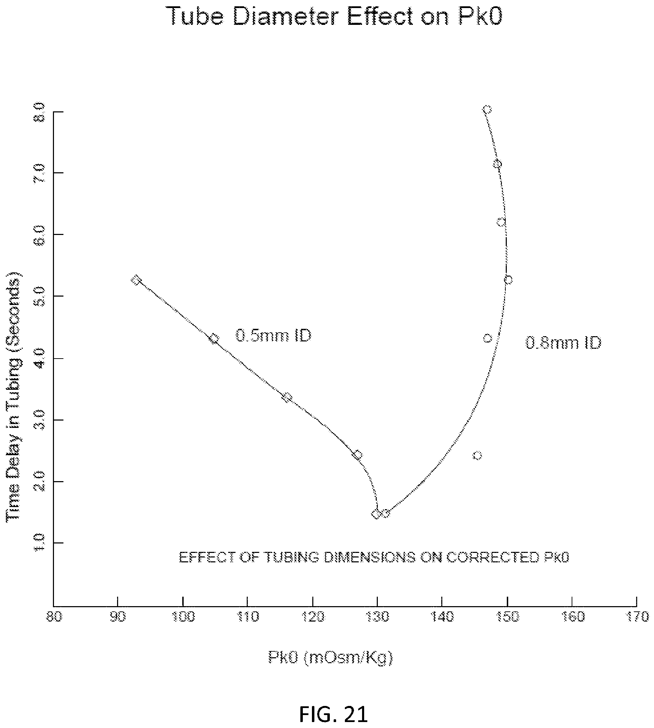

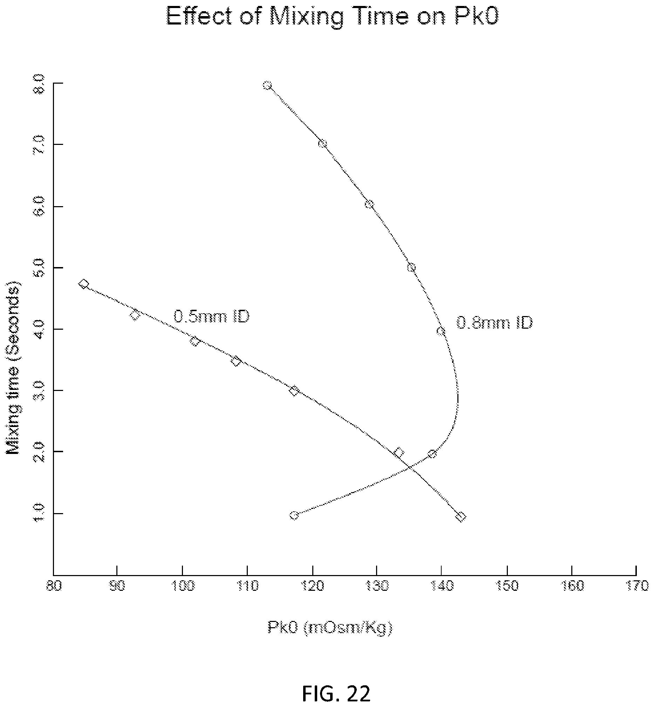

As can be seen in FIG. 21 and FIG. 22, two different diameters of tubing are utilized in certain embodiments in order to determine their effect on the Pk0 of red blood cells.

FIG. 21 explores the effect of different delays in the delivery of the sample to the sensor are calculated as functions of different tubing lengths. As the length of the 0.5mm inner diameter tubing (indicated by black 'x's) is shortened, the time delay in the sample moving through the incubation tubing drops. Simultaneously, the Pk0 measured for the red blood cells also increases from about 90mOsm/kg to about 130mOsm/kg.

For similar lengths of tubing having a 0.8mm inner diameter (indicated by 'o's), a different effect is seen on Pk0. As a length of tubing is increased, Pk0 increases until it reaches a maximum of about 145mOsm. This value approximates a known Pk0 of a healthy individual.

Accordingly, it would be desirable to utilize incubation tubing having about a 0.8mm inner diameter and of a sufficient length in order to provide for consistent results. In certain embodiments a length of incubation tubing ranges from about 0.5m to about 1.5m. In certain preferable embodiments, a length of the incubation tubing is about 1m long.

In FIG. 22 differences in mixing time are also seen to have a demonstrable effect on Pk0. Increasing mixing times of the sample when using either 0.8mm diameter tubing or 0.5mm diameter tubing results in a, generally, marked decrease in Pk0. However, when using 0.8mm diameter tubing, there is an initial increase in the Pk0 from 120mOsm/kg to about 138mOsm/kg. Pk0 is maintained at about that Pk0 until the mixing time is at or exceeds 5sec. Accordingly, it is preferable to maintain a mixing time of about 2 to 5 seconds when mixing the sample and using a 0.8mm inner diameter tubing.

## 24. Exemplified Embodiment of Dyes or Markers Particles Used in Calibrations

In another embodiment, fluorescent dyes, particles, or other markers can be added to at least one of syringe, sample, and/or diluent. Markers allow for determination of, among other things, a change of gradient of agents in fluid, e.g. changing osmotic gradients, and/or monitoring of fluid flow through tubing.

In certain embodiments, a fluorescent light source is located at or near the sensor. In certain embodiments, a fluorescent light source is located in the incubation tube. In certain embodiments, a fluorescent light source is located approximately perpendicular to the incubation tube. In certain embodiments, a fluorescent light source is an ultraviolet light. Exposure of fluid comprising one or more fluorescent dyes or fluorescent markers to a light source (e.g., a ultraviolet light source) causes a dye to fluoresce brightly. Fluorescence can be detected and/or measured using a standard opto-sensor, light absorption, and/or other techniques.

As fluorescent dyes used are very dilute and have known osmotic properties, dyes can be added to diluents prior to testing. In an embodiment, dyes are added by a manufacturer to a diluent in order to provide for highly accurate control over dye properties and/or concentrations. Tested concentrations may be recorded on diluent packaging, thus providing confirmation of the diluent-dye mixture and/or predicted concentration at a sensor, or dye concentration may also be measured when entering the apparatus, that is before and after mixing the diluents. In another embodiment, a dye may be added to help identify an environmental condition at a sensor, aiding calibration, test integrity, and/or a detection of a malfunction.

In another embodiment, one or more dyes are added to each diluent, wherein each dye used has its own spectral signature. Dye allows for relative and/or absolute volumes and/or flow rates of diluents and/or sample to be monitored by measuring their brightness when exposed to an excitation source. Reactive chromic active dyes could alternatively be used to measure resultant mixture proportions at a sensor.

In another embodiment, fixed particles of known size can be added to a diluent, for calibration and/or with each test, to ensure accurate cell size measurements. As particles, such as latex microspheres, do not change parameters with changing environmental conditions, they are a good way to calibrate sensors during a test. In certain embodiments, only a single size particle is used for each sensor tested. In certain embodiments, multiple different sized particles are used (e.g., having a known distribution of sizes). In certain embodiments such particles may be a spherical microparticle, e.g., a latex bead or a synthetic vesicle (e.g., a microvesicle). In certain embodiments, a volume of the particles is less than less than 70 femtoliters, less than 60 femtoliters, less than 50 femtoliters, less than 40 femtoliters, less than 30 femtoliters, less than 20 femtoliters or less. In certain embodiments, the volume of the microparticle is greater than 100 femtoliters, greater than 110 femtoliters, greater than 120 femtoliters, greater than 140 femtoliters, greater than 150 femtoliters, greater than 200 femtoliters or greater. In certain embodiments, a selection of the microparticle size is based on a known or approximate size distribution of a particle (e.g., a cell or population of cells) of interest. In certain embodiments, microparticles are more than 1 standard deviation, more than 2 standard deviations, more than 3 standard deviations or greater different than the mean size of the cells of interest. In certain preferable embodiments, microparticles are more than 2 standard deviations away from a mean cell size of interest. The standard deviation may be obtained from either a known or predicted distribution of cells of interest.

In another embodiment, two or more different sized particles in very dilute concentrations are tested together. In certain embodiments, an artificial sample comprising microparticles may be measured as a calibration run. In certain embodiments, microparticles may be added to a diluent and/or sample. Addition of microparticles to a testing environment allows for calibration measurements to be performed concomitantly with sample tests.

Latex microparticles do not alter their size with varying osmotic pressure or environmental conditions. Therefore, it is simple to identify and separate cells from synthetic microparticles. This allows for size determinations and/or calibrations to be based on synthetic particles in the sample alone.

In a certain embodiment, microparticles added to a diluent and/or sample may have at least two different populations, e.g., one population having very small size and a second population having a very large size, outside of a range of a target population of cells. Accordingly, measurements from these two particle populations provide two reference points with which to compare cell size measurements. Synthetic particles will not interfere with test sample measurements as they are very dilute, and are out of range of sizes recorded for the test sample. Any cell that falls outside a typical cell size range is excluded from test data. Over the course of a, for example, 30 second test, a statistically significant number of synthetic particles will be measured to provide reference size measurements and/or use in continuous calibration.

## 25. Exemplified Embodiments of Calibrating with Blood

In an embodiment, a standard biological control sample can be used to calibrate or confirm calibration of an implementation of the method of the invention. A microliter of human blood contains about 5 million red cells, or about 250 million red cells per drop of blood. As each test typically measures parameters of 250,000 cells, a single drop of blood could be used for 1000 tests without any loss of precision or accuracy, making whole blood with known properties of red blood cells a simple and cost-effective method of performing calibration tests. As the properties measured are stable and change in a slow, predictable manner over time, using a single sample to calibrate many instruments is a viable method of providing control samples. Preferably, such a calibration is performed under the same environmental/experimental conditions and samples are stored using the same storage conditions.

In another embodiment as discussed herein, it is possible to calibrate an instrument by using a local population of presumed healthy controls and comparing the (for example) average size and shape of cells obtained from the healthy controls to the expected results. As a general, healthy population has a remarkably consistent and narrow range of normal values (e.g., Pk0, cell shape, cell size) for red blood cells. Deviation from the expected normal values can be used as correction factors to compensate for instrument variation and/or to indicate a malfunction.

In an embodiment, the method and apparatus described herein gives very consistent results between different, healthy blood donors. A collection of at least 5, at least 10, at least 15, at least 20, at least 25, at least 30, at least 50 or more samples taken from a population of such donors may serve as a control. In preferable embodiments, 20 samples obtained from different subjects serves as the set of control samples. Moreover, such samples have a miniscule probability of deviating from the daily average and thus can be used for daily calibration. This probability is close to zero given that an estimate of any one subject in a population having an abnormal scan is close to zero (e.g., approximately 0.05-0.005 (e.g., 20!/1!x19!^0.05x0.95 (=0)).

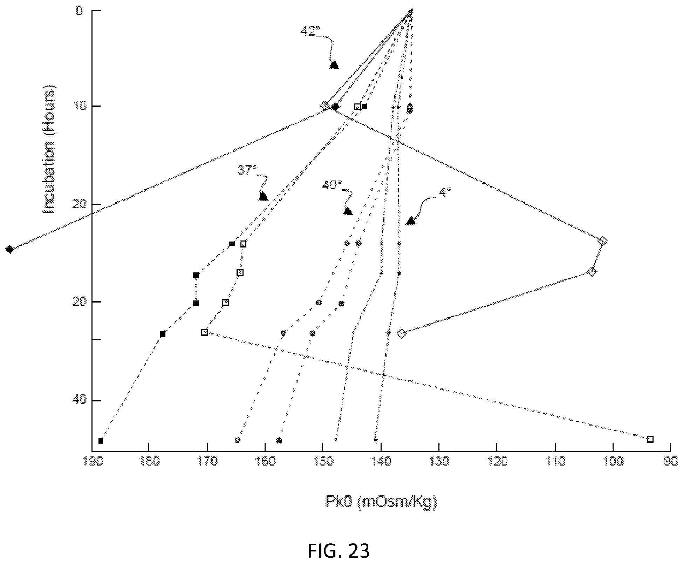

As can be seen in FIG. 23, the temperature at which the blood is incubated may affect this calibration by, among other things, altering the Pk0. Whole blood samples incubated at temperatures of 4°C, 37°C, and 42°C over a period of up to 44h prior to testing using an embodiment of the technologies described herein. As can be seen, with increasing times of incubation, the Pk0 varies significantly from the initial measured Pk0. Maintaining an incubation temperature of 4°C yields the least deviation from the Pk0 as initially measured when the samples are incubated for about 10h or less.

For long term storage of samples, it is preferable to store samples in aliquots under liquid nitrogen prior to testing as this has been found to result in the least deviation from the initial Pk0 of the cell sample.

## 26. Exemplified Embodiments of Osmolality Sensors

The response of the cell suspension to its surrounding osmolality is used to calculate properties of the cell. Therefore, it is important to ensure that the osmolalities of the test solution is known as it passes through the sensor.

In one embodiment, a transimpedance amplifier is used to maintain a constant voltage though the sensor. As the osmolality falls, the conductivity of the surrounding fluid falls due to fewer dissolved salt ions and to maintain the same voltage the current increases, as part of a transimpedance circuit design. For existing tests, only the change in voltage, relative or absolute, is recorded. The voltage pulse corresponding to a particle passing through the sensor is measured this way. However, by recording a signal (e.g., an electrical signal such as the current, voltage, resistance, inductance or impedance) passing through the aperture, the conductivity of the suspension passing through the aperture can be measured. Accordingly, the osmolality and change in osmolality over time determined using these measured values. Knowing the osmolality during calibration or during a test is inherently valuable.

## 27. Exemplified Embodiment of Pulse Analysis

As a cell passes through a sensor as described herein, the signal generated, typically an electronic pulse, contains information indicative of the characteristics of the shape of the cell and/or its induced change in shape. Typical bi-concave red cells appear much as Thurber's elephant under a blanket, with an initial peak, then a lower plateau, and then a trailing return to the baseline. Spherical cells appear taller, narrower, and symmetrical, but of similar width, appearing as an inverted parabola.

Analysis of the shape of the electronic pulse is an alternate means of determining the shape of a cell, and in particular when the cells are at a maximal size, or any other test osmolarities, and can be used in conjunction with, or instead of, pulse height measurements. For instance, the area under a pulse changes in proportion to a cells volume, and the volume recorded against the environment generates size, shape and volume measurements that are inherently useful and can be used for generating shape/osmolality curves as taught in U.S. Pat. No. 6,668,229 or used in International Application No. PCT/US19/64543, filed on December 4, 2019).

Our prior patent taught how to analyze populations of cells in a changing environment, based on sensor size measurements which are converted into volume measurements. Using pulse analysis, multiple characteristics of cells electrical parameters can be analyzed as they pass through a sensor, such as pulse height, width, symmetry and area under the pulse, creating similar but more detailed results.

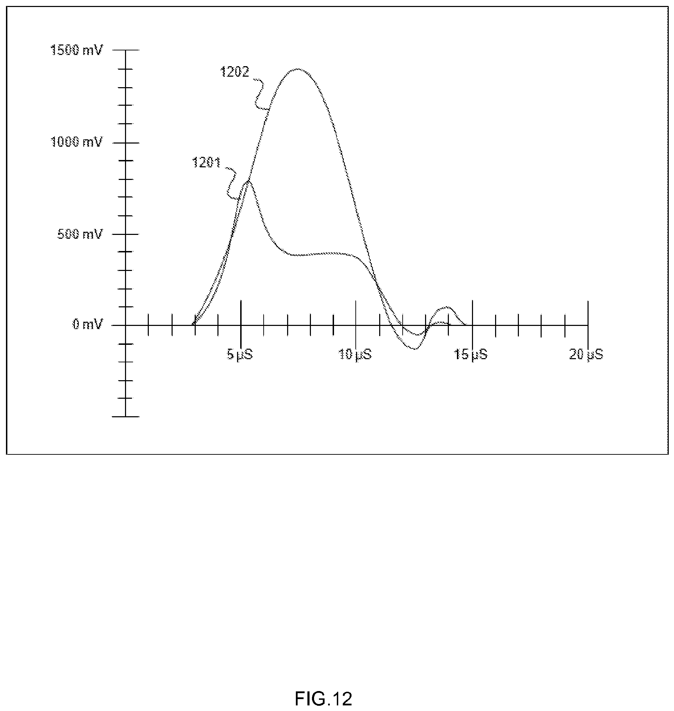

FIG. 12 is an exemplified embodiment of two cells (1201 and 1202) having passed through a sensor of an embodiment of an apparatus as described herein. FIG. 12 shows a typical pulse shape 1201 as a red cell in an isotonic suspension passes through a sensor. This pulse can be digitized and recorded, and a mean pulse shape for aliquots of cells in the same or changing environments compared to reveal the mean cell shape based on pulse analysis. Mean Cell Volume (MCV) which is typically based on the maximum pulse height, is replaced, or augmented by, the area under each pulse, or an average of such measurements, improving the volume measurements of cells, particularly in a changing environment. The point of maximal volume, is verified by the pulse profile 1202, which is symmetrical and of maximal area beneath its profile and of maximal height. Deviations between the peak pulse height and the pulse shape are indicative of disease.

Once the cell pulse profile is electronically captured, many forms of analysis and curve fitting can be performed to describes the features of each pulse, which may then be statistically or individually analyzed. The pulse peak height, such as seen used in prior patent U.S. Pat. No. 6,422,065, thus becomes one of many measurements that can be derived from a cell as it passes through a sensor.

## 28. Embodiment of Multiplexed Sensor And Syringe Testing Apparatus

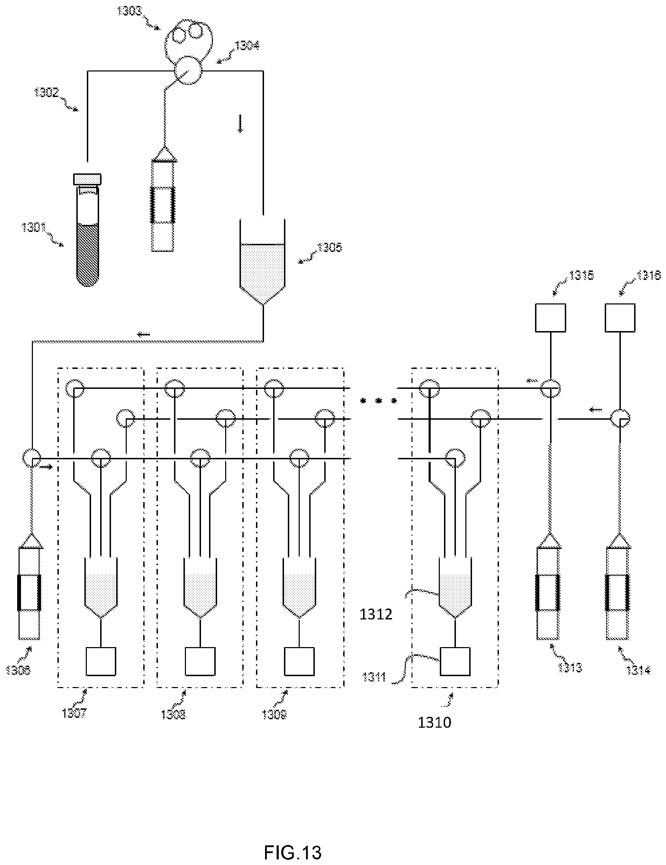

FIG. 13 shows an alternate embodiment of apparatus and methods described herein. In FIG. 13, a discrete series of diluents with a range of fixed osmolalities are mixed with aliquots from a test sample of whole blood. In an embodiment, the dilution the sample to a diluent is about 1:2000, about 1:3000, about 1:4000, about 1:5000, about 1:6000, about 1:7000, about 1:8000, about 1:10,000, about 1:12,000, about 1:14,000. In a preferred embodiment, the dilution ratio is about 1:10,000. As each mixture passed through a sensor, with the resultant size of the cell recorded.

The sample 1301 is prepared and diluted as is typical for cell sizing and counting applications or may have its dilution adjusted as described herein. A series of similar aliquots of cells are tested in similar cell testing arrangement 1307-1310, with one environmental factor, such as osmolality, altered by changing the ratios of, for example, saline 1315 and water 1316. Saline syringe 1313 and water syringe 1314 are metered to deliver range of different ratios of reagents into cell testing arrangement 1307-1310 creating a series of testing environments having different osmolalities into which similar volumes of cells are added, thereby creating suspensions of known volume, osmolality and whole blood volume.

For creating environments of varying osmolalities, the diluents are typically water and saline (e.g., phosphate buffered saline), which may be premixed with vortex mixes (described above) which is then mixed with the suspended blood sample in a second vortex mixing chamber, or other techniques, before dispensing into a holding vial 1312, for instance. In certain embodiments, mixing occurs directly in the holding vial 1312. In other embodiments, alternative mixing units as described herein and elsewhere may be used to mix the blood sample and the diluent. Without wishing to be bound to any particular theory, premixing the diluent and sample containing cells minimizes the osmotic shock to the cells and ensures uniform distribution of reagents and cells.

The suspension from vial 1312 is then tested as it passes through sensor 1311, which makes measurement of the features of the sample including the size of the cells, the concentration or number of cells in the sample, and other measurements as described herein. Such mixing and testing is preferably perform concurrently for each cell testing arrangement 1307-1310.

For clarity, only 4 series of cell testing arrangement 1302-1305 have been shown, but any number of cell testing arrangement 1302-1305 may be used. In certain embodiments, the testing sensor may be a single shared sensor between the arrangements. There are many well-known sample preparation techniques that can be used to create a series of known dilutions and environments, such as osmolality, that can alternatively be used.

The resulting data, when plotted for cell size against discrete osmolality, creates an approximation of the continuous data generated described in U.S. Provisional Patent Application No.: 62/775,703 "Apparatus and Controller-Implemented Method for Determining Physiological Condition Based on Measurement of Physical Cell Properties," and can be analyzed using well know curve fitting techniques to create a plot of osmolality against size.

## 29. Embodiment of Multiplexed Apparatus for Simultaneous Testing of Multiple Diluents

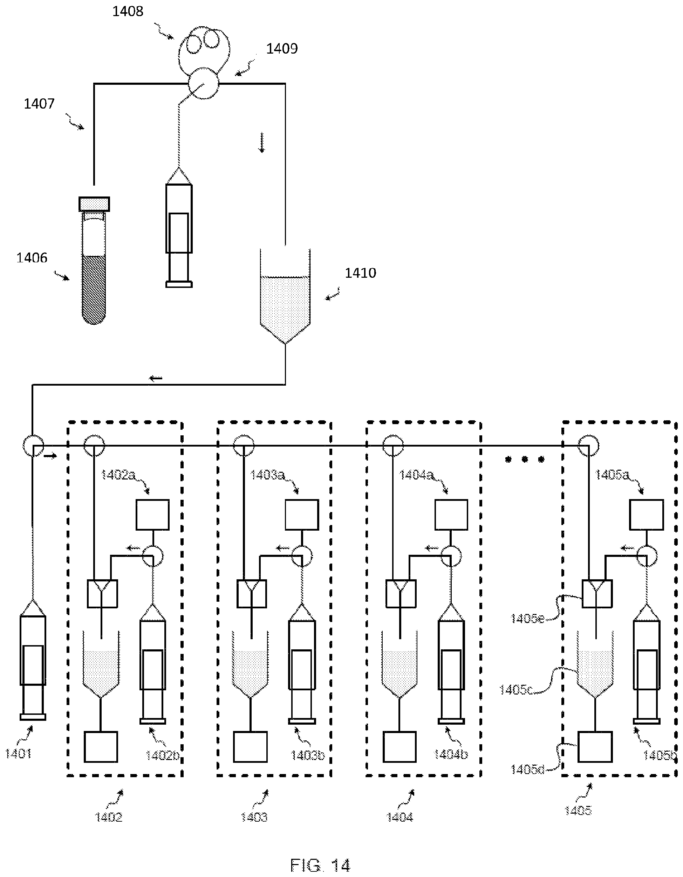

In another embodiment, the method and apparatus herein provides for a means to test a sample simultaneously using multiple environmental conditions. FIG. 14 shows an alternate implementation of the invention, where reagents 1402a-1405a used in cell testing arrangements 1402-1405 are pre-made, such that only a blood suspension 1401 and a single diluent of known tonicity, or other environmental factor, is used for each cell testing arrangement. This reduces the mixing to only that of the suspended sample 1401 and a single reagent 1402a, for example, allowing for higher throughput and simplification of the mechanical components. In certain embodiments, the diluent and sample may be mixed in a vortex mixer as described herein, for instance, at 1405e. In certain embodiments, the diluent and sample are mixed without a vortex mixer by simply combining the solution in the vials 1402a-1405d. Alternative mixing arrangement or mixing directly in the holding vial 1405c, for instance, produce similar results, but premixing ensures uniform distribution of reagents and cells.

In certain embodiments, each testing apparatus 1402-1405 is similar in terms of its physical setup. A known, uniform quantity of the sample 1406 is delivered to each vial with a known quantity of reagent 1402b-1405a by metering syringe 1402b-1405b and mixed during delivery into vial 1405c by mixing chamber 1405e, for instance. The sample in this newly mixed environment is then tested using a sensor 1405d, for instance, making measurement of the cell suspension features, such as cell size, cell count or other measurements. Such mixing and testing is preferably perform concurrently for each cell testing arrangement 1402-1405.

For clarity, only 4 series of cell testing arrangement 1402-1405 have been shown, but any number of cell testing arrangement 1402-1405 may be used. Likewise, the testing sensor may be a single shared sensor, or multiple discrete sensors as shown.

In certain embodiments, as the most informative parts of the data obtained regarding cell size occurs around 140 mOsm/kg, the population mean for a peak cell size for red blood cells, the distribution of the points of the discrete diluents do not need to be equally spaced. In preferable embodiments, there are more measured osmolalities close to this peak value. For instance, a useful series of ten diluents may have intervals of 20 mOsm/kg but near the population peak size value, where the diluents may have 5 mOsm/kg intervals, or any other useful interval. A typical series of ten discrete diluents could be 280, 230, 190, 170, 160, 150, 144, 140, 130 120, 100 mOsm/kg. It will be apparent to anyone well versed in the art that more or fewer diluents can be used, as well as a different distribution or number of mOsm. Indeed, as the peak size of cells is very well regulated, having fractions of a mOsm series around the expected peak can provide even greater precision for measuring the maximum cell size.

In another embodiment, a suspension of cells is created in a known tonicity while a different, series or changing diluents of known tonicity is added to the cells in a nonpressurized vessel [e.g., in a flask, not in a syringe or syringe like system]. The resulting change in cell properties and the suspending cells tonicity is recorded. In certain embodiments, the suspension may be made hyper- or hypotonic. In other embodiments, other physical properties may be altered in a known or measured way so as to induce a changes in cell shape. In certain embodiments, the diluent may be delivered continuously to the vessel by a syringe, a pulsing pump, such as a FMI pump, or by other fluid delivery method, and may incorporate active mixers or stirring mechanisms.

In a preferred embodiment, a hypertonic suspension of red cells is made to which a hypotonic diluent is added in slightly increasing volumes to compensate for volumes being flowed to the sensor. As the suspension becomes increasingly diluted, the osmolality of the solution of the suspension drops in a known manner. The sensor discretely or continuously samples the suspension measuring a property related to cell size while recording the osmolality. In certain preferred embodiments, the range of informative tonicities is varied from 280 to 60 mOsm/kg. In certain embodiments, samples comprising red blood cells may be added to maintain a consistent or desirable cell concentration.

The same principal can be used to alter a properties related to red blood cell cell size. A suspension of cells can be, without limitation, continuously heated, subjected to shear force, subjected to ultrasonic pressure, physical pressure, or subjected to other stresses that induce a changes in a property of red blood cells related to cell size, and then measured using a sensor either discretely or continuously on mean or cell by cell basis, creating a relationship between a cells environment and the cell's response to that environment.

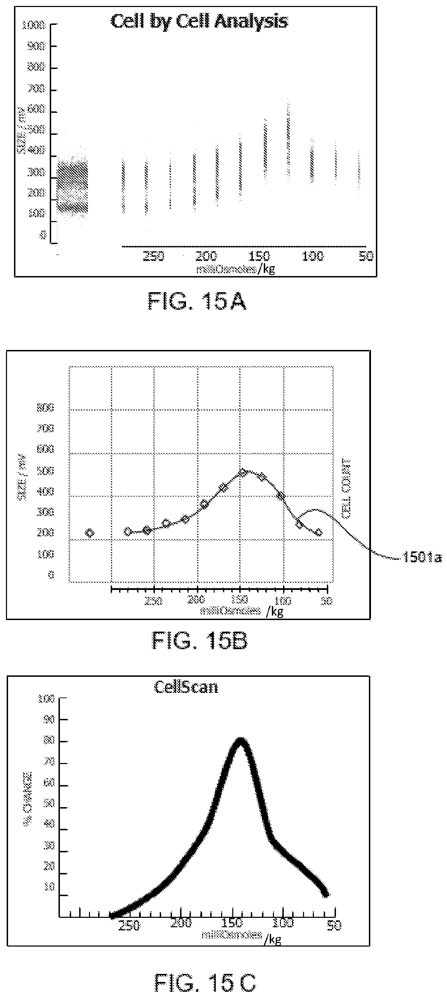

FIG. 15A shows results from a series of discrete measurements as displayed on a cell by cell plot. As discussed elsewhere herein, osmolality may be measured at discrete osmolalities. From this plot, individual mean cell sizes (e.g., as they are related to the measured voltage) are found and used to form a continuous curve, 1501a using standard curve fitting techniques (e.g., as seen in FIG. 15B). As can be seen, in FIG. 15B a good approximation of a continuous curve is achieved using discretized data. The continuous data may also be used to generate a "cell scan" plot of the data, where the percentage change of cell volume at each osmolality is calculated at each measured osmolality and a continuous curve may be generated based on the results at each osmolality interval (e.g., as seen in FIG. 15C).

The cell by cell data can be used to create a continuous data (not shown) by using statistical methods for averaging or infilling data from discrete populations. Such techniques are seen, for example, in creating weather maps and for similar mapping uses.

## 30. Exemplified Embodiment of Predetermined Window Filters for Data Analysis

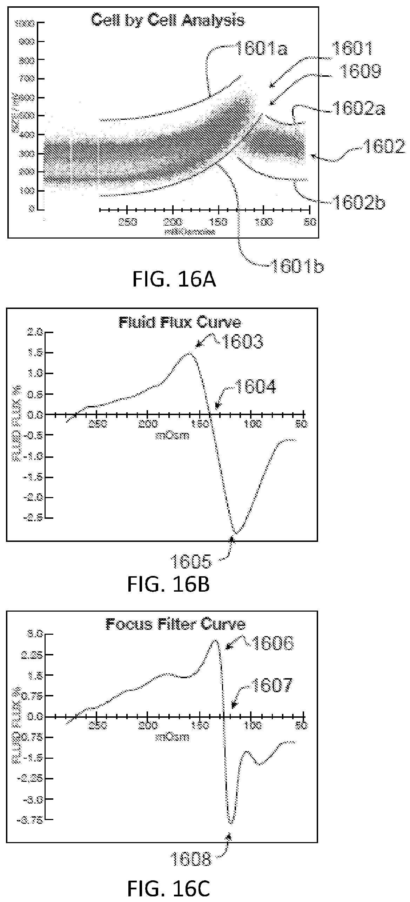

FIG. 16A-D are graphs of data collected and analyzed using the technologies as described herein. FIG. 16A is a cell-by-cell analysis showing voltage recorded by a sensor for individual red blood cells of a healthy individual over decreasing osmolality (in a range from 280 mOsm/kg to 54 mOsm/kg). Population density of cells is represented by color, with zero density corresponding to white, the lowest nonzero density corresponding to the darkest points (e.g., blue), and, as density progressively increases, color of the points lightens (e.g., from green to yellow to orange to red to black to aqua). Cell size and number against osmolality are recorded on a cell-by-cell basis is shown in FIG. 16A. The number of blood cells within each aliquot were counted (typically, e.g., at least 1000), and the cell-by-cell data was then used to produce an exact frequency distribution of cell size. Frequency distributions of each sample are conveniently displayed using different colors (e.g., a color map), as shown in FIG. 16A. In a cell-by-cell graph, population density is represented by color, with zero density corresponding to white, the lowest nonzero density corresponding to the darkest points (e.g., blue), and, as density progressively increases, color of the points lightens (e.g., from green to yellow to orange to red to black to aqua).

One feature of the cell-by-cell graph is that a portion of the graph is associated with intact cells 1601 (e.g., from about 300 mOsm/kg to about 70 mOsm/kg); during this period, the cell population is constant, then increase in volume slowly, and thereafter, the cell population increases in volume more rapidly coming to a maximum, and then falls. The static initial period is the result of cell's exposure to isotonic fluid, and the remainder is the result of exposure to progressive increase in osmotic stress.

"Pk0" coincides with the minimum absolute osmotic pressure (e.g., most hypotonic pressure) to which a cell can be subjected without loss of integrity. Pk0 can be identified by determining the right-most extent of the intact cell population in the cell-by-cell graph, i.e., the point of osmolality immediately preceding the point at which the cells ruptured. In FIG. 16A, this minimum pressure is the "peak". As the osmolality of the surrounding solution is reduced, the red blood cell ruptures and forms a ghost cell, which releases its contents into the surrounding medium.

In the cell-by-cell graph, there typically appears to the right of the expanding intact cell (EIC) population, a second and smaller cluster. This smaller cluster comprises "ghost cells," which are cells that have ruptured and thereafter resealed themselves (labeled 1602 in FIG. 16A). Between the EIC population and the ghost cell cluster appears a relatively colorless or cell free area, termed the "ghost gap" (labeled 1609 in FIG. 16A). The presence of a ghost gap is normal for cells of healthy individuals and is diminished or absent for individuals with certain types of physiological conditions.

Existing data collection and analysis collect and analyze the cell by cell distribution for a particular osmolality, and uses a fixed upper and lower cut-off or filter, to limit and/or remove undersized or oversized cells for the analysis data set. Cutoffs for intact cells parallel to the x-axis.

Additional information about cell properties can be derived from cell by cell tests in osmotic environments when a variable filter is used, preferably one that tracks cell populations. In FIG. 16A, a variable upper filter 1601a and variable lower filter 1601b is automatically generated based on a statistical measure of a population of cells, such as the mean or mode of a selection of cells. As mean cell size and/or cell size distribution changes, so do the filters, which limit measurements to an intact population measured within a particular range. Similarly, a ghost cell population 1602, can be made more distinct when analyzed with a variable upper filter 1602a and variable lower filter 1602b, which is automatically generated based on a statistical measure of a population of cells, such as a mean and/or mode of a selection of cells. As mean cell size and/or cell size distribution changes, so do the filters, which limit measurements to the intact population.

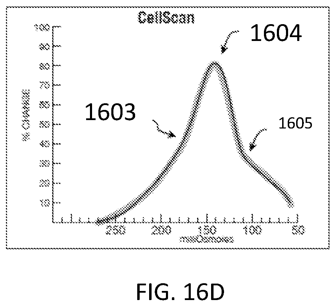

Data filtered and/or analyzed shows different results with a same population of cells analyzed. Such differences can be seen, for example, when analyzing fluid flux curves (FFCs). The Fluid Flux Curve (FFC) was determined by taking the first order derivative (with respect to osmolality) of a Cell Scan Plot (FIG. 16D). Using the technologies described herein, the cell-by-cell analysis (FIG. 16A) was converted into a plot of percent change of cell volume vs. osmolality ("Cell Scan Plot", e.g., FIG. 16D) by converting the individual peak voltage into a cell volume, then calculating a mean volume for each 100 cell volumes, and plotting the means to generate the Cell Scan Plot. A percentage change of cell volume at each osmolality is calculated and compared to a mean cell volume of an isotonic cell (e.g., FIG. 16D). On such a plot, Pk0 (see 1604) is the osmotic pressure at which the net water flow into the cell is zero (i.e., when a cell achieved its maximum volume, i.e., when it is a perfect sphere). As described herein, in some embodiments, Pk0 can be used as an indicator of an individual's health status.

In an FFC, Pk0 occurred at a zero crossing (1604), which was where the slope of the Cell Scan Plot changes from positive to negative. A positive value on the FFC represents a net flow of fluid into the cell, while negative rates represented a net flow of fluid out of the cell. In the FFC, the positive peak 1603 and negative peak 1605 corresponds to the maximum and minimum, respectively, on the FFC. As used herein, "Pymax" is the magnitude of fluid flux at the maximum, and "Pymin" is the magnitude of fluid flux at the minimum.

A comparison of the results from a mean cell size with fixed filters versus sample specific predetermined window filters is shown in FIG. 16B and FIG. 16C. FIG. 16B shows use of fixed filters and the same sample when tested with dynamic, sample-specific filters, termed a "window filter" is shown in FIG. 16C. The maximum inflow of fluids into the red blood cells (1603 of FIG. 16B, 1606 of FIG. 16c), the point of net zero fluid flow (1604 of FIG. 16B, 1607 of FIG. 16C) and the maximum outflow of fluids from the cells (1605 of FIG. 16B, 1608 of FIG. 16C) show different values when tested with fixed filters and dynamic sample-specific filters.

Other techniques of analyzing cell by cell graphs, or equivalent density distributions, can be used to determine other discriminators of an intact and/or ghost cell population. For instance, populations could be analyzed by looking at populations of cells within at perpendicular cuts to the line separating the two populations. For example, examining a radial section between 1601a and 1601b and 1602a and 1602b. This is facilitated by storing all of the data, determining the locations of the populations, and then analyzing the populations.

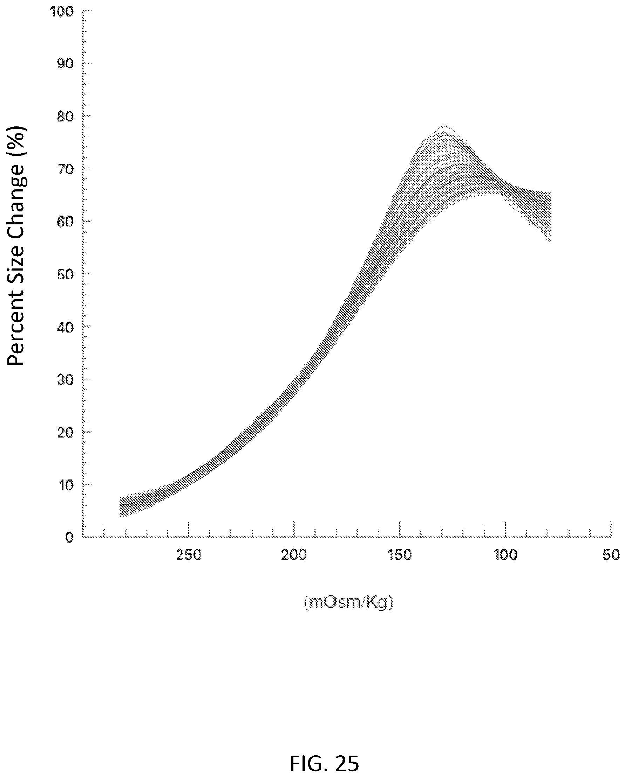

In certain embodiments, an informative measure is mean cell size at each osmolality. In some embodiments, mean cell size is generated by averaging cells size measurements over short intervals of about 1 mOsm, and plotted as a continuous curve of rate of change of size against osmolality. To improve the quality of the curve/data, data may be smoothed. For example, smoothing may be performed using a series of moving averages as is shown in FIG. 25. Smoothing performed on the data generates very smooth curves. While smoothing with moving averages produce smooth, continuous data, it does alter the shape of the curve and alters measurements. FIG. 25 shows the effect of smoothing repeatedly on same data for percent size change of cells against osmolality, plotting as a series of about 50 successive smoothings.

In some embodiments, an improved method of determining a best curve for the data points (e.g., in FIG. 25) is by fitting a curve to data points. One of skill in the art would be aware of various curve fitting methods and techniques. It was found that fitting an piecewise polynomial to the data points does not introduce artifacts into the data. Fitting an piecewise polynomial to data generated by methods and devices described herein generates a best fit function. In some embodiments, this kind of best fit function can provide particular benefits as it allows simple and precise determination of the peaks, minima, maxima and other features of the data. Further, coefficients generated using a piecewise polynomial function fully describe a curve's shape. For example, a piecewise polynomial may be used on data generated regarding a cell property such as cell shape, size, voltage, etc. In some embodiments, coefficients generated from a piecewise polynomial allow for classification of disease and/or grading of degree of abnormality. For example, coefficients may allow for classification of a disease or conditions a subject has as being cancer, malaria, pregnancy and/or other disease or condition (e.g., as discussed herein). Parameters currently generated from discrete data points could be generated with better accuracy and precision by using a polynomial in place of the discrete data points.

### 31. Exemplified Effects of Diluent Parameters and Cell Concentration on Measured Pk0

Using an embodiment of the method described herein, an effect of diluent and cell population being passed to the sensor was found to have an effect on the calculation of Pk0.

Regulating the osmolality of diluents is important. The osmolality of reagents for existing automated cell analyzers is poorly regulated, as it makes little difference to their test results. Worse, existing automated cell analyzers routinely make their reagents hypertonic, as it helps to inhibit organism growth. As a further improvement, reagents are made to more exacting standards. In certain embodiments, diluents are made within 4 mOsm/kg, within 3 mOsm/kg, within 1 mOsm/kg, within 0.1mOsm/kg, within 0.05 mOsm/kg or less of a target value. Prior systems used diluents made within 5mOsm/kg or less of the desired value. This variation results in poor repeatability of tests and unreliable results. Accordingly, it is desirable to keep environmental parameters and cell concentrations uniform between tests through using diluents which match more closely the environmental parameters of the biological sample, including without limitation, pH and osmolality.

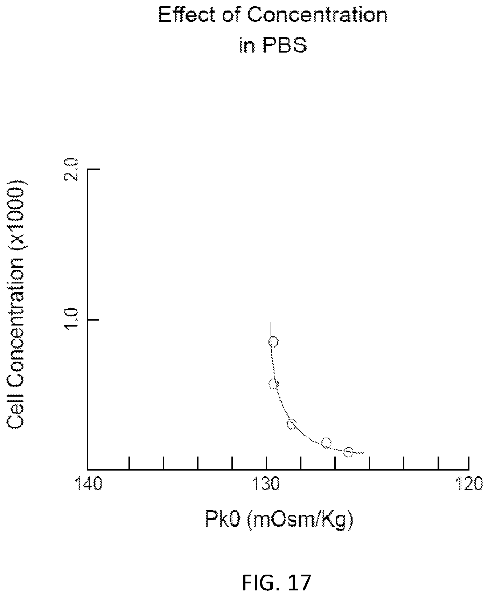

FIG. 17 shows a plot of Pk0 when using different concentrations of cells being flowed to the sensor. When the flow of cells to the sensor is less than about 500 cells/second, the Pk0 increases.

Furthermore, it is noted that there is an effect on cell concentration on the Pk0. The vertical axis of the two plots represents the concentration of cells being passed to the sensor of the apparatus during a test run. After cell flow to a sensor drops below a threshold value (about 500 cells/sec in the upper plot and about 400 cells/sec in the lower plot), the Pk0 of the red blood cells shifts to being lower than when measured at higher concentrations of cells.

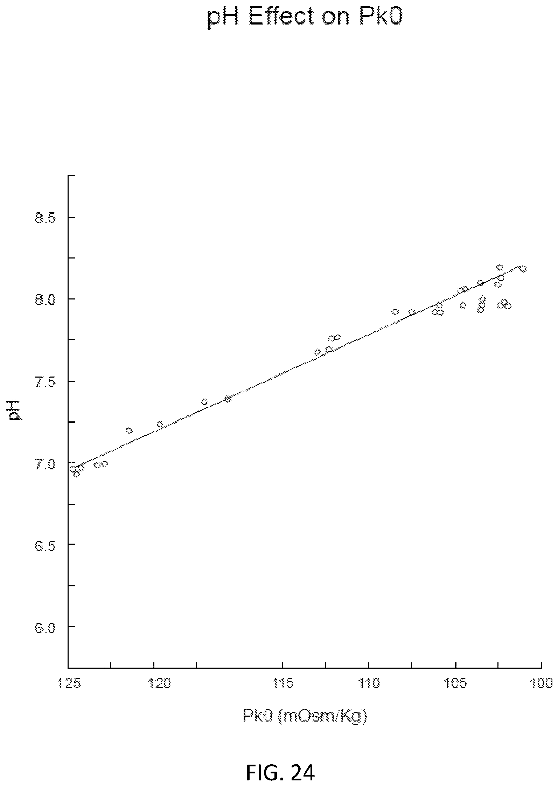

FIG. 24 shows the effect of pH on Pk0 as measured with an embodiment. As the pH of the testing environment increases, there is a concurrent decrease in the Pk0 of red blood cells. Accordingly, it is important to maintain cell concentration being delivered to a sensor of the apparatus at a certain, desirable threshold. In preferable embodiments, the desirable threshold of the cell concentration is the same or about the same for each sample tested. In certain embodiments, the desirable threshold is dependent on parameters (e.g., aperture size, incubation tube length, mixing time, pH, flow rate).

### 32. Computer System and Network Environment

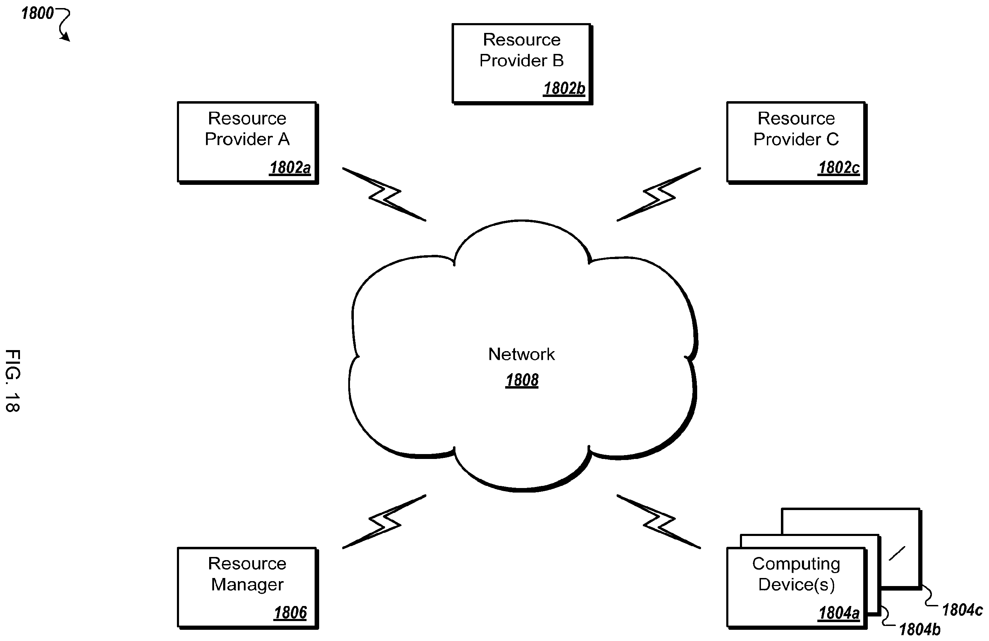

As shown in FIG. 18, an implementation of a network environment 1800 for use in providing systems, methods, and architectures for retrieving, managing, and analyzing clinical trial data from a plurality of sources as described herein is shown and described. In brief overview, referring now to FIG. 18, a block diagram of an exemplary cloud computing environment 1800 is shown and described. The cloud computing environment 1800 may include one or more resource providers 1802a, 1802b, 1802c (collectively, 1802). Each resource provider 1802 may include computing resources. In some implementations, computing resources may include any hardware and/or software used to process data. For example, computing resources may include hardware and/or software capable of executing algorithms, computer programs, and/or computer applications. In some implementations, exemplary computing resources may include application servers and/or databases with storage and retrieval capabilities. Each resource provider 1802 may be connected to any other resource provider 1802 in the cloud computing environment 1800. In some implementations, the resource providers 1802 may be connected over a computer network 1808. Each resource provider 1802 may be connected to one or more computing device 1804a, 1804b, 1804c (collectively, 1804), over the computer network 1808.

The cloud computing environment 1800 may include a resource manager 1806. The resource manager 1806 may be connected to the resource providers 1802 and the computing devices 1804 over the computer network 1808. In some implementations, the resource manager 1806 may facilitate the provision of computing resources by one or more resource providers 1802 to one or more computing devices 1804. The resource manager 1806 may receive a request for a computing resource from a particular computing device 1804. The resource manager 1806 may identify one or more resource providers 1802 capable of providing the computing resource requested by the computing device 1804. The resource manager 1806 may select a resource provider 1802 to provide the computing resource. The resource manager 1806 may facilitate a connection between the resource provider 1802 and a particular computing device 1804. In some implementations, the resource manager 1806 may establish a connection between a particular resource provider 1802 and a particular computing device 1804. In some implementations, the resource manager 1806 may redirect a particular computing device 1804 to a particular resource provider 1802 with the requested computing resource.

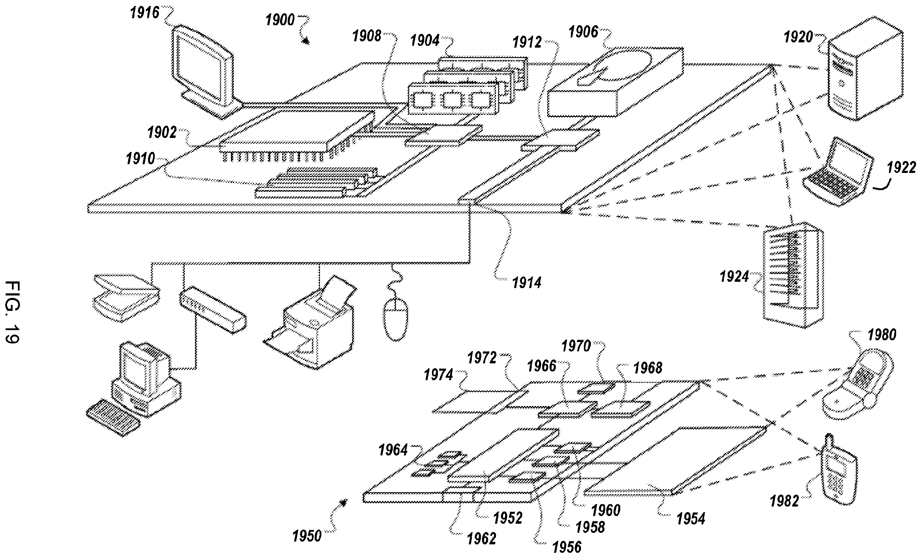

FIG. 19 shows an example of a computing device 1900 and a mobile computing device 1950 that can be used to implement the techniques described in this disclosure. The computing device 1900 is intended to represent various forms of digital computers, such as laptops, desktops, workstations, personal digital assistants, servers, blade servers, mainframes, and other appropriate computers. The mobile computing device 1950 is intended to represent various forms of mobile devices, such as personal digital assistants, cellular telephones, smart-phones, and other similar computing devices. The components shown here, their connections and relationships, and their functions, are meant to be examples only, and are not meant to be limiting.

The computing device 1900 includes a processor 1902, a memory 1904, a storage device 1906, a high-speed interface 1908 connecting to the memory 1904 and multiple high-speed expansion ports 1910, and a low-speed interface 1912 connecting to a low-speed expansion port 1914 and the storage device 1906. Each of the processor 1902, the memory 1904, the storage device 1906, the high-speed interface 1908, the high-speed expansion ports 1910, and the low-speed interface 1912, are interconnected using various busses, and may be mounted on a common motherboard or in other manners as appropriate. The processor 1902 can process instructions for execution within the computing device 1900, including instructions stored in the memory 1904 or on the storage device 1906 to display graphical information for a GUI on an external input/output device, such as a display 1916 coupled to the high-speed interface 1908. In other implementations, multiple processors and/or multiple buses may be used, as appropriate, along with multiple memories and types of memory.

The memory 1904 stores information within the computing device 1900. In some implementations, the memory 1904 is a volatile memory unit or units. In some implementations, the memory 1904 is a non-volatile memory unit or units. The memory 1904 may also be another form of computer-readable medium, such as a magnetic or optical disk.

The storage device 1906 is capable of providing mass storage for the computing device 1900. In some implementations, the storage device 1906 may be or contain a computer-readable medium, such as a floppy disk device, a hard disk device, an optical disk device, or a tape device, a flash memory or other similar solid state memory device, or an array of devices, including devices in a storage area network or other configurations. Instructions can be stored in an information carrier. The instructions, when executed by one or more processing devices (for example, processor 1902), perform one or more methods, such as those described above. The instructions can also be stored by one or more storage devices such as computer- or machine-readable mediums (for example, the memory 1904, the storage device 1906, or memory on the processor 1902).

The high-speed interface 1908 manages bandwidth-intensive operations for the computing device 1900, while the low-speed interface 1912 manages lower bandwidth-intensive operations. Such allocation of functions is an example only. In some implementations, the high-speed interface 1908 is coupled to the memory 1904, the display 1916 (e.g., through a graphics processor or accelerator), and to the high-speed expansion ports 1910, which may accept various expansion cards (not shown). In the implementation, the low-speed interface 1912 is coupled to the storage device 1906 and the low-speed expansion port 1914. The low-speed expansion port 1914, which may include various communication ports (e.g., USB, Bluetooth(t, Ethernet, wireless Ethernet) may be coupled to one or more input/output devices, such as a keyboard, a pointing device, a scanner, or a networking device such as a switch or router, e.g., through a network adapter.

The computing device 1900 may be implemented in a number of different forms, as shown in the figure. For example, it may be implemented as a standard server 1920, or multiple times in a group of such servers. In addition, it may be implemented in a personal computer such as a laptop computer 1922. It may also be implemented as part of a rack server system 1924. Alternatively, components from the computing device 1900 may be combined with other components in a mobile device (not shown), such as a mobile computing device 1950. Each of such devices may contain one or more of the computing device 1900 and the mobile computing device 1950, and an entire system may be made up of multiple computing devices communicating with each other.

The mobile computing device 1950 includes a processor 1952, a memory 1964, an input/output device such as a display 1954, a communication interface 1966, and a transceiver 1968, among other components. The mobile computing device 1950 may also be provided with a storage device, such as a micro-drive or other device, to provide additional storage. Each of the processor 1952, the memory 1964, the display 1954, the communication interface 1966, and the transceiver 1968, are interconnected using various buses, and several of the components may be mounted on a common motherboard or in other manners as appropriate.

The processor 1952 can execute instructions within the mobile computing device 1950, including instructions stored in the memory 1964. The processor 1952 may be implemented as a chipset of chips that include separate and multiple analog and digital processors. The processor 1952 may provide, for example, for coordination of the other components of the mobile computing device 1950, such as control of user interfaces, applications run by the mobile computing device 1950, and wireless communication by the mobile computing device 1950.

The processor 1952 may communicate with a user through a control interface 1958 and a display interface 1956 coupled to the display 1954. The display 1954 may be, for example, a TFT (Thin-Film-Transistor Liquid Crystal Display) display or an OLED (Organic Light Emitting Diode) display, or other appropriate display technology. The display interface 1956 may comprise appropriate circuitry for driving the display 1954 to present graphical and other information to a user. The control interface 1958 may receive commands from a user and convert them for submission to the processor 1952. In addition, an external interface 1962 may provide communication with the processor 1952, so as to enable near area communication of the mobile computing device 1950 with other devices. The external interface 1962 may provide, for example, for wired communication in some implementations, or for wireless communication in other implementations, and multiple interfaces may also be used.

The memory 1964 stores information within the mobile computing device 1950. The memory 1964 can be implemented as one or more of a computer-readable medium or media, a volatile memory unit or units, or a non-volatile memory unit or units. An expansion memory 1974 may also be provided and connected to the mobile computing device 1950 through an expansion interface 1972, which may include, for example, a SIMM (Single In Line Memory Module) card interface. The expansion memory 1974 may provide extra storage space for the mobile computing device 1950, or may also store applications or other information for the mobile computing device 1950. Specifically, the expansion memory 1974 may include instructions to carry out or supplement the processes described above, and may include secure information also. Thus, for example, the expansion memory 1974 may be provide as a security module for the mobile computing device 1950, and may be programmed with instructions that permit secure use of the mobile computing device 1950. In addition, secure applications may be provided via the SIMM cards, along with additional information, such as placing identifying information on the SIMM card in a nonhackable manner.

The memory may include, for example, flash memory and/or NVRAM memory (non-volatile random access memory), as discussed below. In some implementations, instructions are stored in an information carrier. that the instructions, when executed by one or more processing devices (for example, processor 1952), perform one or more methods, such as those described above. The instructions can also be stored by one or more storage devices, such as one or more computer- or machine-readable mediums (for example, the memory 1964, the expansion memory 1974, or memory on the processor 1952). In some implementations, the instructions can be received in a propagated signal, for example, over the transceiver 1968 or the external interface 1962.

The mobile computing device 1950 may communicate wirelessly through the communication interface 1966, which may include digital signal processing circuitry where necessary. The communication interface 1966 may provide for communications under various modes or protocols, such as GSM voice calls (Global System for Mobile communications), SMS (Short Message Service), EMS (Enhanced Messaging Service), or MMS messaging (Multimedia Messaging Service), CDMA (code division multiple access), TDMA (time division multiple access), PDC (Personal Digital Cellular), WCDMA (Wideband Code Division Multiple Access), CDMA2000, or GPRS (General Packet Radio Service), among others. Such communication may occur, for example, through the transceiver 1968 using a radio-frequency. In addition, short-range communication may occur, such as using a Bluetooth®, Wi-Fi™, or other such transceiver (not shown). In addition, a GPS (Global Positioning System) receiver module 1970 may provide additional navigation- and location-related wireless data to the mobile computing device 1950, which may be used as appropriate by applications running on the mobile computing device 1950.

The mobile computing device 1950 may also communicate audibly using an audio codec 1960, which may receive spoken information from a user and convert it to usable digital information. The audio codec 1960 may likewise generate audible sound for a user, such as through a speaker, e.g., in a handset of the mobile computing device 1950. Such sound may include sound from voice telephone calls, may include recorded sound (e.g., voice messages, music files, etc.) and may also include sound generated by applications operating on the mobile computing device 1950.

The mobile computing device 1950 may be implemented in a number of different forms, as shown in the figure. For example, it may be implemented as a cellular telephone 1980. It may also be implemented as part of a smart-phone 1982, personal digital assistant, or other similar mobile device.

Various implementations of the systems and techniques described here can be realized in digital electronic circuitry, integrated circuitry, specially designed ASICs (application specific integrated circuits), computer hardware, firmware, software, and/or combinations thereof. These various implementations can include implementation in one or more computer programs that are executable and/or interpretable on a programmable system including at least one programmable processor, which may be special or general purpose, coupled to receive data and instructions from, and to transmit data and instructions to, a storage system, at least one input device, and at least one output device.

These computer programs (also known as programs, software, software applications or code) include machine instructions for a programmable processor, and can be implemented in a high-level procedural and/or object-oriented programming language, and/or in assembly/machine language. As used herein, the terms machine-readable medium and computer-readable medium refer to any computer program product, apparatus and/or device (e.g., magnetic discs, optical disks, memory, Programmable Logic Devices (PLDs)) used to provide machine instructions and/or data to a programmable processor, including a machine-readable medium that receives machine instructions as a machine-readable signal. The term machine-readable signal refers to any signal used to provide machine instructions and/or data to a programmable processor.

To provide for interaction with a user, the systems and techniques described here can be implemented on a computer having a display device (e.g., a CRT (cathode ray tube) or LCD (liquid crystal display) monitor) for displaying information to the user and a keyboard and a pointing device (e.g., a mouse or a trackball) by which the user can provide input to the computer. Other kinds of devices can be used to provide for interaction with a user as well; for example, feedback provided to the user can be any form of sensory feedback (e.g., visual feedback, auditory feedback, or tactile feedback); and input from the user can be received in any form, including acoustic, speech, or tactile input.

The systems and techniques described here can be implemented in a computing system that includes a back end component (e.g., as a data server), or that includes a middleware component (e.g., an application server), or that includes a front end component (e.g., a client computer having a graphical user interface or a Web browser through which a user can interact with an implementation of the systems and techniques described here), or any combination of such back end, middleware, or front end components. The components of the system can be interconnected by any form or medium of digital data communication (e.g., a communication network). Examples of communication networks include a local area network (LAN), a wide area network (WAN), and the Internet.

The computing system can include clients and servers. A client and server are generally remote from each other and typically interact through a communication network. The relationship of client and server arises by virtue of computer programs running on the respective computers and having a client-server relationship to each other.

Elements of different implementations described herein may be combined to form other implementations not specifically set forth above. Elements may be left out of the processes, computer programs, databases, etc. described herein without adversely affecting their operation. In addition, the logic flows depicted in the figures do not require the particular order shown, or sequential order, to achieve desirable results. Various separate elements may be combined into one or more individual elements to perform the functions described herein.

Throughout the description, where apparatus and systems are described as having, including, or comprising specific components, or where processes and methods are described as having, including, or comprising specific steps, it is contemplated that, additionally, there are apparatus, and systems of the present invention that consist essentially of, or consist of, the recited components, and that there are processes and methods according to the present invention that consist essentially of, or consist of, the recited processing steps.

It should be understood that the order of steps or order for performing certain action is immaterial so long as the invention remains operable. Moreover, two or more steps or actions may be conducted simultaneously.

While the invention has been particularly shown and described with reference to specific preferred embodiments, it should be understood by those skilled in the art that various changes in form and detail may be made therein without departing from the spirit and scope of the invention as defined by the appended claims.

All publications, patents, and patent applications mentioned herein are hereby incorporated by reference in their entirety as if each individual publication, patent or patent application was specifically and individually indicated to be incorporated by reference.

**Equivalents**

The scope of the present invention is not intended to be limited to the above Description, but rather is as set forth in the following claims:

**Claims**

1. A method for measuring a concentration of cells in a biological sample and/or a physical property of cells of a biological sample using an apparatus, the method comprising:

diluting a first portion of a biological sample using a first diluent using a first dilution ratio;

flowing the diluted first portion of the biological sample along with a second diluent to an aperture (912, 722) of a sensor (424,513) at a first flow rate;

measuring a first property of each cell of a subset of cells of the diluted first portion of the biological sample having passed through the aperture (912, 722);

determining an approximate cell concentration of the biological sample based on the first flow rate and the first measured physical property of each cell of the subset of cells of the diluted first portion of the biological sample;

diluting a second portion of the biological sample using the first diluent and the second diluent at a second dilution ratio based on the approximate cell concentration;

flowing the diluted second portion to the aperture (912, 722) at a second flow rate;

measuring a second property of each cell of a subset of cells of the diluted second portion of the biological sample having passed through the aperture (912, 722); and

determining a concentration of cells in the biological sample and/or one or more physical properties of the cells of the biological sample based on the second measured property of each cell of the subset of cells from the diluted second portion of the biological sample, wherein the method comprises adding a plurality of microparticles to the biological sample.

2.  The method of claim 1, wherein

(a) the plurality of microparticles is **characterized by** a size distribution pattern, and/or

(b) optionally the microparticle sizes are more than 2 standard deviations from a mean of a size of a cell population of interest, wherein the standard deviation is the standard deviation from the mean of a known or predicted distribution of the cell population of interest, and/or

(c) optionally the method comprises:

measuring a physical property of the plurality of microparticles of the second portion of the biological sample having passed through the aperture (912, 722);

and,

determining the one or more physical properties of the cells of the biological sample based on the measured property of the plurality of microparticles having passed through the sensor (424,513),

optionally in case of (c) wherein the one or more determined physical properties of the cells comprises a volume of cells, a diameter of cells, a surface area of cells, and/or a hemoglobin concentration, and/or

optionally (d) wherein the method comprises:

determining one or more environmental properties to which the biological sample is exposed based on one or more measured physical properties of the microparticles having passed through the sensor (424,513),

optionally in case of (d) wherein the one or more environmental properties comprise pH, osmolality, flow rate, and/or mixing ratio.

3.  A method for measuring a concentration of cells in a biological sample and/or a physical property of cells of a biological sample using an apparatus, the method comprising:

diluting a first portion of a biological sample using a first diluent using a first dilution ratio;

flowing the diluted first portion of the biological sample along with a second diluent to an aperture (912, 722) of a sensor (424,513) at a first flow rate;

measuring a first property of each cell of a subset of cells of the diluted first portion of the biological sample having passed through the aperture (912, 722);

determining an approximate cell concentration of the biological sample based on the first flow rate and the first measured physical property of each cell of the subset of cells of the diluted first portion of the biological sample;

diluting a second portion of the biological sample using the first diluent and the second diluent at a second dilution ratio based on the approximate cell concentration;

flowing the diluted second portion to the aperture (912, 722) at a second flow rate;

measuring a second property of each cell of a subset of cells of the diluted second portion of the biological sample having passed through the aperture (912, 722); and

determining a concentration of cells in the biological sample and/or one or more physical properties of the cells of the biological sample based on the second measured property of each cell of the subset of cells from the diluted second portion of the biological sample, wherein the method comprises measuring the osmolality of the biological sample.

4. The method of any one of the preceding claims, wherein

(i) the cells comprise red blood cells, white blood cells, and/or platelets, and/or
(ii) the first and/or second measured property of each cell comprises impedance, resistance, voltage, a magnetic field or magnetic resonance change, a sound, an optical property, light scatter and/or light absorption,
(iii) the method comprises determining the one or more physical properties of the cells of the biological sample and wherein the one or more physical properties of the cells comprises a size, volume, a diameter, a surface area, and/or hemoglobin concentration, and/or
(iv) wherein the method comprises waiting a first predetermined period of time after diluting the first portion and prior to flowing the first portion to the aperture at the first flow rate,
optionally in case of (iv) wherein the first predetermined period of time comprises at least 5 minutes.

5. The method of any one of the preceding claims, wherein,

(i) the method comprises adjusting the osmolality of a first and/or second diluent to be within 0.1 mOsm/kg of the osmolality of the biological sample,
(ii) the method comprises:

measuring an optical property of the first portion of the diluted sample having passed to the aperture (912, 722) using an optical sensor (424,513); and,
determining one or more of: a flow rate to the aperture (912, 722), an osmolality, a dilution ratio of the sample to a first and/or second diluent and/or system delays of the first portion of the diluted sample passing through the aperture (912, 722) based on the optical property,
optionally in case of (ii) wherein the optical property of the diluted sample comprises fluorescence and/or an optical density.

6. A method for measuring a concentration and/or a physical property of cells of a biological sample using an apparatus, the method comprising:

flowing a biological sample through a channel of an apparatus at a first flow rate;
simultaneously flowing a first diluent through the channel of the apparatus at a second flow rate with the biological sample to create a diluted sample;
flowing the diluted sample to an aperture (912, 722) of a sensor (424,513);
measuring a first property of each cell of a subset of cells of a portion of the diluted sample having passed through the aperture (912, 722);
determining an approximate cell concentration of the biological sample based on the first and the second flow rates and/or the first measured property of each cell of the subset of cells of the portion of the diluted sample;
adjusting the first flow rate and/or the second flow rate to achieve a desired cell concentration flowing to the aperture (912, 722), based on the approximate cell concentration;
measuring a second property of each cell of a subset of cells of the portion of the diluted sample having passed through the aperture (912, 722) at the desired cell concentration; and
determining a concentration of cells of the biological sample and/or one or more physical properties of the cells of the biological sample based on, at least, the second measured property of each cell of the subset of cells from the portion of the diluted sample, wherein the method comprises adding a plurality of microparticles to the biological sample,
optionally wherein the method comprises measuring a property of each of the plurality of microparticles of the portion of the diluted sample having passed through the aperture (912, 722) at the desired cell density; and, determining, the one or more physical property of the cells of the biological sample based on the measured property of each of the plurality of microparticles having passed through the sensor.

7. A method for measuring a concentration and/or a physical property of cells of a biological sample using an apparatus, wherein the method comprises:

flowing a biological sample through a channel of an apparatus at a first flow rate;

simultaneously flowing a first diluent through the channel of the apparatus at a second flow rate with the biological sample to create a diluted sample;

flowing the diluted sample to an aperture (912, 722) of a sensor (424,513);

measuring a first property of each cell of a subset of cells of a portion of the diluted sample having passed through the aperture (912, 722);

determining an approximate cell concentration of the biological sample based on the first and the second flow rates and/or the first measured property of each cell of the subset of cells of the portion of the diluted sample;

adjusting the first flow rate and/or the second flow rate to achieve a desired cell concentration flowing to the aperture (912, 722), based on the approximate cell concentration;

measuring a second property of each cell of a subset of cells of the portion of the diluted sample having passed through the aperture (912, 722) at the desired cell concentration; and

determining a concentration of cells of the biological sample and/or one or more physical properties of the cells of the biological sample based on, at least, the second measured property of each cell of the subset of cells from the portion of the diluted sample,

wherein the method comprises measuring the osmolality of the biological sample, optionally wherein the method comprises adjusting the osmolality of the first diluent to be within 0.1mOsm/kg of the osmolality of the sample, and/or optionally wherein the method comprises adjusting the osmolality of the second diluent to be within 0.1mOsm/kg of the osmolality of the sample.

8. The method of any one of claims 6 or 7, wherein

(i) the method comprises determining the physical property of the cells of the biological sample and wherein the physical property of the cells comprises a size, volume, a diameter, and/or a surface area, and/or

(ii) the cells comprise one or more of red blood cells, white blood cells, and/or platelets, and/or

(iii) the method comprises diluting the biological sample using a second diluent at a first dilution ratio, and/or

(iv) the measured property of the cells is an impedance, a resistance, inductance, a current, a voltage, a magnetic field or magnetic resonance change, a sound, light scatter, and/or light absorption.

9. The method of any one of claims 6 to 8, wherein

(i) the method comprises automatically adjusting the first flow rate and/or the second flow rate to achieve and/or maintain the desired cell density of the portion of the diluted sample passing through the aperture (912, 722) of the sensor (424,513), and/or

(ii) wherein the method comprises automatically adjusting the first flow rate and/or the second flow rate to achieve a gradient of osmolalities of the portion of the diluted sample passing through the aperture (912, 722) of the sensor (424,513),

in case of (ii): optionally wherein the method further comprises determining a peak osmolality of the cells of the biological sample based on the measured property of each cell of the subset of cells from the portion of the diluted sample, and/or

in case of (i) or (ii): (a) optionally wherein diluting the biological sample comprises simultaneously flowing the second diluent through the channel at a third flow rate and automatically adjusting the third flow rate to achieve and/or maintain the desired cell concentration of the portion of the diluted sample passing through the aperture (912, 722) of the sensor (424,513), and/or

in case of (i) or (ii): (b) optionally wherein diluting the biological sample comprises simultaneously flowing the second diluent through the channel at a third flow rate and automatically adjusting the third flow rate to achieve a gradient of osmolalities of the portion of the diluted sample passing through the aperture (912, 722).

10. The method of any one of claims 6 to 9, wherein the method comprises measuring an optical property of the portion of the diluted sample having passed to the aperture (912, 722) using an optical sensor (424,513); and,

determining one or more of a dilution ratio of the sample to a first and/or second diluent, a flow rate, an osmolality, and/or a cell density based on, at least, the measured optical property,

(a) optionally wherein the optical property comprises fluorescence and/or optical density of the diluted sample, and/or

(b) optionally wherein the method comprises determining one or more physical properties of the cells of the biological sample based on the optical property, and/or

optionally in case of (b) wherein the one or more physical properties of the cells comprise size, shape, volume, diameter, and/or surface area, and/or

(c) optionally wherein the step of determining the density of cells of the biological sample and/or the one or more physical properties of the cells of the biological sample is further based on a comparison of a measured electrical property of each cell of the subset of cells from the portion of the diluted sample with a reference value,

optionally in case of (c) wherein the measured electrical property comprises impedance, resistance, inductance, current and/or voltage.

11. A method for measuring a concentration of cells in a biological sample and/or a physical property of cells of a biological sample using an apparatus, wherein the method comprises:

diluting a portion of a biological sample using a first diluent using a dilution ratio;

flowing the diluted portion to a sensor (424,513) of the apparatus along with a second diluent and a third diluent at a first combined flow rate;

measuring a first physical property of each cell of a subset of cells of the diluted portion of having passed through the aperture (912, 722) at the first combined flow rate;

determining an approximate cell concentration of the biological sample based on the first combined flow rate and the first measured physical property of each cell of the subset of cells of the diluted portion;

determining a second combined flow rate based on the approximate cell concentration;

flowing the biological sample, the second diluent, and the third diluent at a second combined flow rate to the aperture (912, 722);

measuring the first physical property of each cell of a subset of cells of the portion having passed through the aperture (912, 722) at the second combined flow rate; and

determining a concentration of cells in the biological sample and/or a second physical property of the cells of the biological sample based on the second physical property of each cell of the subset of cells from biological sample having flowed to the aperture (912, 722) using the second combined flow rate, wherein the method comprises adding a plurality of microparticles to the biological sample.

12. The method of claim 11, wherein a microparticle size is more than 2 standard deviations from the mean of the cell size of interest, wherein the standard deviation is the standard deviation from the mean of a known or predicted distribution of the cell population of interest.

13. The method of any one of claims 11 or 12, wherein the method comprises waiting a predetermined period of time prior to flowing the portion to the aperture (912, 722) at the first combined flow rate, optionally wherein the predetermined period of time is at least 5 minutes.

14. The method of any one of claims 11 to 13, wherein

(i) the method comprises measuring a physical property of the plurality of microparticles of the portion of the biological sample having passed through the aperture (912, 722) when flowed to the aperture (912, 722) at the second combined flow rate; and,

determining one or more physical properties of the cells of the biological sample based on, at least, the measured physical property of the plurality of microparticles having passed through the sensor (424,513), and/or

(ii) the method comprises measuring the osmolality of the biological sample,

optionally in case of (ii) wherein the method comprises adjusting the osmolality of the first diluent to be within 0.1mOsm/kg of the osmolality of the biological sample.

**Patentansprüche**

1. Verfahren zum Messen einer Konzentration von Zellen in einer biologischen Probe und/oder einer physikalischen Eigenschaft von Zellen einer biologischen Probe unter Verwendung einer Vorrichtung, wobei das Verfahren umfasst:

Verdünnung eines ersten Teils einer biologischen Probe mit einem ersten Verdünnungsmittel unter Verwendung eines ersten Verdünnungsverhältnisses;

Fließen lassen des verdünnten ersten Teils der biologischen Probe zusammen mit einem zweiten Verdünnungsmittel mit einer ersten Durchflussrate zu einer Apertur (912, 722) eines Sensors (424, 513);

Messen einer ersten Eigenschaft jeder Zelle einer Untergruppe von Zellen des verdünnten ersten Teils der biologischen Probe, die die Apertur passiert hat (912, 722);

Bestimmen einer ungefähren Zellkonzentration der biologischen Probe auf der Grundlage der ersten Durchflussrate und der ersten gemessenen physikalischen Eigenschaft jeder Zelle der Untergruppe von Zellen des verdünnten ersten Teils der biologischen Probe;

Verdünnen eines zweiten Teils der biologischen Probe unter Verwendung des ersten Verdünnungsmittels und des zweiten Verdünnungsmittels in einem zweiten Verdünnungsverhältnis auf der Grundlage der ungefähren Zellkonzentration;

Fließen lassen des verdünnten zweiten Teils mit einer zweiten Durchflussrate zur Apertur (912, 722);

Messen einer zweiten Eigenschaft jeder Zelle einer Untergruppe von Zellen des verdünnten zweiten Teils der biologischen Probe, die die Apertur passiert hat (912, 722); und

Bestimmen einer Konzentration von Zellen in der biologischen Probe und/oder einer oder mehrerer physikalischer Eigenschaften der Zellen der biologischen Probe basierend auf der zweiten gemessenen Eigenschaft jeder Zelle der Untergruppe von Zellen aus dem verdünnten zweiten Teil der biologischen Probe, wobei das Verfahren das Hinzufügen einer Vielzahl von Mikropartikeln zu der biologischen Probe umfasst.

2. Verfahren nach Anspruch 1, wobei

a) die Mehrzahl der Mikropartikel durch ein Größenverteilungsmuster gekennzeichnet ist und/oder

(b) optional beträgt die Mikropartikelgrößen mehr als 2 Standardabweichungen von einem Mittelwert der Größe einer interessierenden Zellpopulation, wobei die Standardabweichung die Standardabweichung vom Mittelwert einer bekannten oder vorhergesagten Verteilung der interessierenden Zellpopulation ist, und/oder

c) optional umfasst das Verfahren:

Messen einer physikalischen Eigenschaft der Mehrzahl von Mikropartikeln des zweiten Teils der biologischen Probe, die die Apertur passiert haben (912, 722); und

Bestimmen einer oder mehrerer physikalischer Eigenschaften der Zellen der biologischen Probe auf der Grundlage der gemessenen Eigenschaft der Vielzahl von Mikropartikeln, die den Sensor passiert haben (424, 513),

optional im Fall von (c), wobei die eine oder mehrere bestimmte physikalischen Eigenschaften der Zellen ein Zellvolumen, einen Zelldurchmesser, eine Oberfläche von Zellen und/oder eine Hämoglobinkonzentration umfassen und/oder

optional (d), wobei das Verfahren umfasst:

Bestimmen einer oder mehrerer Umgebungseigenschaften, denen die biologische Probe ausgesetzt ist, basierend auf einer oder mehreren gemessenen physikalischen Eigenschaften der Mikropartikel, die den Sensor passiert haben (424, 513),

optional im Fall von (d), wobei die eine oder mehrere Umgebungseigenschaften den pH-Wert, die Osmolalität, die Durchflussrate und/oder das Mischungsverhältnis umfassen.

3. Verfahren zum Messen einer Konzentration von Zellen in einer biologischen Probe und/oder einer physikalischen Eigenschaft von Zellen einer biologischen Probe unter Verwendung einer Vorrichtung, wobei das Verfahren umfasst:

Verdünnung eines ersten Teils einer biologischen Probe mit einem ersten Verdünnungsmittel unter Verwendung eines ersten Verdünnungsverhältnisses;

Fließen lassen des verdünnten ersten Teils der biologischen Probe zusammen mit einem zweiten Verdünnungsmittel zu einer Apertur (912, 722) eines Sensors (424, 513) mit einer ersten Durchflussrate;

Messen einer ersten Eigenschaft jeder Zelle einer Untergruppe von Zellen des verdünnten ersten Teils der biologischen Probe, die die Apertur passiert hat (912, 722);

Bestimmen einer ungefähren Zellkonzentration der biologischen Probe auf der Grundlage der ersten Flussrate und der ersten gemessenen physikalischen Eigenschaft jeder Zelle der Untergruppe von Zellen des verdünnten ersten Teils der biologischen Probe;

Verdünnen eines zweiten Teils der biologischen Probe unter Verwendung des ersten Verdünnungsmittels und des zweiten Verdünnungsmittels in einem zweiten Verdünnungsverhältnis auf der Grundlage der ungefähren Zellkonzentration;

Fließen lassen des verdünnten zweiten Teils zur Apertur (912, 722) mit einer zweiten Durchflussrate;

Messen einer zweiten Eigenschaft jeder Zelle einer Untergruppe von Zellen des verdünnten zweiten Teils der biologischen Probe, die die Apertur passiert hat (912, 722); und

Bestimmen einer Konzentration von Zellen in der biologischen Probe und/oder einer oder mehrerer physikalischer Eigenschaften der Zellen der biologischen Probe basierend auf der zweiten gemessenen Eigenschaft jeder Zelle der Untergruppe von Zellen aus dem verdünnten zweiten Teil der biologischen Probe, wobei das Verfahren die Messung der Osmolalität der biologischen Probe umfasst.

4.  Verfahren nach einem der vorhergehenden Ansprüche, wobei

(i) die Zellen roten Blutkörperchen, weißen Blutkörperchen und/oder Blutplättchen umfassen, und/oder

(ii) die erste und/oder zweite gemessene Eigenschaft jeder Zelle umfasst die Impedanz, den Widerstand, die Spannung, ein Magnetfeld oder eine magnetische Resonanzänderung, einen Schall, eine optische Eigenschaft, Lichtstreuung und/oder Lichtabsorption,

(iii) das Verfahren der Bestimmung einer oder mehrerer physikalischer Eigenschaften der Zellen der biologischen Probe umfasst, wobei die eine oder mehrere physikalische Eigenschaften der Zellen eine Größe, ein Volumen, einen Durchmesser, eine Oberfläche und/oder eine Hämoglobinkonzentration umfassen und/oder

(iv) wobei das Verfahren das Abwarten einer ersten vorbestimmten Zeitspanne nach dem Verdünnen der ersten Portion und vor dem Fließen lassen der ersten Portion zur Apertur mit der ersten Flussrate umfasst,

optional im Falle von (iv), wobei der erste vorbestimmte Zeitraum mindestens 5 Minuten umfasst.

5.  Verfahren nach einem der vorhergehenden Ansprüche, wobei

(i) das Verfahren umfasst die Einstellung der Osmolalität eines ersten und/oder zweiten Verdünnungsmittels auf einen Wert von 0,1 mOsm/kg von der Osmolalität der biologischen Probe,

(ii) das Verfahren umfasst:

Messen einer optischen Eigenschaft des ersten Teils der verdünnten Probe, der zur Apertur (912, 722) gelangt ist, unter Verwendung eines optischen Sensors (424, 513); und

Bestimmen einer oder mehrerer der folgenden Angaben: einer Durchflussrate zur Apertur (912, 722), einer Osmolalität, eines Verdünnungsverhältnisses der Probe zu einem ersten und/oder zweiten Verdünnungsmittel und/oder einer Systemverzögerung des ersten Teils der verdünnten Probe, der die Apertur (912, 722) passiert, basierend auf der optischen Eigenschaft,

optional im Fall von (ii), wobei die optische Eigenschaft der verdünnten Probe Fluoreszenz und/oder eine optische Dichte umfasst.

6.  Verfahren zum Messen einer Konzentration und/oder einer physikalischen Eigenschaft von Zellen einer biologischen Probe mit Hilfe einer Vorrichtung, wobei das Verfahren umfasst:

Fließen lassen einer biologischen Probe durch einen Kanal einer Vorrichtung mit einer ersten Durchflussrate;

gleichzeitiges Fließen lassen eines ersten Verdünnungsmittels durch den Kanal der Vorrichtung mit einer zweiten Durchflussrate mit der biologischen Probe, um eine verdünnte Probe zu erzeugen;

Fließen lassen der verdünnten Probe zu einer Apertur (912, 722) eines Sensors (424, 513);

Messen einer ersten Eigenschaft jeder Zelle einer Untergruppe von Zellen eines Teils der verdünnten Probe, der die Apertur passiert hat (912, 722);

Bestimmen einer ungefähren Zellkonzentration der biologischen Probe auf der Grundlage der ersten und der zweiten Flussrate und/oder der ersten gemessenen Eigenschaft jeder Zelle der Untergruppe von Zellen des Teils der verdünnten Probe;

Einstellen der ersten Durchflussrate und/oder der zweiten Durchflussrate, um eine gewünschte Zellkonzentration zu erreichen, die zur Apertur (912, 722) fließt, basierend auf der ungefähren Zellkonzentration;

Messen einer zweiten Eigenschaft jeder Zelle einer Untergruppe von Zellen des Teils der verdünnten Probe, der die Apertur (912, 722) bei der gewünschten Zellkonzentration passiert hat; und

Bestimmen einer Konzentration von Zellen der biologischen Probe und/oder einer oder mehrerer physikalischer Eigenschaften der Zellen der biologischen Probe auf der Grundlage mindestens der zweiten gemessenen Eigenschaft jeder Zelle der Untergruppe von Zellen aus dem Teil der verdünnten Probe, wobei das Verfahren das Hinzufügen einer Vielzahl von Mikropartikeln zu der biologischen Probe umfasst,

optional wobei das Verfahren das Messen einer Eigenschaft jedes der mehreren Mikropartikel des Teils der verdünnten Probe umfasst, der die Apertur (912, 722) bei der gewünschten Zelldichte passiert hat; und

Bestimmen der einen oder mehrerer physikalischer Eigenschaften der Zellen der biologischen Probe auf der Grundlage der gemessenen Eigenschaft jedes der mehreren Mikropartikel, die den Sensor passiert haben.

7. Verfahren zum Messen einer Konzentration und/oder einer physikalischen Eigenschaft von Zellen einer biologischen Probe unter Verwendung einer Vorrichtung, wobei das Verfahren umfasst:

Fließen lassen einer biologischen Probe durch einen Kanal einer Vorrichtung mit einer ersten Durchflussrate; gleichzeitiges Fließen lassen eines ersten Verdünnungsmittels durch den Kanal der Vorrichtung mit einer zweiten Durchflussrate mit der biologischen Probe, um eine verdünnte Probe zu erzeugen;
Fließen lassen der verdünnten Probe zu einer Apertur (912, 722) eines Sensors (424, 513);
Messen einer ersten Eigenschaft jeder Zelle einer Untergruppe von Zellen eines Teils der verdünnten Probe, der die Apertur passiert hat (912, 722);
Bestimmen einer ungefähren Zellkonzentration der biologischen Probe auf der Grundlage der ersten und der zweiten Flussrate und/oder der ersten gemessenen Eigenschaft jeder Zelle der Untergruppe von Zellen des Teils der verdünnten Probe;
Einstellen der ersten Flussrate und/oder der zweiten Flussrate, um eine gewünschte Zellkonzentration zu erreichen, die zur Apertur (912, 722) fließt, basierend auf der ungefähren Zellkonzentration;
Messen einer zweiten Eigenschaft jeder Zelle einer Untergruppe von Zellen des Teils der verdünnten Probe, der die Apertur (912, 722) bei der gewünschten Zellkonzentration passiert hat; und
Bestimmen einer Konzentration von Zellen der biologischen Probe und/oder einer oder mehrerer physikalischer Eigenschaften der Zellen der biologischen Probe auf der Grundlage mindestens der zweiten gemessenen Eigenschaft jeder Zelle der Untergruppe von Zellen aus dem Teil der verdünnten Probe,
wobei das Verfahren das Messen der Osmolalität der biologischen Probe umfasst, optional wobei das Verfahren das Einstellen der Osmolalität des ersten Verdünnungsmittels umfasst, um innerhalb von 0,1 mOsm/kg von der Osmolalität der Probe zu liegen, und/oder gegebenenfalls wobei das Verfahren das Einstellen der Osmolalität des zweiten Verdünnungsmittels umfasst, um innerhalb von 0,1 mOsm/kg der Osmolalität der Probe zu liegen.

8. Verfahren nach einem der Ansprüche 6 oder 7, wobei

(i) das Verfahren die Bestimmung der physikalischen Eigenschaft der Zellen der biologischen Probe umfasst, wobei die physikalische Eigenschaft der Zellen eine Größe, ein Volumen, einen Durchmesser und/oder eine Oberfläche umfasst
und/oder
(ii) die Zellen umfassen ein oder mehrere rote Blutkörperchen, weiße Blutkörperchen und/oder Blutplättchen
und/oder
(iii) das Verfahren umfasst die Verdünnung der biologischen Probe mit einem zweiten Verdünnungsmittel in einem ersten Verdünnungsverhältnis und/oder
(iv) die gemessene Eigenschaft der Zellen ist eine Impedanz, ein Widerstand, eine Induktivität, ein Strom, eine Spannung, eine Magnetfeld- oder Magnetresonanzänderung, ein Schall, Lichtstreuung und/oder Lichtabsorption.

9. Verfahren nach einem der Ansprüche 6 bis 8, wobei

(i) das Verfahren umfasst die automatische Anpassung der ersten Durchflussrate und/oder der zweiten Durchflussrate, um die gewünschte Zelldichte des Teils der verdünnten Probe zu erreichen und/oder aufrechtzuerhalten, der durch die Apertur (912, 722) des Sensors (424, 513) strömt, und/oder
(ii) wobei das Verfahren das automatische Einstellen der ersten Flussrate und/oder der zweiten Flussrate umfasst, um einen gewünschten Gradienten der Osmolalitäten des Teils der verdünnten Probe zu erreichen und/oder zu erhalten, der durch die Apertur (912, 722) des Sensors (424, 513) strömt,
im Fall von (ii): optional, wobei das Verfahren ferner das Bestimmen einer Peak-Osmolalität der Zellen der biologischen Probe auf der Grundlage der gemessenen Eigenschaft jeder Zelle der Untergruppe von Zellen aus dem Teil der verdünnten Probe umfasst, und/oder
im Fall von (i) oder (ii): (a) optional wobei die Verdünnung der biologischen Probe das gleichzeitige Fließen des zweiten Verdünnungsmittels durch den Kanal mit einer dritten Durchflussrate und das automatische Einstellen der dritten Durchflussrate umfasst, um die gewünschte Zellkonzentration des Teils der verdünnten Probe, der durch die Apertur (912, 722) des Sensors (424, 513) strömt, zu erreichen und/oder aufrechtzuerhalten, und/oder
im Fall von (i) oder (ii): (b) optional wobei die Verdünnung der biologischen Probe das
gleichzeitige Fließen des zweiten Verdünnungsmittels durch den Kanal mit einer dritten Durchflussrate und das automatische Einstellen der dritten Durchflussrate umfasst, um einen Gradienten der Osmolalitäten des Teils der verdünnten Probe zu erreichen, der durch die Apertur (912, 722) fließt.

10. Verfahren nach einem der Ansprüche 6 bis 9, wobei das Verfahren das Messen einer optischen Eigenschaft des Teils der verdünnten Probe, der zur Apertur (912, 722) gelangt ist, unter Verwendung eines optischen Sensors (424, 513); und

Bestimmen eines oder mehrerer Verdünnungsverhältnisse der Probe zu einem ersten und/oder zweiten Verdünnungsmittel, einer Flussrate, einer Osmolalität und/oder einer Zelldichte zumindest auf der Grundlage der gemessenen optischen Eigenschaft umfasst,

(a) optional wobei die optische Eigenschaft die Fluoreszenz und/oder die optische Dichte der verdünnten Probe umfasst, und/oder
(b) optional wobei das Verfahren das Bestimmen einer oder mehrerer physikalischer Eigenschaften der Zellen der biologischen Probe auf der Grundlage der optischen Eigenschaft umfasst, und/oder
optional im Fall von (b), wobei eine oder mehrere physikalische Eigenschaften der Zellen Größe, Form, Volumen, Durchmesser und/oder Oberfläche umfassen und/oder
(c) optional, wobei der Schritt des Bestimmens der Zelldichte der biologischen Probe und/oder der einen oder mehrerer physikalischer Eigenschaften der Zellen der biologischen Probe weiterhin auf einem Vergleich einer gemessenen elektrischen Eigenschaft jeder Zelle der Untergruppe von Zellen aus dem Teil der verdünnten Probe mit einem Referenzwert basiert,

optional im Fall von (c), wobei die gemessene elektrische Eigenschaft Impedanz, Widerstand, Induktivität, Strom und/oder Spannung umfasst.

11. Verfahren zum Messen einer Konzentration von Zellen in einer biologischen Probe und/oder einer physikalischen Eigenschaft von Zellen einer biologischen Probe unter Verwendung einer Vorrichtung, wobei das Verfahren umfasst:

Verdünnen eines Teils einer biologischen Probe mit einem ersten Verdünnungsmittel unter Verwendung eines Verdünnungsverhältnisses;
Fließen lassen des verdünnten Teils zu einem Sensor (424,513) der Vorrichtung zusammen mit einem zweiten Verdünnungsmittel und einem dritten Verdünnungsmittel bei einer ersten kombinierten Durchflussrate;
Messen einer ersten physikalischen Eigenschaft jeder Zelle einer Untergruppe von Zellen des verdünnten Teils, die die Apertur (912, 722) mit der ersten kombinierten Durchflussrate passiert haben;
Bestimmen einer ungefähren Zellkonzentration der biologischen Probe auf der Grundlage der ersten kombinierten Durchflussrate und der ersten gemessenen physikalischen Eigenschaft jeder Zelle der Untergruppe von Zellen des verdünnten Teils;
Bestimmen einer zweiten kombinierten Durchflussrate basierend auf der ungefähren Zellkonzentration;
Fließen lassen der biologischen Probe, des zweiten Verdünnungsmittels und des dritten Verdünnungsmittels mit einer zweiten kombinierten Durchflussrate zur Apertur (912, 722);
Messen der ersten physikalischen Eigenschaft jeder Zelle einer Untergruppe von Zellen des Teils, der die Apertur (912, 722) bei der zweiten kombinierten Durchflussrate passiert haben; und
Bestimmen einer Konzentration von Zellen in der biologischen Probe und/oder einer zweiten physikalischen Eigenschaft der Zellen der biologischen Probe basierend auf der zweiten physikalischen Eigenschaft jeder Zelle der Untergruppe von Zellen der biologischen Probe, die zur Apertur (912, 722) geflossen sind, unter Verwendung der zweiten kombinierten Durchflussrate, wobei das Verfahren das Hinzufügen einer Vielzahl von Mikropartikeln zu der biologischen Probe umfasst.

12. Verfahren nach Anspruch 11, wobei eine Mikropartikelgröße mehr als 2 Standardabweichungen vom Mittelwert der interessierenden Zellgröße beträgt, wobei die Standardabweichung die Standardabweichung vom Mittelwert einer bekannten oder vorhergesagten Verteilung der interessierenden Zellpopulation ist.

13. Verfahren nach einem der Ansprüche 11 oder 12, wobei das Verfahren das Abwarten einer vorbestimmten Zeitspanne umfasst, bevor der Teil bei der ersten kombinierten Durchflussrate zur Apertur (912, 722) fließt, gegebenenfalls wobei die vorbestimmte Zeitspanne mindestens 5 Minuten beträgt.

14. Verfahren nach einem der Ansprüche 11 bis 13, wobei

(i) das Verfahren umfasst das Messen einer physikalischen Eigenschaft der Mehrzahl von Mikropartikeln des Teils der biologischen Probe, der die Apertur (912, 722) passiert hat, wenn er mit der zweiten kombinierten Durchflussrate zur Apertur (912, 722) geströmt ist; und

Bestimmen einer oder mehrerer physikalischer Eigenschaften der Zellen der biologischen Probe zumindest auf der Grundlage der gemessenen physikalischen Eigenschaft der Vielzahl von Mikropartikeln, die den Sensor passiert haben (424, 513), und/oder

(ii) die Methode umfasst die Messung der Osmolalität der biologischen Probe,

optional im Fall von (ii), wobei das Verfahren das Einstellen der Osmolalität des ersten Verdünnungsmittels umfasst, um innerhalb von 0,1 mOsm/kg von der Osmolalität der biologischen Probe zu liegen.

## Revendications

1. Procédé de mesure d'une concentration de cellules dans un échantillon biologique et/ou d'une propriété physique des cellules d'un échantillon biologique à l'aide d'un appareil, le procédé comprenant :

dilution d'une première portion d'un échantillon biologique à l'aide d'un premier diluant à l'aide d'un premier taux de dilution ;

faire couler la première portion diluée de l'échantillon biologique avec un second diluant vers une ouverture (912, 722) d'un capteur (424, 513) à un premier débit ;

mesure d'une première propriété de chaque cellule d'un sous-ensemble de cellules de la première partie diluée de l'échantillon biologique ayant traversé l'ouverture (912, 722) ;

la détermination d'une concentration cellulaire approximative de l'échantillon biologique sur la base du premier débit et de la première propriété physique mesurée de chaque cellule du sous-ensemble de cellules de la première partie diluée de l'échantillon biologique ;

diluer une deuxième partie de l'échantillon biologique à l'aide du premier diluant et du deuxième diluant à un deuxième rapport de dilution basé sur la concentration cellulaire approximative ;

écoulement de la deuxième portion diluée vers l'ouverture (912, 722) à un second débit ;

mesure d'une seconde propriété de chaque cellule d'un sous-ensemble de cellules de la seconde portion diluée de l'échantillon biologique ayant traversé l'ouverture (912, 722) ; et

Détermination d'une concentration de cellules dans l'échantillon biologique et/ou d'une ou de plusieurs propriétés physiques des cellules de l'échantillon biologique sur la base de la deuxième propriété mesurée de chaque cellule du sous-ensemble de cellules de la deuxième partie diluée de l'échantillon biologique, le procédé comprenant l'ajout d'une pluralité de microparticules à l'échantillon biologique.

2. Procédé selon la revendication 1, dans lequel

(a) la pluralité des microparticules est **caractérisée par** un modèle de distribution granulométrique, et/ou

(b) éventuellement, la taille des microparticules est supérieure à 2 écarts-types par rapport à la moyenne de la taille d'une population cellulaire d'intérêt, où l'écarttype est l'écart-type par rapport à la moyenne d'une distribution connue ou prédite de la population cellulaire d'intérêt, et/ou

(c) à titre facultatif, le procédé comprend :

mesure d'une propriété physique de la pluralité de microparticules de la seconde partie de l'échantillon biologique ayant traversé l'ouverture (912, 722) ; et

déterminer les propriétés physiques d'une ou de plusieurs cellules de l'échantillon biologique sur la base de la propriété mesurée de la pluralité de microparticules ayant traversé le capteur (424,513),

éventuellement dans le cas de c) dans lequel la ou les propriétés physiques déterminées des cellules comprennent un volume de cellules, un diamètre de cellules, une surface de cellules et/ou une concentration d'hémoglobine, et/ou

Optionnellement (d) dans lequel le procédé comprend :

détermination d'une ou de plusieurs propriétés environnementales auxquelles l'échantillon biologique est exposé sur la base d'une ou de plusieurs propriétés physiques mesurées des microparticules ayant traversé le capteur (424,513),

éventuellement dans le cas de l'alinéa d) dans lequel la ou les propriétés environnementales comprennent le pH, l'osmolalité, le débit et/ou le rapport de mélange.

**3.** Procédé de mesure d'une concentration de cellules dans un échantillon biologique et/ou d'une propriété physique des cellules d'un échantillon biologique à l'aide d'un appareil, le procédé comprenant :

dilution d'une première portion d'un échantillon biologique à l'aide d'un premier diluant à l'aide d'un premier taux de dilution ;
faire couler la première portion diluée de l'échantillon biologique avec un second diluant vers une ouverture (912, 722) d'un capteur (424, 513) à un premier débit ;
mesure d'une première propriété de chaque cellule d'un sous-ensemble de cellules de la première partie diluée de l'échantillon biologique ayant traversé l'ouverture (912, 722) ;
la détermination d'une concentration cellulaire approximative de l'échantillon biologique sur la base du premier débit et de la première propriété physique mesurée de chaque cellule du sous-ensemble de cellules de la première partie diluée de l'échantillon biologique ;
diluer une deuxième partie de l'échantillon biologique à l'aide du premier diluant et du deuxième diluant à un deuxième rapport de dilution basé sur la concentration cellulaire approximative ;
écoulement de la deuxième portion diluée vers l'ouverture (912, 722) à un second débit ;
mesure d'une seconde propriété de chaque cellule d'un sous-ensemble de cellules de la seconde portion diluée de l'échantillon biologique ayant traversé l'ouverture (912, 722) ; et
Détermination d'une concentration de cellules dans l'échantillon biologique et/ou d'une ou de plusieurs propriétés physiques des cellules de l'échantillon biologique sur la base de la deuxième propriété mesurée de chaque cellule du sous-ensemble de cellules de la deuxième partie diluée de l'échantillon biologique, le procédé comprenant la mesure de l'osmolalité de l'échantillon biologique.

**4.** Procédé selon l'une quelconque des revendications précédentes, dans lequel

(i) les cellules comprennent des globules rouges, des globules blancs et/ou des plaquettes, et/ou
ii) la première et/ou la deuxième propriété mesurée de chaque pile comprend l'impédance, la résistance, la tension, un champ magnétique ou une variation de résonance magnétique, un son, une propriété optique, une diffusion de la lumière et/ou une absorption de la lumière,
(iii) la méthode comprend la détermination d'une ou de plusieurs propriétés physiques des cellules de l'échantillon biologique et dans laquelle la ou les propriétés physiques des cellules comprennent une taille, un volume, un diamètre, une surface et/ou une concentration d'hémoglobine, et/ou
(iv) dans lequel le procédé comprend l'attente d'une première période de temps prédéterminée après la dilution de la première portion et avant de faire couler la première portion vers l'ouverture au premier débit,
éventuellement dans le cas du point (iv) dans lequel la première période de temps prédéterminée comprend au moins 5 minutes.

**5.** Procédé selon l'une quelconque des revendications précédentes, dans lequel,

i) le procédé consiste à ajuster l'osmolalité d'un premier et/ou d'un second diluant pour qu'elle ne s'écarte pas de 0,1 mOsm/kg de l'osmolalité de l'échantillon biologique,
ii) La méthode comprend :

mesure d'une propriété optique de la première portion de l'échantillon dilué passée à l'ouverture (912, 722) à l'aide d'un capteur optique (424, 513) ; et
la détermination d'un ou de plusieurs des éléments suivants : un débit vers l'ouverture (912, 722), une osmolalité, un rapport de dilution de l'échantillon à un premier et/ou un second diluant et/ou des retards du système de la première partie de l'échantillon dilué passant à travers l'ouverture (912, 722) sur la base de la propriété optique,
éventuellement dans le cas de (ii) dans lequel la propriété optique de l'échantillon dilué comprend la fluorescence et/ou une densité optique.

**6.** Procédé de mesure d'une concentration et/ou d'une propriété physique des cellules d'un échantillon biologique à l'aide d'un appareil, le procédé comprenant :

l'écoulement d'un échantillon biologique à travers un canal d'un appareil à un premier débit ;
l'écoulement simultané d'un premier diluant à travers le canal de l'appareil à un second débit avec l'échantillon biologique pour créer un échantillon dilué ;
l'écoulement de l'échantillon dilué vers une ouverture (912, 722) d'un capteur (424, 513) ;

mesure d'une première propriété de chaque cellule d'un sous-ensemble de cellules d'une partie de l'échantillon dilué ayant traversé l'ouverture (912, 722) ;

la détermination d'une concentration cellulaire approximative de l'échantillon biologique sur la base du premier et du second débit et/ou de la première propriété mesurée de chaque cellule du sous-ensemble de cellules de la partie de l'échantillon dilué ;

réglage du premier débit et/ou du second débit pour obtenir une concentration de cellule souhaitée s'écoulant vers l'ouverture (912, 722), sur la base de la concentration approximative de la cellule ;

mesure d'une seconde propriété de chaque cellule d'un sous-ensemble de cellules de la partie de l'échantillon dilué ayant traversé l'ouverture (912, 722) à la concentration cellulaire souhaitée ; et

la détermination d'une concentration de cellules de l'échantillon biologique et/ou d'une ou de plusieurs propriétés physiques des cellules de l'échantillon biologique sur la base, au moins, de la deuxième propriété mesurée de chaque cellule du sous-ensemble de cellules de la portion de l'échantillon dilué, le procédé comprenant l'ajout d'une pluralité de microparticules à l'échantillon biologique,

Optionnellement, dans lequel le procédé comprend la mesure d'une propriété de chacune des multiples microparticules de la partie de l'échantillon dilué ayant traversé l'ouverture (912, 722) à la densité cellulaire souhaitée ; et

en déterminant la propriété physique d'une ou plusieurs cellules de l'échantillon biologique sur la base de la propriété mesurée de chacune des nombreuses microparticules ayant traversé le capteur.

7. Procédé de mesure d'une concentration et/ou d'une propriété physique des cellules d'un échantillon biologique à l'aide d'un appareil, dans lequel le procédé comprend :

l'écoulement d'un échantillon biologique à travers un canal d'un appareil à un premier débit ;

l'écoulement simultané d'un premier diluant à travers le canal de l'appareil à un second débit avec l'échantillon biologique pour créer un échantillon dilué ;

l'écoulement de l'échantillon dilué vers une ouverture (912, 722) d'un capteur (424, 513) ;

mesure d'une première propriété de chaque cellule d'un sous-ensemble de cellules d'une partie de l'échantillon dilué ayant traversé l'ouverture (912, 722) ;

la détermination d'une concentration cellulaire approximative de l'échantillon biologique sur la base du premier et du second débit et/ou de la première propriété mesurée de chaque cellule du sous-ensemble de cellules de la partie de l'échantillon dilué ;

réglage du premier débit et/ou du second débit pour obtenir une concentration de cellule souhaitée s'écoulant vers l'ouverture (912, 722), sur la base de la concentration approximative de la cellule ;

mesure d'une seconde propriété de chaque cellule d'un sous-ensemble de cellules de la partie de l'échantillon dilué ayant traversé l'ouverture (912, 722) à la concentration cellulaire souhaitée ; et

la détermination d'une concentration de cellules de l'échantillon biologique et/ou d'une ou de plusieurs propriétés physiques des cellules de l'échantillon biologique sur la base, au moins, de la deuxième propriété mesurée de chaque cellule du sous-ensemble de cellules de la portion de l'échantillon dilué,

dans lequel le procédé comprend la mesure de l'osmolalité de l'échantillon biologique, éventuellement dans lequel le procédé comprend l'ajustement de l'osmolalité du premier diluant pour qu'elle soit à moins de 0,1 mOsm/kg de l'osmolalité de l'échantillon, et/ou, éventuellement, dans lequel le procédé comprend le réglage de l'osmolalité du second diluant pour qu'elle soit à moins de 0,1 mOsm/kg de l'osmolalité de l'échantillon.

8. Procédé selon l'une quelconque des revendications 6 ou 7, dans lequel

(i) le procédé comprend la détermination de la propriété physique des cellules de l'échantillon biologique et dans lequel la propriété physique des cellules comprend une taille, un volume, un diamètre et/ou une surface, et/ou

(ii) les cellules comprennent un ou plusieurs globules rouges, globules blancs et/ou plaquettes, et/ou

iii) la méthode comprend la dilution de l'échantillon biologique à l'aide d'un second diluant à un premier taux de dilution, et/ou

(iv) la propriété mesurée des piles est une impédance, une résistance, une inductance, un courant, une tension, un champ magnétique ou une variation de résonance magnétique, un son, une diffusion de la lumière et/ou une absorption de la lumière.

9. Procédé selon l'une quelconque des revendications 6 à 8, dans lequel

(i) le procédé comprend le réglage automatique du premier débit et/ou du second débit pour atteindre et/ou maintenir la densité cellulaire souhaitée de la partie de l'échantillon dilué passant par l'ouverture (912, 722) du capteur (424, 513), et/ou

ii) dans lequel le procédé comprend le réglage automatique du premier débit et/ou du second débit pour obtenir un gradient d'osmolalités de la partie de l'échantillon dilué passant à travers l'ouverture (912, 722) du capteur (424, 513),

dans le cas (ii) : éventuellement, dans lequel le procédé comprend en outre la détermination d'un pic d'osmolalité des cellules de l'échantillon biologique sur la base de la propriété mesurée de chaque cellule du sous-ensemble de cellules à partir de la partie de l'échantillon dilué, et/ou

dans le cas de (i) ou (ii) : (a) de manière facultative, dans lequel la dilution de l'échantillon biologique comprend l'écoulement simultané du deuxième diluant à travers le canal à un troisième débit et l'ajustement automatique du troisième débit pour atteindre et/ou maintenir la concentration cellulaire souhaitée de la partie de l'échantillon dilué passant par l'ouverture (912, 722) du capteur (424, 513), et/ou

dans le cas de (i) ou (ii) : (b) au choix, dans lequel la dilution de l'échantillon biologique comprend l'écoulement simultané du deuxième diluant à travers le canal à un troisième débit et l'ajustement automatique du troisième débit pour obtenir un gradient d'osmolalités de la partie de l'échantillon dilué passant à travers l'ouverture (912, 722).

10. Procédé selon l'une quelconque des revendications 6 à 9, dans lequel le procédé comprend la mesure d'une propriété optique de la partie de l'échantillon dilué passée à l'ouverture (912, 722) à l'aide d'un capteur optique (424, 513) ; et

la détermination d'un ou de plusieurs des éléments suivants : un taux de dilution de l'échantillon par rapport à un premier et/ou un second diluant, un débit, une osmolalité et/ou une densité cellulaire en fonction, au moins, de la propriété optique mesurée,

(a) de manière facultative, lorsque la propriété optique comprend la fluorescence et/ou la densité optique de l'échantillon dilué, et/ou

(b) éventuellement, lorsque la méthode comprend la détermination d'une ou de plusieurs propriétés physiques des cellules de l'échantillon biologique sur la base de la propriété optique, et/ou

éventuellement dans le cas de (b) dans lequel la ou les propriétés physiques des cellules comprennent la taille, la forme, le volume, le diamètre et/ou la surface, et/ou

(c) facultativement, dans lequel l'étape consistant à déterminer la masse volumique des cellules de l'échantillon biologique et/ou la ou les propriétés physiques des cellules de l'échantillon biologique est en outre fondée sur une comparaison d'une propriété électrique mesurée de chaque cellule du sous-ensemble de cellules de la partie de l'échantillon dilué avec une valeur de référence,

éventuellement dans le cas de (c) dans lequel la propriété électrique mesurée comprend l'impédance, la résistance, l'inductance, le courant et/ou la tension.

11. Procédé de mesure d'une concentration de cellules dans un échantillon biologique et/ou d'une propriété physique des cellules d'un échantillon biologique à l'aide d'un appareil, dans lequel le procédé comprend :

dilution d'une partie d'un échantillon biologique à l'aide d'un premier diluant à l'aide d'un taux de dilution ;

l'écoulement de la partie diluée vers un capteur (424,513) de l'appareil avec un deuxième diluant et un troisième diluant à un premier débit combiné ;

mesure d'une première propriété physique de chaque cellule d'un sous-ensemble de cellules de la partie diluée d'avoir traversé l'ouverture (912, 722) au premier débit combiné ;

la détermination d'une concentration cellulaire approximative de l'échantillon biologique sur la base du premier débit combiné et de la première propriété physique mesurée de chaque cellule du sous-ensemble de cellules de la partie diluée ;

détermination d'un second débit combiné en fonction de la concentration approximative de la cellule ;

écoulement de l'échantillon biologique, du deuxième diluant et du troisième diluant à un deuxième débit combiné vers l'ouverture (912, 722) ;

mesure de la première propriété physique de chaque cellule d'un sous-ensemble de cellules de la portion ayant traversé l'ouverture (912, 722) au deuxième débit combiné ; et

Détermination d'une concentration de cellules dans l'échantillon biologique et/ou d'une seconde propriété physique des cellules de l'échantillon biologique sur la base de la seconde propriété physique de chaque cellule du sous-ensemble de cellules de l'échantillon biologique ayant coulé vers l'ouverture (912, 722) à l'aide du second débit combiné, le procédé comprenant l'ajout d'une pluralité de microparticules à l'échantillon biologique.

**12.** Procédé selon la revendication 11, dans lequel la taille d'une microparticule est supérieure à 2 écarts-types par rapport à la moyenne de la taille de la cellule d'intérêt, dans lequel l'écart-type est l'écart-type par rapport à la moyenne d'une distribution connue ou prédite de la population cellulaire d'intérêt.

**13.** Procédé selon l'une quelconque des revendications 11 ou 12, dans lequel le procédé comprend l'attente d'une période de temps prédéterminée avant d'écouler la portion vers l'ouverture (912, 722) au premier débit combiné, éventuellement dans lequel la période de temps prédéterminée est d'au moins 5 minutes.

**14.** Procédé selon l'une quelconque des revendications 11 à 13, dans lequel

(i) le procédé comprend la mesure d'une propriété physique de la pluralité de microparticules de la partie de l'échantillon biologique ayant traversé l'ouverture (912, 722) lorsqu'elle s'écoule vers l'ouverture (912, 722) au deuxième débit combiné ; et
déterminer une ou plusieurs propriétés physiques des cellules de l'échantillon biologique sur la base, au moins, de la propriété physique mesurée de la pluralité de microparticules ayant traversé le capteur (424,513), et/ou
ii) la méthode comprend la mesure de l'osmolalité de l'échantillon biologique,
éventuellement dans le cas de (ii), dans lequel le procédé comprend l'ajustement de l'osmolalité du premier diluant pour qu'elle soit à moins de 0,1 mOsm/kg de l'osmolalité de l'échantillon biologique.

FIG.1

FIG. 2A

FIG. 2B

FIG. 3

FIG. 4

FIG. 5

FIG. 6

FIG. 7A

FIG. 7B

FIG. 7C

FIG.8

FIG. 9A

FIG. 9B

FIG. 10

FIG. 11A

FIG. 11B

FIG.12

FIG.13

FIG. 14

FIG. 15A

FIG. 15B

FIG. 15 C

FIG. 16A

FIG. 16B

FIG. 16C

FIG. 16D

Effect of Concentration
in PBS

FIG. 17

FIG. 18

FIG. 19

FIG. 20

FIG. 21

Effect of Mixing Time on Pk0

FIG. 22

FIG. 23

pH Effect on Pk0

FIG. 24

FIG. 25

FIG. 26A

FIG. 26B

## REFERENCES CITED IN THE DESCRIPTION

**Patent documents cited in the description**

- US 62943757 B
- WO 1997024601 A
- US 6668229 B
- US 6422065 B
- US 2017159003 A1
- US 1964543 W
- US 2029114 W
- WO 9724529 A
- US 64220651 B
- US 6070476 A
- US 62775703